(19) Europäisches Patentamt / European Patent Office / Office européen des brevets

(11) EP 2 785 841 B1

(12) # FASCICULE DE BREVET EUROPEEN

(45) Date de publication et mention de la délivrance du brevet:
**01.01.2020 Bulletin 2020/01**

(21) Numéro de dépôt: **12813118.2**

(22) Date de dépôt: **28.11.2012**

(51) Int Cl.:
***C12N 15/115*** *(2010.01)* ***C12N 9/88*** *(2006.01)*
***C07K 1/22*** *(2006.01)* *C07K 14/47* *(2006.01)*

(86) Numéro de dépôt international:
**PCT/IB2012/056786**

(87) Numéro de publication internationale:
**WO 2013/080134 (06.06.2013 Gazette 2013/23)**

(54) **APTAMÈRES ANTI-FH, PROCÉDÉ POUR LEUR OBTENTION ET UTILISATIONS**

ANTI-FH APTAMERE, HERSTELLUNGSVERFAHREN DAFÜR UND VERWENDUNGEN DAVON

ANTI-FH APTAMERS, METHOD FOR PRODUCING SAME, AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2011 FR 1160862**

(43) Date de publication de la demande:
**08.10.2014 Bulletin 2014/41**

(73) Titulaire: **Laboratoire Français du Fractionnement et des Biotechnologies**
**91940 Les Ulis (FR)**

(72) Inventeurs:
- **PERRET, Gérald**
**94600 Choisy le Roi (FR)**
- **CIBIEL, Agnès**
**77210 Avon (FR)**

(74) Mandataire: **Verhaeghe Bect, Elodie**
**Cabinet Becker & Associés**
**25, rue Louis le Grand**
**75002 Paris (FR)**

(56) Documents cités:
**WO-A1-2010/094901** **WO-A2-02/077262**
**WO-A2-2004/055153** **WO-A2-2006/062716**
**WO-A2-2011/053982**

- **DATABASE Geneseq [Online] 28 décembre 2007 (2007-12-28), "Viral regulatory miRNA SEQ ID NO 165956 of WO2004048511.", XP002683246, extrait de EBI accession no. GSN:AJJ13636 Database accession no. AJJ13636**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

### DOMAINE DE L'INVENTION

La présente invention se rapporte au domaine de l'obtention de préparations de facteur H purifié, à usage thérapeutique.

### ART ANTERIEUR

Le complément joue un rôle essentiel dans la défense de l'organisme contre des agents infectieux et dans le processus inflammatoire. Il représente un système auxiliaire de l'immunité, en particulier de la réponse humorale et de la réponse innée.

Le Facteur H est le principal régulateur de la voie alterne du complément. Il agit en phase liquide comme à la surface des cellules. Initialement désignée " bêta 1 H globuline ", cette glycoprotéine sérique de 155 kDa est également appelée Facteur H 1, FH, CFH, ou HF1 . Le Facteur H est synthétisé dans le foie, les macrophages, les fibroblastes, les cellules endothéliales et les plaquettes. La forme sécrétée de la protéine est composée de 20 unités répétitives (ou " short consensus repeats ", SCR) de 60 acides aminés.

L'activité anti-complémentaire du Facteur H se traduit par la régulation du taux de complexes immuns dans le sang, il participe par conséquent à l'équilibre entre les processus résultant en leur génération ou en leur dégradation. Le facteur H réduit la demi-vie de la C3 convertase (C3bBb) alterne en liant le C3b et en dissociant le Bb et sert de cofacteur au Facteur I dans la protéolyse du C3b, libre ou lié à la surface des cellules, en C3bi. Ainsi, les complexes immuns composés d'un complexe antigène-anticorps associé au composant du complément C3b ne peuvent plus activer la cascade subséquente du complément (composants C5-C9).

Le Facteur H a été proposé dans le traitement du Syndrome Hémolytique et Urémique atypique (SHUa) associé à une anomalie héréditaire du système du complément (demande de brevet FR2894145). Par ailleurs, le Facteur H est indiqué pour le traitement de néphropathies chroniques (WO2007/038995).

Une source appropriée pour la préparation d'une préparation de Facteur H à usage thérapeutique est du plasma sanguin. Le plasma sanguin est utilisé depuis longtemps pour la préparation de produits dérivés du sang de type albumine, préparations d'immunoglobulines, concentrés de facteur de la coagulation (Facteur VIII, Facteur IX etc.), etc. Des méthodes de fractionnement du plasma sont connues, permettant d'enrichir certaines fractions en produits désirés. Des schémas simplifiés du fractionnement plasmatique industriel Cohn/Oncley modifié, et du fractionnement plasmatique industriel Kistler/Nitschmann sont présentés dans la demande de brevet WO2008/1 13589, qui concerne une préparation d'une formulation de facteur H à partir d'une fraction éthanolique.

La demande de brevet WO2007/066017 décrit un procédé de purification du Facteur H comprenant une chromatographie par échange d'anions, suivie par deux étapes de chromatographie d'affinité de type héparine, puis, dans l'ordre, une chromatographie par échange de cations, et une chromatographie par échange d'anions. La demande de brevet WO2008/113589 décrit l'obtention d'un Facteur H purifié par une chromatographie d'affinité avec héparine à partir d'une fraction éthanolique de plasma sanguin, suivie d'une chromatographie par échange d'anions, et une chromatographie par échange de cations.

Ces procédés restent imparfaits, soit parce qu'ils consomment trop d'héparine, qui est un produit cher (WO2007/066017), soit parce qu'ils ne parviennent pas à un niveau de pureté du Facteur totalement satisfaisant pour un usage thérapeutique (WO2008/1 13589). L'invention vise à résoudre ces problèmes.

Les demandes de brevet WO2011/058284 et WO2011/058285 décrivent des procédés de purification de facteur H humain d'origine plasmatique comprenant notamment une succession d'étapes de chromatographie d'échange d'ions, ce qui englobe au moins une étape de chromatographie d'échange de cations et au moins une étape de chromatographie d'échanges d'anions. Les caractéristiques de ces procédés sont spécifiquement adaptées à l'obtention de facteur H purifié à partir de fractions de plasma humain.

Il existe un besoin pour la disponibilité de nouveaux procédés d'obtention de facteur H sous forme purifiée, alternatifs ou améliorés par rapport aux procédés connus.

### RESUME DE L'INVENTION

Il est fourni selon l'invention de nouveaux outils, utiles pour la détection ou la purification du Facteur H, ainsi que des procédés de détection ou de purification du Facteur H dans lesquels ces outils nouveaux sont utilisés.

La présente invention fournit des aptamères nucléiques se liant sélectivement au Facteur H, ainsi que des supports de détection et des supports d'affinité sur lesquels ces aptamères nucléiques sont immobilisés.

La présente invention concerne des aptamères nucléiques se liant au Facteur H et comprenant la séquence SEQ ID NO:243. Lesdits aptamères nucléiques englobent notamment les aptamères tels que spécifiés dans la revendication 2.

De préférence, les aptamères nucléiques ci-dessus sont obtenus selon une méthode dérivée du procédé général d'obtention d'aptamères connu sous le nom de "SELEX", ladite méthode pouvant comprendre une ou plusieurs étapes originales de nature à conférer aux aptamères obtenus des caractéristiques accroissant leur aptitude à se lier de manière réversible au Facteur H cible, notamment dans des conditions optimales de mise en œuvre d'une étape d'enrichissement en Facteur H par chromatographie d'affinité.

Dans certains modes de réalisation, les aptamères nucléiques de l'invention sont caractérisés en ce qu'ils se lient à un facteur H choisi parmi un Facteur H plasmatique, un Facteur H recombinant et un Facteur H transgénique.

Dans certains modes de réalisation, les aptamères nucléiques de l'invention sont caractérisés en ce qu'ils se lient à un Facteur H choisi parmi un Facteur H humain et un facteur H non humain.

Dans certains autres modes de réalisation, les aptamères nucléiques consistent en des aptamères désoxyribonucléotidiques.

La présente invention est aussi relative à un support d'affinité pour la fixation sélective du Facteur H, comprenant un matériau support solide sur lequel sont immobilisés des aptamères nucléiques tels que définis ci-dessus. Elle concerne aussi des procédés d'obtention dudit support d'affinité.

La présente invention a également trait à un procédé pour la purification de Facteur H comprenant les étapes suivantes :

a) mettre en contact un échantillon contenant un Facteur H avec un support d'affinité tel que défini ci-dessus, afin de former des complexes entre (i) les aptamères nucléiques immobilisés sur ledit support d'affinité et (ii) ledit Facteur H,
b) libérer le Facteur H à partir des complexes formés à l'étape a), et
c) récupérer le Facteur H sous forme purifiée.

## DESCRIPTION DES FIGURES

La **Figure 1** illustre la capacité de liaison au Facteur H humain de la collection d'acides nucléiques de départ.
La Figure ***1A*** illustre les courbes de liaison des acides nucléiques de la banque d'acides nucléiques de départ au Facteur H humain plasmatique préalablement immobilisé sur un support. Sur la Figure 1A, les courbes de liaison des acides nucléiques de la banque de départ sont situées approximativement au niveau du signal de base de la solution tampon exempte d'acides nucléiques.
La Figure ***1B*** illustre les courbes de liaison du Facteur H humain plasmatique aux acides nucléiques de la banque d'aptamères de départ préalablement immobilisés sur un support. Sur la Figure 1B, les courbes de liaison du Facteur H humain plasmatique aux acides nucléiques de la banque de départ sont situées approximativement au niveau du signal de base de la solution tampon exempte de Facteur H.
En abscisse : le temps, exprimé en secondes. En ordonnées : signal de résonance, exprimé en Unités de Résonance arbitraires.
La **Figure 2** illustre la capacité des acides nucléiques sélectionnés après un nombre croissant de cycles de réitération du procédé de type SELEX à se lier au Facteur H humain plasmatique.
La Figure ***2A*** illustre les courbes de liaison des acides nucléiques de la banque d'acides nucléiques de départ et des populations enrichies en acides nucléiques se liant au Facteur H humain plasmatique préalablement immobilisé sur un support. Courbes "1" : comprend (i) les courbes de la solution tampon de sélection (comprenant la courbe la plus inférieure), (ii) la courbe des acides nucléiques contenus dans la banque de départ, et (iii) la courbe des acides nucléiques obtenus à l'issue du cycle n° 4 (SHT4) du procédé SELEX. Courbe n° 2 : la courbe des acides nucléiques obtenus à l'issue du cycle n° 5 (SHT5) du procédé SELEX. Courbe n° 3 : la courbe des acides nucléiques obtenus à l'issue du cycle n° 6 (SHT6) du procédé Selex. Courbe n° 4 : la courbe des acides nucléiques obtenus à l'issue du cycle n° 7 (SHT7) du procédé Selex. Courbe n° 5 : la courbe des acides nucléiques obtenus à l'issue du cycle n° 9 (SHT9) du procédé Selex. Courbe n° 6 : la courbe des acides nucléiques obtenus à l'issue du cycle n° 8 (SHT8) du procédé Selex.
La Figure ***2B*** illustre les courbes de liaison du Facteur H humain plasmatique à la population enrichie en acides nucléiques issue du cycle n° 6 de sélection par la méthode SELEX. Les acides nucléiques ont été préalablement immobilisés sur un support. Courbe n° 1 : courbe de la solution tampon de sélection exempte de Facteur H. Courbe n° 2 : courbe de liaison du Facteur H humain plasmatique à la concentration finale de 200 nM. Courbe n° 3 : courbe de liaison du Facteur H humain plasmatique à la concentration finale de 400 nM.
En abscisse : le temps, exprimé en secondes. En ordonnées : signal de résonance, exprimé en Unités de résonance arbitraires.

La **Figure 3** illustre un profil de chromatographie d'un Facteur H humain plasmatique purifié par un procédé classique incluant notamment une étape d'affinité héparine sur un support d'affinité sur lequel sont immobilisés des aptamères nucléiques anti-FH. En abscisse : le temps, exprimé en minutes. En ordonnées : valeur d'absorbance (DO) à 254 nanomètres.

La **Figure 4** illustre un profil de chromatographie d'un surnageant de culture de cellules produisant un Facteur H humain recombinant sur un support d'affinité sur lequel sont immobilisés des aptamères nucléiques anti-FH. En abscisse : le temps, exprimé en minutes. En ordonnées : valeur d'absorbance (DO) à 254 nanomètres.

La **Figure 5A** est un cliché d'un gel d'électrophorèse après coloration au nitrate d'argent. De gauche à droite sur la figure : Piste n° 1 : échantillon de FH plasmastique pré-purifé de départ ; Piste n° 2 : fraction non retenue après passage de l'échantillon de FH plasmatique pré-purifé utilisé à la piste n° 1sur un support d'affinité sur lequel sont immobilisés des aptamères nucléiques anti-FH ; Piste n° 3 : fraction d'élution après passage de l'échantillon de FH plasmatique pré-purifié utilisé à la piste n° 1sur un support d'affinité sur lequel sont immobilisés des aptamères nucléiques anti-FH ; Piste n° 4 : marqueurs de poids moléculaire.

La **Figure 5B** est un cliché d'un gel d'électrophorèse après coloration au nitrate d'argent. De gauche à droite sur la figure : Piste n° 1 : marqueurs de poids moléculaire ; Piste n° 2 : échantillon de surnageant de culture de cellules produisant un Facteur H humain recombinant; Piste n° 3 : fraction non retenue après passage de l'échantillon de plasma utilisé à la piste n° 1sur un support d'affinité sur lequel sont immobilisés des aptamères nucléiques anti-FH ; Piste n° 4 : fraction d'élution après passage de l'échantillon de plasma utilisé à la piste n° 1sur un support d'affinité sur lequel sont immobilisés des aptamères nucléiques anti-FH.

La **Figure 6** est une représentation de la structure secondaire e de l'aptamère nucléique MaptH1.1. La **Figure 6A** est une représentation de la structure secondaire de l'aptamère MaptH1.1. complet. La **Figure 6B** est une représentation de la structure secondaire de la partie centrale « SEQ ID N° X » de l'aptamère MaptH1.1..

La **Figure 7** représente les courbes de liaison au ***Facteur H humain plasmatique purifié*** d'une variété d'aptamères nucléiques dérivés de l'aptamère Mapt H1.1. Les courbes allant du haut vers le bas sur la Figure 7 représentent la liaison au Facteur H humain aux aptamères respectifs suivants : (1) MaptH1.1 N13-48, (2) MaptH1.1 N19-58, (3) MaptH1.1 N19-53 ; (4) MaptH1.1 N19-48 et (5) MaptH1.1 N22-45.

## DESCRIPTION DETAILLEE DE L'INVENTION

Le demandeur s'est principalement attaché à concevoir de nouveaux procédés pour la purification du Facteur H, en particulier du Facteur H humain. A cette fin, le demandeur a isolé et caractérisé de nouveaux ligands qui possèdent une aptitude à se lier sélectivement au Facteur H. Ces nouveaux ligands, dont les caractéristiques sont détaillées plus loin dans la présente description, consistent en des acides nucléiques, aussi appelés "aptamères nucléiques", se liant sélectivement au facteur H.

A la connaissance du demandeur, des aptamères nucléiques se liant sélectivement au Facteur H sont décrits pour la première fois dans la présente description.

La présente invention fournit des procédés de purification de Facteur H, dans lesquels il est tiré bénéfice des propriétés de liaison de ces nouveaux ligands du type acide nucléique.

Le demandeur s'est aussi attaché à mettre au point des procédés permettant l'obtention d'aptamères nucléiques se liant spécifiquement au Facteur H.

Par " Facteur H " on entend toute protéine ayant la séquence en acides aminés du Facteur H natif d'un mammifère, et en particulier toute protéine ayant la séquence en acides aminés du Facteur H humain. Le terme " Facteur H " comprend en outre les variations alleliques naturelles et/ou les isoformes du Facteur H trouvées naturellement, et toute forme ou degré de glycosylation ou autre modification post-traductionnelle. Sont aussi compris les homologues ou dérivés du Facteur H qui présentent la même activité biologique ou une activité biologique supérieure par rapport à l'activité de la forme sauvage et/ou qui présentent une identité de séquence d'au moins 80 %, de préférence au moins 85 %, de préférence encore d'au moins 90 % par rapport à ladite forme sauvage.

Le terme " activité biologique " du Facteur H inclut la capacité à inhiber la C3 convertase et/ou à servir de cofacteur du Facteur I, résultant à l'inhibition de l'activation de la cascade du complément. L'activité du Facteur H peut être mesurée de différentes manières, bien connues de l'homme du métier.

Par " aptamère nucléique ", on entend selon l'invention un acide nucléique simple brin se liant sélectivement au Facteur H, qui peut aussi être désigné "aptamère anti-FH" aux fins de la présente description.

La détection de complexes formés entre un aptamère anti-FH selon l'invention et le Facteur H peut être aisément réalisée par l'homme du métier, par exemple en mettant en oeuvre une technique de détection par résonance plasmonique de surface, y compris la technique Biacore®, comme cela est illustré dans les exemples. L'homme du métier peut aussi aisément détecter la formation de complexes entre un aptamère anti-FH selon l'invention et le Facteur H par des techniques classiques du type ELISA, comme cela est connu par l'homme du métier.

On a montré dans les exemples qu'un aptamère nucléique anti-FH est capable de se lier sélectivement au

Facteur H humain n'ayant pas subi de clivage par protéolyse.

De manière générale, les aptamères anti-FH peuvent consister en des molécules d'ADN (acide désoxyribonucléique) ou d'ARN (acide ribonucléique) et ont une capacité à se lier au Facteur H, supérieure à la capacité à se lier à une protéine autre que le Facteur H.

Un aptamère anti-FH peut être obtenu par des procédés qui ont été spécialement mis au point pour les besoins de l'invention, et qui sont détaillés plus loin dans la présente description.

Il est décrit que les aptamères anti-FH peuvent posséder diverses caractéristiques structurelles communes, y inclus une séquence comprenant, de l'extrémité 5' vers l'extrémité 3', successivement (i) une séquence nucléotidique spécifique invariable d'environ 20 nucléotides de longueur, suivie de (ii) une séquence nucléotidique variable d'environ 40 nucléotides de longueur, suivie de (iii) une séquence nucléotidique spécifique invariable d'environ 20 nucléotides de longueur. On précise que les séquences nucléotidiques variables (ii) peuvent posséder entre elles une très forte identité de séquence en nucléotides.

Ladite séquence variable (ii) peut avoir une longueur allant de 15 à 50 nucléotides, comme cela est détaillé plus loin.

Les procédés de sélection d'aptamères anti-FH qui sont spécifiés dans la présente description sont du type permettant l'obtention d'un ensemble d'aptamères anti-FH, aptes à reconnaître le Facteur H, en particulier le Facteur H humain.

D'un point de vue structurel, il est décrit que chaque élément de la famille d'acides nucléiques, ou aptamères nucléiques, qui se lient spécifiquement au Facteur H, comprend au moins 15 nucléotides consécutifs d'un polynucléotide ayant au moins 40 % d'identité en nucléotides avec l'acide nucléique de formule (I) suivante :

5'-[SEQ ID N°1]x-[SEQ ID N° X]-[SEQ ID N°2]y-3' (I),

dans laquelle :

- " SEQ ID N° X " consiste en un acide nucléique de séquence choisie parmi les séquences SEQ ID N° 3 à SEQ ID N° 116,
- " x " est un entier égal à 0 ou 1, et
- " y " est un entier égal à 0 ou 1.

Les aptamères nucléiques ci-dessus englobent ceux dont la séquence SEQ ID N°X a au moins 40 % d'identité en nucléotides avec un acide nucléique de séquence choisie parmi les séquences SEQ ID N° 3 à SEQ ID N° 116. Sont englobés les aptamères nucléiques de ce type dont les séquences flanquantes aux extrémités 5' et 3' ont 100 % d'identité en nucléotides avec les séquences SEQ ID N° 1 et SEQ ID N° 2, respectivement.

Il est décrit que l'acide nucléique de séquence SEQ ID N°X a une longueur de 15, 16, 17, 18, 19, 20, 21 , 22, 23, 24, 25, 26, 27, 28, 29, 30, 31 , 32, 33, 34, 35, 36, 37, 38, 39, 40, 41 , 42, 43, 44, 45, 46, 47, 48, 49, ou 50 nucléotides, la longueur dudit acide nucléique étant limitée par la longueur maximale de la séquence de référence dont elle dérive, ladite séquence de référence étant choisie parmi les séquences SEQ ID N° 3 à SEQ ID N° 116, ce qui inclut SEQ ID N° 4 à 116..

Il est décrit que l'acide nucléique de séquence SEQ ID N°X a une longueur de 35, 36, 37, 38, 39, 40, 41, 42, 43, 44 ou 45 nucléotides de longueur, la longueur dudit acide nucléique étant limitée par la longueur maximale de la séquence de référence dont elle dérive.

Il est également décrit que l'acide nucléique de séquence SEQ ID °X a une longueur de 38, 39, 40 ou 41 nucléotides de longueur.

Comme déjà mentionné précédemment, l'acide nucléique de formule (I) a au moins 15 nucléotides de longueur.

L'acide nucléique de formule (I) a au moins 15, 16, 17, 18, 19, 20, 21 , 22, 23, 24, 25, 26, 27, 28, 29, 30, 31 , 32, 33, 34, 35, 36, 37, 38, 39, 40,41, 42, 43, 44, 45, 46, 47, 48, 49, 50, 51 , 52, 53, 54, 55, 56, 57, 58, 59, 60, 61 , 62, 63, 64, 65, 66, 67, 68, 69, 70, 71 , 72, 73, 74, 75, 76, 77, 78, 79, 80 ou 81 nucléotides de longueur, ce qui englobe les acides nucléiques possédant exactement chacune des longueurs spécifiées.

Lorsque l'entier " x " est égal à 0 et l'entier " y " est égal à 1, les aptamères nucléiques de l'invention englobent les acides nucléiques comprenant au moins 15 nucléotides consécutifs d'un polynucléotide ayant au moins 40 % d'identité en nucléotides avec l'acide nucléique de formule (1-1) suivante :

5' -[SEQ ID N° X]-[SEQ ID N°2] -3' (I-1 ).

Il est décrit des aptamères nucléiques ayant au moins 15 nucléotides consécutifs d'un acide nucléique de

formule (1-1) ci-dessus. LI1 est également décrit des aptamères nucléiques ayant au moins 40 % d'identité en nucléotides avec un acide nucléique de formule (I-1) ci-dessus. Sont englobés les aptamères nucléiques de formule (I-1) dont la séquence flanquante à l'extrémité 3' possède 100 % d'identité en nucléotides avec la séquence SEQ ID N° 2.

Lorsque l'entier " x " est égal à 1 et l'entier " y " est égal à 0, les aptamères nucléiques englobent les acides nucléiques comprenant au moins 15 nucléotides consécutifs d'un polynucléotide ayant au moins 40 % d'identité en nucléotides avec l'acide nucléique de formule (I-2) suivante :

5'-[SEQ ID N°1] -[SEQ ID N° X]-3' (I-2)

Il est décrit des aptamères nucléiques ayant au moins 15 nucléotides consécutifs d'un acide nucléique de formule (I-2) ci-dessus. Il est également décrit des aptamères nucléiques ayant au moins 40 % d'identité en nucléotides avec un acide nucléique de formule (I-2) ci-dessus. Sont englobés les aptamères nucléiques de formule (I-1) dont la séquence flanquante à l'extrémité 5' posède 100 % d'identité en nucléotides avec la séquence SEQ ID N° 1.

Lorsque l'entier " x " est égal à 0 et l'entier " y " est égal à 0, les aptamères nucléiques englobent les acides nucléiques comprenant au moins 15 nucléotides consécutifs d'un polynucléotide ayant au moins 40 % d'identité en nucléotides avec l'acide nucléique de formule (I-3) suivante :

5'-[SEQ ID N° X]-3' (I-3)

Il est aussi décrit des aptamères nucléiques ayant au moins 15 nucléotides consécutifs d'un acide nucléique de formule (I-3) ci-dessus. On décrit également des aptamères nucléiques ayant au moins 40 % d'identité en nucléotides avec un acide nucléique de formule (I-3) ci-dessus.

Les aptamères nucléiques englobent les acides nucléiques comprenant au moins 15 nucléotides consécutifs d'un acide nucléique de séquence choisie parmi les séquences SEQ ID N° 117 à SEQ ID N° 232. Les aptamères nucléiques englobent aussi les acides nucléiques ayant au moins 40 % d'identité en nucléotides avec un acide nucléique de séquence choisie parmi les séquences SEQ ID N° 117 à SEQ ID N° 232.

Les aptamères nucléiques englobent en particulier les acides nucléiques comprenant au moins 15 nucléotides consécutifs d'un acide nucléique de séquence choisie parmi les séquences SEQ ID N° 3 à SEQ ID N° 116, ce qui inclut SEQ ID N° 4 à 116. Les aptamères nucléiques englobent aussi les acides nucléiques ayant au moins 40 % d'identité en nucléotides avec un acide nucléique de séquence choisie parmi les séquences SEQ ID N° 3 à SEQ ID N° 116, ce qui inclut SEQ ID N° 4 à 116.

Selon certains aspects de l'acide nucléique comprenant la séquence SEQ ID N° X, ladite séquence SEQ ID N° X est choisie dans le groupe constitué des acides nucléiques ayant au moins 15 nucléotides consécutifs d'une séquence choisie parmi les séquences SEQ ID N° 3 à SEQ ID N° 116, ce qui inclut SEQ ID N° 4 à 116.

Selon d'autres aspects de l'acide nucléique comprenant la séquence SEQ ID N° X, ladite séquence SEQ ID N° X est choisie dans le groupe constitué des acides nucléiques possédant au moins 40 % d'identité en nucléotides avec une séquence choisie parmi les séquences SEQ ID N° 3 à SEQ ID N° 116, ce qui inclut SEQ ID N° 4 à 116.

Il est décrit un aptamère choisi parmi les acides nucléiques comprenant une séquence choisie parmi les séquences SEQ ID N° 117 à SEQ ID N° 232.

Il est aussi décrit un aptamère choisi parmi les acides nucléiques consistant en une séquence choisie parmi les séquences SEQ ID N° 117 à SEQ ID N° 232.

Les acides nucléiques se liant sélectivement au Facteur H définis dans la présente description peuvent présenter entre eux des homologies structurelles, c'est-à-dire présenter entre eux un haut pourcentage d'identité en nucléotides.

En particulier, certains des acides nucléiques se liant sélectivement au Facteur H peuvent avoir en commun des enchaînements de nucléotides identiques, qui sont désignés "séquences consensus" aux fins de la présente description.

Par exemple, une première famille d'acides nucléiques selon l'invention, ou "Famille 1", se liant au Facteur H est la famille d'acides nucléiques de séquences SEQ ID N° 3 à SEQ ID N° 42, ce qui inclut SEQ ID N° 4 à 42, et SEQ ID N° 117 à SEQ ID N° 158. On précise que les acides nucléiques de séquence SEQ ID N° 117 à SEQ ID N° 156 comprennent les séquences SEQ ID N° 3 à SEQ ID N° 42, ce qui inclut SEQ ID N° 4 à 42, respectivement. Les acides nucléiques de la Famille 1 sont structurellement liés entre eux au moins du fait de la présence d'une séquence consensus commune du type "GGGTCGGGGTTATACG" (SEQ ID N° 233), étant entendu que ladite séquence consensus n'est pas nécessairement retrouvée sous la forme d'un enchaînement continu de nucléotides, deux nucléotides contigus de ladite séquence consensus pouvant être séparés par un ou plusieurs nucléotides dans la séquence de référence. A titre illustratif, la séquence consensus de la Famille 1 est retrouvée, dans la séquence SEQ ID N° 3, sous la forme de la séquence "GGGTCTGGTGGTTATACG" (SEQ ID N° 234).

Il est décrit une seconde famille d'acides nucléiques, "Famille 2", se liant au Facteur H qui est la famille d'acides nucléiques de séquences SEQ ID N° 43 à SEQ ID N° 60 et SEQ ID N° 159 à SEQ ID N° 176. On précise que les acides nucléiques de séquence SEQ ID N° 159 à SEQ ID N° 176 comprennent les séquences SEQ ID N° 43 à SEQ ID N° 60, respectivement. Les acides nucléiques de la Famille 2 sont structurellement liés entre eux au moins du fait de la présence d'une séquence consensus commune du type "CCGGTTACCTGGTGTTGTGGC" (SEQ ID N° 235), étant entendu que ladite séquence consensus n'est pas nécessairement retrouvée sous la forme d'un enchaînement continu de nucléotides, deux nucléotides contigus de ladite séquence consensus pouvant être séparés par un ou plusieurs nucléotides dans la séquence de référence.

On décrit également une troisième famille d'acides nucléiques, "Famille 3", se liant au Facteur H qui est la famille d'acides nucléiques de séquences SEQ ID N° 61 à SEQ ID N° 64 et SEQ ID N° 177 à SEQ ID N° 180. On précise que les acides nucléiques de séquence SEQ ID N° 177 à SEQ ID N° 180 comprennent les séquences SEQ ID N° 61 à SEQ ID N° 64, respectivement. Les acides nucléiques de la Famille 3 sont structurellement liés entre eux au moins du fait de la présence d'une séquence consensus commune du type "GGAGGCTTTTACAGGCGGTGCG" (SEQ ID N° 236), étant entendu que ladite séquence consensus n'est pas nécessairement retrouvée sous la forme d'un enchaînement continu de nucléotides, deux nucléotides contigus de ladite séquence consensus pouvant être séparés par un ou plusieurs nucléotides dans la séquence de référence.

On décrit de plus une quatrième famille d'acides nucléiques, "Famille 4", se liant au Facteur H qui est la famille d'acides nucléiques de séquences SEQ ID N° 65 à SEQ ID N° 66 et SEQ ID N° 181 à SEQ ID N° 182. On précise que les acides nucléiques de séquence SEQ ID N° 181 à SEQ ID N° 182 comprennent les séquences SEQ ID N° 65 à SEQ ID N° 66, respectivement. Les acides nucléiques de la Famille 4 sont structurellement liés entre eux au moins du fait de la présence d'une séquence consensus commune du type "GGGGGGGGTTGGGGGACCGTCCGTTTGAT" (SEQ ID N° 237), étant entendu que ladite séquence consensus n'est pas nécessairement retrouvée sous la forme d'un enchaînement continu de nucléotides, deux nucléotides contigus de ladite séquence consensus pouvant être séparés par un ou plusieurs nucléotides dans la séquence de référence.

On décrit aussi une cinquième famille d'acides nucléiques, "Famille 5", se liant au Facteur H qui est la famille d'acides nucléiques de séquences SEQ ID N° 67 à SEQ ID N° 71 et SEQ ID N° 183 à SEQ ID N° 187. On précise que les acides nucléiques de séquence SEQ ID N° 183 à SEQ ID N° 187 comprennent les séquences SEQ ID N° 67 à SEQ ID N° 71, respectivement. Les acides nucléiques de la Famille 5 sont structurellement liés entre eux au moins du fait de la présence d'une séquence consensus commune du type "TGTGGGGGGTTGGGG" (SEQ ID N° 238), étant entendu que ladite séquence consensus n'est pas nécessairement retrouvée sous la forme d'un enchaînement continu de nucléotides, deux nucléotides contigus de ladite séquence consensus pouvant être séparés par un ou plusieurs nucléotides dans la séquence de référence.

Il est ainsi décrit une famille d'acides nucléiques monobrin ayant au moins 15 nucléotides consécutifs de l'enchaînement de formule (I) défini ci-dessus.

De manière générale, un polynucléotide ayant au moins 15 nucléotides consécutifs d'un acide nucléique de référence, possède au moins 16, 17, 18, 19, 20, 21, 22, 23, 24, 25, 26, 27, 28, 29, 30, 31 , 32, 33, 34, 35, 36, 37, 38, 39, 40,41, 42, 43, 44, 45, 46, 47, 48, 49, 50, 51 , 52, 53, 54, 55, 56, 57, 58, 59, 60, 61 , 62, 63, 64, 65, 66, 67, 68, 69, 70, 71 , 72, 73, 74, 75, 76, 77, 78, 79, ou au moins 80 nucléotides consécutifs dudit acide nucléique de référence, le nombre de nucléotides consécutifs étant limité par la longueur de l'acide nucléique de référence.

Il est aussi décrit une famille d'acides nucléiques monobrin ayant au moins 40 % d'identité en nucléotides avec l'enchaînement de formule (I) défini ci-dessus.

De manière générale, un premier polynucléotide ayant au moins 40 % d'identité en nucléotides avec un second polynucléotide ou acide nucléique possède au moins 41 %, 42 %, 43 %, 44 %, 45 %, 46 %, 47 %, 48 %, 49 %, 50 %, 51 %, 52 %, 53 %, 54 %, 55 %, 56 %, 57 %, 58 %, 59 %, 60 %, 61 %, 62 %, 63 %, 64 %, 65 %, 66 %, 67 %, 68 %, 69 %, 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 % ou 100 % d'identité en nucléotides avec ledit second polynucléotide ou acide nucléique.

Le " pourcentage d'identité " entre deux séquences d'acides nucléiques, au sens de la présente invention, est déterminé en comparant les deux séquences alignées de manière optimale, à travers une fenêtre de comparaison.

La partie de la séquence nucléotidique dans la fenêtre de comparaison peut ainsi comprendre des additions ou des délétions (par exemple des " gaps ") par rapport à la séquence de référence (qui ne comprend pas ces additions ou ces délétions) de manière à obtenir un alignement optimal entre les deux séquences.

Le pourcentage d'identité est calculé en déterminant le nombre de positions auxquelles une base nucléique identique est observé pour les deux séquences comparées, puis en divisant le nombre de positions auxquelles il y a identité entre les deux bases nucléiques par le nombre total de positions dans la fenêtre de comparaison, puis en multipliant le résultat par cent afin d'obtenir le pourcentage d'identité en nucléotides des deux séquences entre elles.

L'alignement optimal des séquences pour la comparaison peut être réalisé de manière informatique à l'aide d'algorithmes connus.

De manière tout à fait préférée, le pourcentage d'identité de séquence est déterminé à l'aide du logiciel CLUSTAL W (version 1 .82) les paramètres étant fixés comme suit : (1) CPU MODE = ClustalW mp ; (2) ALIGNMENT = " full " ; (3) OUTPUT FORMAT = " aln w/numbers " ; (4) OUTPUT ORDER = " aligned " ; (5) COLOR ALIGNMENT = " no " ; (6) KTUP (word size) = " default " ; (7) WINDOW LENGTH = " default " ; (8) SCORE TYPE = " percent " ; (9) TOPDIAG = " default " ; (10) PAIRGAP = " default " ; (11) PHYLOGENETIC TREE/TREE TYPE = " none " ; (12) MATRIX = " default " ; (13) GAP OPEN = " default " ; (14) END GAPS = " default " ; (15) GAP EXTENSION = " default " ; (16) GAP DISTANCES = " default " ; (17) TREE TYPE = " cladogram " et (18) TREE GRAP DISTANCES = " hide ".

Il est aussi décrit un aptamère nucléique anti-FH qui comprend au moins 15 nucléotides consécutifs d'un polynucléotide ayant au moins 80 % d'identité en nucléotides avec l'acide nucléique de formule (I), ce qui englobe les aptamères comprenant au moins 15 nucléotides consécutifs d'un polynucléotide ayant au moins 81 %, 82 %, 83 %, 84 %, 85 %, 86 %, 87 %, 88 %, 89 %, 90 %, 91 %, 92 %, 93 %, 94 %, 95 %, 96 %, 97 %, 98 % ou 100 % d'identité en nucléotides avec ledit acide nucléique de formule (I).

Il est également décrit des aptamères nucléiques anti-FH ayant au moins 80 % d'identité en nucléotides avec l'acide nucléique de formule (I), ce qui englobe les aptamères ayant au moins 81 %, 82 %, 83 %, 84 %, 85 %, 86 %, 87 %, 88 %, 89 %, 90 %, 91 %, 92 %, 93 %, 94 %, 95 %, 96 %, 97 %, 98 % ou 100 % d'identité en nucléotides avec ledit acide nucléique de formule (I).

On décrit aussi des aptamères nucléiques anti-FH comprenant un polynucléotide ayant au moins 80 % d'identité en nucléotides avec un acide nucléique de séquence choisie parmi les séquences SEQ ID N° 3 à SEQ ID N° 116, ce qui inclut SEQ ID N° 4 à 116. Lesdits aptamères anti-FH englobent les aptamères comprenant un polynucléotide ayant au moins 81 %, 82 %, 83 %, 84 %, 85 %, 86 %, 87 %, 88 %, 89 %, 90 %, 91 %, 92 %, 93 %, 94 %, 95 %, 96 %, 97 %, 98 % ou 100 % d'identité en nucléotides avec un acide nucléique de séquence choisie parmi les séquences SEQ ID N° 3 à SEQ ID N° 116, ce qui inclut SEQ ID N° 4 à 116

On rappelle que les aptamères de séquence SEQ ID N° 117 à SEQ ID N° 232 consistent en des aptamères de formule (I) dans laquelle la séquence SEQ ID N° X consiste en la séquence SEQ ID N° 3 à SEQ ID N° 116. On rappelle aussi que les aptamères de séquence SEQ ID N° 3 à SEQ ID N° 116 consistent en des aptamère de formule (I-3) dans lequel les séquences SEQ ID N°1 et SEQ ID N°2 sont absentes.

Sans vouloir être lié par une quelconque théorie, le demandeur pense que l'acide nucléique de séquence SEQ ID N°X est une caractéristique essentielle d'un aptamère de formule (I), l'acide nucléique de séquence SEQ ID N°X conférant à l'aptamère de formule (I) l'aptitude à se lier sélectivement au Facteur H. Ceci peut être déduit notamment du fait que la totalité des acides nucléiques contenus dans le mélange d'acides nucléiques de départ, ou collection d'acides nucléiques de départ, utilisés pour sélectionner les aptamères nucléiques de l'invention, possèdent en commun les séquences SEQ ID N°1 et SEQ ID N°2 alors qu'il est montré dans les exemples que ledit mélange d'acides nucléiques de départ, collectivement, ne se lie pas au Facteur H.

Comme cela est illustré à l'exemple 6, les aptamères nucléiques selon l'invention qui sont constitués exclusivement de la séquence SEQ ID N° X ont une haute aptitude à lier le Facteur H, en particulier le Facteur H humain. Les résultats de l'exemple 6 montrent que la présence d'une séquence SEQ ID N°X dans la séquence d'un aptamère nucléique est suffisante pour conférer audit aptamère nucléique la capacité à se lier au Facteur H, en particulier au Facteur H humain. Il est ainsi montré qu'une variété d'aptamères comprenant seulement une partie de la séquence SEQ ID N° X d'un aptamère selon l'invention conservent une bonne aptitude à se lier au Facteur H, en particulier au Facteur H humain.

Il est montré qu'un aptamère nucléique comprenant tout ou partie de la séquence allant du nucléotide en position 28 jusqu'au nucléotide en position 58 de l'aptamère MaptH1.1 de séquence SEQ ID N°144 se lie avec une bonne affinité au Facteur H, en particulier au Facteur H humain. On précise que la séquence allant du nucléotide en position 28 jusqu'au nucléotide en position 58 de l'aptamère MaptH1.1 de séquence SEQ ID N° 144 est référencée comme la séquence SEQ ID N° 30 dans la présente description.

Plus précisément, il est montré qu'une variété d'aptamères nucléiques dérivés de l'aptamère nucléique parent MaptH1.1 de séquence SEQ ID N° 144, lesquels aptamères comprennent seulement une partie de la séquence SEQ ID N°30 (qui est la séquence « SEQ ID N°X » de l'aptamère de séquence SEQ ID N° 144), se lient au Facteur H, et notamment au Facteur H humain.

Il est montré que la séquence allant du nucléotide en position 13 jusqu'au nucléotide en position 48 de l'aptamère nucléique Mapt H1.1 de séquence SEQ ID N° 144, c'est-à-dire une séquence de seulement 36 nucléotides de longueur, confère à un acide nucléique qui contient cette séquence l'aptitude à se lier au Facteur H, en particulier au Facteur H humain. Cela est illustré à l'exemple 6 par la capacité de liaison au Facteur H des aptamères nucléiques MaptH1.1 N13-48 (SEQ ID N° 239), MaptH1.1 N19-58 (SEQ ID N° 240), MaptH1.1 N19-53 (SEQ ID N° 241) ; MaptH1.1 N19-48 (SEQ ID N° 242) et MaptH1.1 N22-45 (SEQ ID N° 243).

Ainsi, la présente invention concerne un aptamère nucléique se liant au Facteur H, ledit aptamère nucléique comprenant la séquence SEQ ID N° 243.

En particulier, la présente invention est relative à un aptamère nucléique de formule (III) suivante :

5'-[N1]x-[SEQ ID N° 243]-[N2]y-3' (III),

dans laquelle :

- " x " est un entier égal à 0 ou 1, et
- " y " est un entier égal à 0 ou 1,
- N1 est un acide nucléique ayant de 1 à 100 nucléotides de longueur, et
- N2 est un acide nucléique ayant de 1 à 100 nucléotides de longueur.

Au sens de l'invention, un acide nucléique ayant de 1 à 100 nucléotides de longueur englobent les acides nucléiques ayant 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57,58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 et 100 nucléotides de longueur.

Dans certains modes de réalisation d'un aptamère nucléique de formule (III), x et y sont tous les deux égaux à 1 et N et N2 sont tous les deux présents.

Dans certains modes de réalisation d'un aptamère nucléique de formule (III), x est égal à 0 et N1 est donc absent.

Dans certains modes de réalisation d'un aptamère nucléique de formule (III), y est égal à 0 et N2 est donc absent.

Dans certains modes de réalisation d'un aptamère nucléique de formule (III), x et y sont tous les deux égaux à 0 et N1 et N2 sont tous les deux absents.

Dans certains modes de réalisation d'un aptamère nucléique de formule (III), N1, lorsqu'il est présent (lorsque x est égal à 1), est un acide nucléique ayant une longueur allant de 3 à 8 nucléotides, ce qui englobe un acide nucléique ayant une longueur de 3, 4, 5, 6, 7 ou 8 nucléotides.

Dans certains modes de réalisation d'un aptamère nucléique de formule (III), N2, lorsqu'il est présent (lorsque y est égal à 1), est un acide nucléique ayant une longueur allant de 3 à 13 nucléotides, ce qui englobe un acide nucléique ayant une longueur de 3, 4, 5, 6, 7, 8, 9, 10, 12, ou 13 nucléotides.

Dans certains modes de réalisation d'un aptamère nucléique de formule (III), x et y sont tous les deux égaux à 1, N1 est un acide nucléique de 8 nucléotides de longueur et N2 est un acide nucléique de 3 nucléotides de longueur, comme pour l'aptamère nucléique MaptH1.1 N13-48 de séquence SEQ ID N° 239.

Dans certains modes de réalisation d'un aptamère nucléique de formule (III), x et y sont tous les deux égaux à 1, N1 est un acide nucléique de 3 nucléotides de longueur et N2 est un acide nucléique de 13 nucléotides de longueur, comme pour l'aptamère nucléique MaptH1.1 N19-58 de séquence SEQ ID N° 240.

Dans certains modes de réalisation d'un aptamère nucléique de formule (III), x et y sont tous les deux égaux à 1, N1 est un acide nucléique de 3 nucléotides de longueur et N2 est un acide nucléique de 8 nucléotides de longueur, comme pour l'aptamère nucléique MaptH1.1 N19-53 de séquence SEQ ID N° 241.

Dans certains modes de réalisation d'un aptamère nucléique de formule (III), x et y sont tous les deux égaux à 1, N1 est un acide nucléique de 3 nucléotides de longueur et N2 est un acide nucléique de 3 nucléotides de longueur, comme pour l'aptamère nucléique MaptH1.1 N19-48 de séquence SEQ ID N° 242.

La présente invention englobe les aptamères nucléiques se liant au Facteur H ci-dessus décrits qui sont obtenus selon le procédé général comprenant les étapes suivantes :

a) fournir un mélange, ou collection, d'acides nucléiques,
b) mettre en contact le mélange d'acides nucléiques fourni à l'étape a), ou le mélange d'acides nucléiques fourni à l'étape d) lorsque l'étape b) est répétée, avec un facteur H purifié, dans des conditions permettant la formation de complexes entre (i) des acides nucléiques dudit mélange et (ii) le facteur H purifié,
c) réaliser une séparation entre (i) le ou les acides nucléiques ayant formé des complexes avec le facteur H et (ii) les acides nucléiques n'ayant pas formé de complexes avec le facteur H, et récupérer le ou les acides nucléiques ayant formé des complexes avec le facteur H,
d) amplifier les acides nucléiques ayant formé des complexes avec le facteur H, de manière à obtenir un mélange, ou collection, d'acides nucléiques se liant au facteur H,
e) réitérer les étapes b) à d) un nombre de fois suffisant pour l'obtention d'une collection d'acides nucléiques ayant la capacité de liaison désirée au facteur H, et
f) collecter le ou les acides nucléiques se liant au facteur H obtenus à la dernière occurrence de l'étape e).

Dans des modes de réalisation avantageux, ledit procédé comprend, entre deux réitérations successives des étapes b) à d), au moins une étape de contre-sélection par élimination de ceux des acides nucléiques amplifiés à l'étape d) réalisée en dernier lieu qui se lient à des constituants d'un sérum dépourvu de Facteur H, en particulier d'un sérum

dépourvu de Facteur H humain.

Dans des modes de réalisation avantageux du procédé, on fait varier la quantité d'aptamères nucléiques anti-FH utilisée à l'étape b), ce qui inclut que l'on fait varier le rapport quantitatif aptamères anti-FH/Facteur H, dans deux occurrences distinctes de réalisation de l'étape b).

Dans des modes de réalisation avantageux, on fait varier la quantité de Facteur H purifié utilisée à l'étape b),), ce qui inclut que l'on fait varier le rapport quantitatif aptamères anti-FH/Facteur H, dans deux occurrences distinctes de réalisation de l'étape b).

D'autres modes de réalisation avantageux d'un procédé d'obtention d'aptamères nucléiques anti-FH selon l'invention sont détaillés plus loin dans la description, y compris dans les exemples.

Divers modes de réalisation spécifiques ci-dessus, qui permettent d'adapter les caractéristiques de liaison desdits aptamères nucléiques au Facteur H, sont détaillés plus loin dans la présente description.

Dans certains modes de réalisation, les aptamères nucléiques de l'invention se lient à un Facteur H choisi parmi un Facteur H plasmatique, un facteur H recombinant et un facteur H transgénique.

Dans certains modes de réalisation, les aptamères nucléiques de l'invention se lient spécifiquement à un Facteur H choisi parmi le Facteur H humain et un Facteur H non humain.

Dans des modes de réalisation préférés, les aptamères nucléiques de l'invention consistent en des aptamères désoxyribonucléotidiques, aussi appelés aptamères ADN.

Comme cela sera décrit plus loin, les aptamères nucléiques anti-FH selon l'invention peuvent être utilisés avec succès pour la fabrication de supports de chromatographie d'affinité, utiles pour séparer le Facteur H des autres protéines éventuellement présentes dans un échantillon.

Pour la réalisation de supports de chromatographie d'affinité notamment, les aptamères nucléiques anti-FH de l'invention sont préférentiellement inclus dans une structure chimique, aussi appelée " composé " dans la présente description, qui comprend notamment un moyen espaceur et, le cas échéant, un moyen d'immobilisation de l'aptamère sur un support solide.

Dans certains modes de réalisation, l'aptamère nucléique selon l'invention est inclus dans la structure d'un composé de formule (II) suivante :

$$[IMM]x\text{-}[SPAC]y\text{-}\ [APT]\ (II),$$

dans laquelle :

- [IMM] signifie un composé d'immobilisation sur un support,
- [SPAC] signifie une chaîne espaceur,
- [APT] signifie un aptamère anti-FH tel que défini dans la présente description, ce qui englobe les aptamères de formule (I),
- x est un entier égal à 0 ou 1, et
- y est un entier égal à 0 ou 1.

L'invention englobe (i) les composés de formule (II) ci-dessus pour lesquels x=0 et y=1 ainsi que (ii) les composés de formule (II) ci-dessus pour lesquels x=1 et y=0.

Dans certains modes de réalisation du composé de formule (II), [APT] consiste en un acide désoxyribonucléique dont la séquence est choisie parmi les séquences SEQ ID N° 3 à SEQ ID N° 116, les séquences SEQ ID N0 117 à 232 et les séquences SEQ ID N° 239 à 243.

La " chaîne espaceur " désignée [SPAC] dans le composé de formule (II) peut être de tout type connu. Ladite chaîne espaceur a pour fonction d'éloigner physiquement l'acide nucléique de la surface du support solide sur lequel ledit composé peut être immobilisé et de permettre une mobilité relative de l'acide nucléique par rapport à la surface du support solide sur lequel il peut être immobilisé. La chaîne espaceur limite ou évite que des encombrements stériques, dus à une trop grande proximité du support solide et de l'acide nucléique, gênent les événements de liaison entre ledit acide nucléique et des molécules de protéine de la coagulation susceptibles d'être mises en contact avec celui-ci. Dans le composé de formule (II), la chaîne espaceur est liée préférentiellement à l'extrémité 5' ou à l'extrémité 3' de l'acide nucléique aptamère.

Avantageusement la chaîne espaceur est liée à la fois à une extrémité de l'aptamère et au support solide. Cette construction avec espaceur a pour avantage de ne pas immobiliser directement l'aptamère sur le support solide. De préférence la chaîne espaceur est un oligonucléotide non spécifique ou du Polyéthylène Glycol (PEG) ou autre chaine hydrocarbonée hydrophile. Lorsque la chaîne espaceur consiste en un oligonucléotide non spécifique, ledit oligonucléotide comprend avantageusement au moins 5 nucléotides de longueur, de préférence entre 5 et 15 nucléotides de longueur.

Dans les modes de réalisation d'un composé de formule (II) dans lesquels la chaîne espaceur consiste en un Polyéthylène Glycol, ladite chaîne espaceur englobe un polyéthylène glycol de type PEG(C18), commercialisé par exemple par la Société Sigma Aldrich.

La purification de Facteur H peut être réalisée à la fois avec des composés de formule (II) comprenant une chaîne espaceur [SPAC] et avec des composés de formule (II) ne possédant pas de chaîne espaceur [SPAC].

Pour immobiliser l'aptamère sur la chaîne espaceur, l'acide nucléique peut être modifié chimiquement avec différents groupements chimiques tels que des groupements permettant d'immobiliser ledit acide nucléique de façon covalente, tels que des thiols, des amines ou tout autre groupement capable de réagir avec des groupements chimiques présents sur le support solide.

Dans le composé de formule (II) le composé [IMM] consiste en un composé choisi parmi (i) un composé capable de former une ou plusieurs liaisons covalente(s) avec le matériau de support solide et (ii) un composé capable de se lier spécifiquement sur le support solide par l'intermédiaire de liaison faibles non covalentes, y compris des liaisons hydrogènes, des forces électrostatiques ou des forces de Van der Waals.

Dans certains cas, le composé [IMM], du fait qu'il est constitué d'une chaîne d'atomes liés entre eux par des liaisons covalentes, peut se comporter comme une chaîne espaceur. Toutefois, par définition, un composé [IMM] ne peut jamais signifier une chaîne [SPAC] dans un composé de formule (II) selon l'invention. En d'autres termes, dans un composé de formule (II), la chaîne [SPAC], lorsqu'elle est présente, ne peut être liée directement au support de chromatographie, que ce soit par des liaisons covalentes ou des liaisons faibles non covalentes.

Le premier type de composé [IMM] englobe les agents de couplage bifonctionnels, comme le glutaraldéhyde, le SIAB ou encore le SMCC.

Le composé SIAB est décrit par exemple par Hermanson GT. (1996, Bioconjugate techniques, San Diego : Académie Press, pp 239-242). Le composé SIAB comprend deux groupes réactifs, respectivement un groupe iodoacétate et un groupe ester Sulfo-NHS, ces groupes réagissant respectivement sur des groupes amino et sulfhydryl.

Le composé SMCC est décrit par exemple par Samoszuk M. K. et al. (1989, Antibody, Immunoconjugates Radiopharm., 2(1) : 37-46). Le composé SMCC comprend deux groupes réactifs, respectivement un groupe ester Sulfo-NHS et un groupe maléimide, qui réagissent respectivement avec un groupe amino et un groupe sulfhydryl.

Le second type de composé [IMM] englobe la biotine, qui est capable de se lier de manière spécifique non covalente à des molécules d'avidine ou de streptavidine présentes sur le support solide. Les exemples illustrent un procédé d'obtention d'un support de détection du Facteur H, par immobilisation d'aptamères nucléiques couplés à la biotine sur un support sur lequel on a greffé chimiquement de la streptavidine. Dans ces modes de réalisation, [IMM] est constitué de la molécule de biotine.

Selon une autre alternative, le composé [IMM] consiste en un groupe amine réactif. Ledit groupe amine réactif est lié à [SPAC] lorsque [SPAC] est présent. Ledit groupe amine réactif est lié à [APT] lorsque [SPAC] est absent. Les exemples illustrent aussi un procédé d'obtention d'un support d'affinité pour la purification du Facteur H, par immobilisation d'aptamères nucléiques comprenant un groupe amine réactif sur [SPAC], qui ont été immobilisés par greffage chimique sur un support solide comprenant des groupes carboxyles activés, et plus précisément des groupes carboxyles activés par le NHS.

Une fois immobilisé sur le support solide via l'espaceur, l'aptamère anti-FH est avantageusement modifié à son extrémité libre (extrémité non liée à l'espaceur) grâce à, et sans s'y limiter, un nucléotide chimiquement modifié (tel que 2'omethyl ou 2'fluoropyrimidine, 2' ribopurine, phosphoramidite), un nucléotide inversé ou un groupement chimique (PEG, polycations, cholestérol). Ces modifications permettent de protéger l'aptamère anti-FH des dégradations enzymatiques.

Le support solide peut être une colonne de chromatographie d'affinité composée d'un gel dérivé de l'agarose, de la cellulose ou d'un gel synthétique tel qu'un dérivé acrylamide, méthacrylate ou polystyrène, une puce telle qu'une puce adaptée à la résonance plasmonique de surface, une membrane telle qu'une membrane polyamide, polyacrylonitrile ou polyester, une bille magnétique ou paramagnétique.

La présente invention est également relative à un complexe entre : (a) un aptamère nucléique choisi parmi un acide nucléique de formule (I), et un composé de formule (II), et (b) le Facteur H.

La présente invention a aussi pour objet un support pour l'immobilisation du Facteur H, caractérisé en ce qu'il comprend un matériau support solide sur lequel sont greffées une pluralité de molécules consistant chacune en, ou comprenant chacune, un aptamère nucléique, lesdites molécules étant choisies parmi (a) un acide nucléique de formule (I), et (b) un composé de formule (II).

Le support ci-dessus peut être utilisé pratiquement dans toutes les applications pour lesquelles on cherche à immobiliser le Facteur H, ce qui englobe les applications à des fins de purification du Facteur H et les applications à des fins de détection du Facteur H.

La réalisation de supports sur lesquels sont immobilisés des aptamères nucléiques de l'invention se liant spécifiquement au facteur H sont largement illustrés dans les exemples, dans lesquels les aptamères de l'invention sont utilisés notamment comme agents de capture, utilisables pour la purification ou pour la détection du Facteur H, par

exemple le facteur H humain, dans des échantillons.

La présente invention concerne donc aussi un procédé pour l'immobilisation du Facteur H sur un support, comprenant une étape au cours de laquelle on met en contact un échantillon comprenant du Facteur H avec un support solide sur lequel a été préalablement immobilisée une substance choisie parmi un acide nucléique de formule (I), et un composé de formule (II). Ledit procédé peut comprendre, selon les objectifs techniques poursuivis, une étape additionnelle de récupération des molécules de Facteur H immobilisées, complexées avec les molécules d'acides nucléiques de formule (I). L'étape additionnelle de récupération du Facteur H, consiste préférentiellement en une étape de mise en contact des complexes de Facteur H avec les acides nucléiques de formule (I) avec un agent chélateur des cations métalliques, tel que l'EDTA.

Pour la réalisation de procédés de purification de protéines par chromatographie d'affinité en utilisant des supports solides sur lesquels sont immobilisés les aptamères d'intérêt, l'homme du métier peut se référer aux travaux décrits par Romig et al. (1999, J Chomatogr B Biomed Sci Apl, Vol. 731 (2) : 275-284).

De manière générale, les supports solides sur lesquels peuvent être immobilisés les aptamères de l'invention englobe tout type de support ayant la structure et la composition retrouvée communément pour les supports de filtre, de support de silicium pour des puces, des membranes, etc. Les supports solides englobent notamment les résines, les résines pour colonne de chromatographie d'affinité, les billes de polymères, les billes magnétiques, etc. Les supports solides englobent aussi notamment les matériaux à base de verre ou de métal, tels que l'acier, l'or, l'argent, l'aluminium, le cuivre, le silicium, le verre, la céramique. Les supports solides englobent aussi notamment des matériaux polymères, tels qu'un polyéthylène, un polypropylène, un polyamide, un fluorure de polyvinylidène, et des combinaisons de ceux-ci.

Le support solide peut être revêtu avec un matériau facilitant l'attachement, la fixation, la formation de complexes, l'immobilisation ou l'interaction avec les aptamères. Dans certains modes de réalisation, le support solide est une lame de verre dont la surface est revêtue d'une couche d'or, d'une couche ayant subi un traitement par carboxyméthylation, d'une couche de dextran, de collagène, d'avidine, de streptavidine, etc. De cette manière, les aptamères selon l'invention peuvent être immobilisés sur le support solide par l'intermédiaire d'un revêtement d'attachement, comme par exemple décrit ci-dessus, soit par réaction chimique avec création de liaisons covalentes, soit par association par des liaisons non covalentes telles que des liaisons hydrogène, des forces électrostatiques, des forces de Van der Waals, etc.

Les exemples décrivent des modes de réalisation de supports solides sur lesquels sont immobilisés, par des liaisons non covalentes, les aptamères de l'invention. Dans les exemples, on décrit notamment des supports solides consistant en une lame de verre revêtue d'une couche de molécules de streptavidine, et des aptamères de l'invention conjugués à une molécule de biotine qui sont immobilisé sur le support par association non covalente biotine/streptavidine.

Dans les exemples, on décrit aussi des supports solides consistant en un matériau de polystyrène revêtu d'une couche de molécules de streptavidine, et des aptamères de l'invention conjugués à une molécule de biotine qui sont immobilisés sur le support par association non covalente biotine/streptavidine.

Dans certains modes de réalisation, les aptamères de l'invention peuvent être immobilisés sur un support solide adapté pour la chromatographie d'affinité, l'électrochromatographie et l'électrophorèse capillaire, comme décrit par exemple par Ravelet et al. (2006, J Chromatogr A, Vol. 1 17(1) : 1 -10), Connor et al. (2006, J Chromatogr A, Vol. 1 1 1 (2) : 1 15-1 19), Cho et al. (2004, Electrophoresis, Vol. 25 (21 - 22) : 3730-3739) ou encore Zhao et al. (2008, Anal Chem, Vol. 80(10) : 3915-3920).

Un aptamère de formule (I) se liant spécifiquement au Facteur H, ou un composé de formule (II), peut être aussi avantageusement utilisé comme agent de capture de Facteur H, dans des procédés et dispositifs de détection ou de diagnostic.

Selon encore un autre aspect, la présente invention concerne aussi un procédé pour détecter la présence de Facteur H dans un échantillon comprenant les étapes suivantes :

a) mettre en contact (i) un acide nucléique de formule (I), ou un composé de formule (II) ou un support solide sur lequel sont immobilisés une pluralité de molécules dudit acide nucléique ou dudit composé, avec (ii) ledit échantillon; et

b) détecter la formation de complexes entre (i) ledit acide nucléique de formule (I), ou ledit composé de formule (II) ou ledit support et (ii) le Facteur H.

Pour la réalisation d'un procédé de détection selon l'invention, le support solide utilisé peut être un support solide choisi parmi les supports solides décrits précédemment en relation avec le procédé de purification du Facteur H.

Pour la réalisation d'un procédé ou d'un dispositif de détection de Facteur H, l'homme du métier peut se référer notamment aux techniques décrites dans la demande de brevet européen n° EP 1972 693, la demande PCT n° WO 2008/038696, la demande PCT n° WO 2008/025830, ou encore la demande PCT n° WO 2007/0322359.

Dans certains modes de réalisation, l'étape b) de détection de la formation de complexes entre (i) ledit acide

nucléique ou ledit support solide et (ii) le facteur H peut être réalisé par mesure du signal de résonance plasmonique de surface, comme cela est décrit dans les exemples.

Dans certains autres modes de réalisation, l'étape b) de détection de la formation de complexes entre (i) ledit acide nucléique ou ledit support solide et (ii) le facteur H peut être réalisée par la mise en contact desdits complexes éventuellement formés avec un ligand spécifique du Facteur H, ledit ligand étant détectable. Par exemple, on peut utiliser comme ligand détectable du Facteur H, des anticorps monoclonaux ou polyclonaux anti-Facteur H marqués avec une enzyme, en l'occurrence la peroxydase de raifort, comme cela est conventionnellement utilisé dans les tests de type ELISA. Comme anticorps spécifiques du facteur H on peut utiliser notamment les anticorps polyclonaux anti-Facteur H tels que ceux commercialisés par la Société Enzo Life Sciences (Lyon, France) ou bien des anticorps monoclonaux anti-Facteur H tels que ceux commercialisés par la Société Abbiotec.

De manière surprenante, il est montré selon l'invention que l'on peut fabriquer un support d'affinité tel que défini dans la présente description en utilisant, comme aptamères nucléiques anti-FH, des aptamères ADN, pourtant considérés dans l'état de la technique comme des acides nucléiques ligands dont la mise en œuvre est malaisée, et dont la spécificité pour la protéine cible est moindre que la spécificité de la molécule d'ARN de séquence correspondante. Notamment, il est admis dans l'état de la technique que le ADN ligands ont une flexibilité moindre que l'ARN correspondant et qu'en conséquence sont moins aptes que les ARN ligands à subir des changements de conformation. On rappelle que lorsqu'un aptamère nucléique se lie à la protéine cible, il se produit un changement de conformation. Il a aussi été décrit que plus le changement de conformation de l'aptamère nucléique est rapide et plus l'affinité dudit aptamère nucléique pour la protéine cible est élevée (Michaud et al., 2003, Anal Chem, Vol. 76 : 1015-1020 ; Brumbt et al., 2005, Anal Chem, Vol. 77 : 1993-1998).

Ainsi, dans certains modes de réalisation d'un support d'affinité selon l'invention, les aptamères anti-FH consistent en des aptamères ADN.

En conséquence, dans certains modes de réalisation d'un support d'affinité selon l'invention, lesdits acides nucléiques immobilisés, le cas échéant inclus dans la structure d'un composé de formule (I), consistent en des acides désoxyribonucléiques.

Dans certains autres modes de réalisation d'un support d'affinité selon l'invention, lesdits acides nucléiques consistent, pour une première partie d'entre eux, en des acides désoxyribonucléiques, et pour la partie restante en des acides ribonucléiques.

Un autre avantage des aptamères nucléiques concerne leur facilité de fabrication, comparée aux difficultés de synthèse des aptamères ARN, ainsi que leur prix de revient qui est significativement plus réduit que le prix de revient d'un aptamère ARN.

Ces modes de réalisation spécifiques sont illustrés dans les exemples. La présente invention a également pour objet un dispositif de chromatographie d'affinité pour la purification du Facteur H, comprenant un conteneur dans lequel est disposé une quantité adaptée d'un support d'affinité tel que défini dans la présente description.

Des formes variées de conteneurs pour supports de chromatographie sont connus dans l'état de la technique et sont englobés par la signification du terme " conteneur " ci-dessus.

Les caractéristiques importantes d'un tel conteneur englobent la présence d'un moyen d'alimentation du dispositif de chromatographie d'affinité en échantillon de départ et d'un moyen de sortie du liquide après sa mise en contact avec le support d'affinité.

La présente invention a également trait à un procédé pour la purification de Facteur H comprenant les étapes suivantes :

a) mettre en contact un échantillon contenant un Facteur H avec un support d'affinité tel que défini ci-dessus, afin de former des complexes entre (i) les aptamères nucléiques immobilisés sur ledit support d'affinité et (ii) ledit Facteur H,
b) libérer le Facteur H à partir des complexes formés à l'étape a), et
c) récupérer le Facteur H sous forme purifiée.

Les échantillons de départ à partir desquels est purifié le Facteur H avec un support d'affinité selon l'invention, englobent tout type de solution liquide dans laquelle le Facteur H est en suspension ou est solubilisé. Des modes de réalisation spécifiques de tels échantillons, en particulier en relation avec le procédé de purification décrit ci-après, seront définis ultérieurement dans la présente description.

Dans certains modes de réalisation préférés, ledit échantillon contient du Facteur H humain. Avantageusement, dans ces modes de réalisation, l'échantillon contenant du Facteur H humain consiste en un échantillon liquide qui contient ladite protéine d'intérêt, y compris un échantillon liquide comprenant ledit Facteur H et qui est susceptible de contenir aussi des molécules de Facteur H homologue d'un mammifère non humain. Dans certains modes de réalisation du procédé de purification ci- dessus, ledit échantillon consiste en une solution biologique, tels qu'un fluide corporel, une cellule, un broyat cellulaire, un tissu, un broyat tissulaire, un organe ou un organisme entier.

Dans certains modes de réalisation du procédé de purification ci-dessus, ledit échantillon consiste en une solution biologique liquide provenant d'un animal tel que du sang, un dérivé du sang (dérivé sanguin), du lait de mammifère ou un dérivé du lait de mammifère.

Ledit échantillon peut consister en du plasma, du cryoprécipité du plasma, du lait clarifié ou leurs dérivés.

Dans des modes de réalisation particulièrement préférés du procédé de purification ci- dessus, ledit échantillon provient d'un animal transgénique pour le Facteur H humain.

Avantageusement la solution est du lait de mammifère ou un dérivé du lait de mammifère transgénique pour ladite protéine d'intérêt humaine. Au sens de l'invention, les animaux transgéniques englobent (i) les mammifères non humains tels que les vaches, les chèvres, les lapins, les porcs, les singes, les rats ou les souris, (ii) les oiseaux ou encore (iii) les insectes tels que les moustiques, les mouches ou les vers à soie. Dans certains modes de réalisation préférés, l'animal transgénique pour la protéine d'intérêt humaine est un mammifère transgénique non humain, de manière tout à fait préférée une lapine transgénique pour ladite protéine d'intérêt humaine. Avantageusement, le mammifère transgénique produit ladite protéine d'intérêt humaine recombinante dans ses glandes mammaires, du fait de l'insertion dans son génome d'une cassette d'expression comprenant acide nucléique codant ladite protéine d'intérêt qui est placé sous le contrôle d'un promoteur spécifique permettant l'expression de la protéine transgénique dans le lait dudit mammifère transgénique.

Une méthode de production du Facteur H humain dans le lait d'un animal transgénique peut comprendre les étapes suivantes : une molécule d'ADN comprenant un gène codant le facteur H humain, ledit gène étant sous le contrôle d'un promoteur d'une protéine sécrétée naturellement dans le lait (tels que le promoteur de la caséine, de la béta-caséine, de la lactalbumine, de la béta-lactoglobuline ou le promoteur WAP) est intégrée dans un embryon d'un mammifère non humain. L'embryon est ensuite placé dans une femelle de mammifère de la même espèce. Une fois que le mammifère issu de l'embryon s'est suffisamment développé, la lactation du mammifère est induite, puis le lait collecté. Le lait contient alors la protéine humaine recombinante d'intérêt. Un exemple de procédé de préparation de protéine dans le lait d'une femelle de mammifère autre que l'être humain est donné dans le document EP 0 527 063, dont l'enseignement peut être repris pour la production de la protéine de l'invention. Un plasmide contenant le promoteur WAP (Whey Acidic Protein) est fabriqué par introduction d'une séquence comportant le promoteur du gène WAP, ce plasmide étant réalisé de manière à pouvoir recevoir un gène étranger placé sous la dépendance du promoteur WAP. Le plasmide contenant le promoteur et le gène codant pour la protéine de l'invention sont utilisés pour obtenir des lapines transgéniques, par micro-injection dans le pronucléus mâle d'embryons de lapines. Les embryons sont ensuite transférés dans l'oviducte de femelles hormonalement préparées. La présence des transgènes est révélée par la technique de Southern à partir de l'ADN extrait des lapereaux transgéniques obtenus. Les concentrations dans le lait des animaux sont évaluées à l'aide de tests radio-immunologiques spécifiques.

D'autres documents décrivent des procédés de préparation de protéines dans le lait d'une femelle de mammifère autre que l'homme. On peut citer, sans s'y limiter les documents US 7,045,676 (souris transgénique) et EP 1 739 170. (production de facteur von Willebrand dans un mammifère transgénique).

Le procédé de purification de l'invention est également parfaitement adapté à l'obtention de Facteur H humain purifié à partir d'un échantillon de plasma sanguin humain, ou d'une fraction de plasma sanguin humain, par exemple la fraction cryoprécipitée de plasma sanguin humain.

Dans certains modes de réalisation du procédé de purification ci-dessus, le Facteur H est le facteur H humain.

Dans certains modes de réalisation du procédé de purification ci-dessus, l'échantillon comprend au moins un Facteur H non-humain.

Dans certains modes de réalisation du procédé de purification ci-dessus, l'échantillon de départ peut consister en le matériel brut, soit l'échantillon de plasma sanguin humain, ou une fraction de celui-ci, soit le fluide corporel d'un mammifère non humain transgénique pour le Facteur H, et qui contient le facteur H à purifier. Les fluides corporels d'un mammifère non humain transgénique englobent le lait ou une fraction du lait, par exemple une fraction délipidée du lait ou bien encore une fraction appauvrie en micelles de caséine.

Toutefois, le mode de réalisation ci-dessus n'est pas le mode de réalisation privilégié du procédé de purification de l'invention, notamment en raison du risque de colmatage du support d'affinité par les nombreuses protéines présentes dans l'échantillon brut de départ. Dans des modes de réalisation préférés, ledit échantillon de départ consiste en une solution liquide contenant le facteur H en suspension dans ladite solution, ladite solution liquide consistant en un produit intermédiaire généré au cours d'un procédé de purification multi-étapes du Facteur H.

A titre illustratif, pour un procédé de purification du Facteur H à partir d'un fluide corporel d'un mammifère non humain transgénique pour ladite protéine, l'échantillon de départ peut consister en un éluat d'une étape de chromatographie d'interaction hydrophobe, telle qu'une étape de chromatographie dans laquelle on utilise un support chromatographique du type MEP HyperCel(R).

De la même manière, pour un procédé de purification du Facteur H à partir de plasma humain, l'échantillon de départ peut consister en une fraction plasmatique déjà enrichie en Facteur H.

A titre illustratif, on peut utiliser comme matériau de départ le surnageant d'un cryoprécipité de plasma sanguin

éventuellement mélangé avec une fraction plasmatique non retenue sur une chromatographie échangeuse d'anions, du type DEAE-Sephadex. On peut aussi utiliser comme matériau de départ le surnageant d'une précipitation éthanolique du plasma sanguin. Egalement à titre illustratif, on peut utiliser comme matériau de départ l'éluat d'une chromatographie d'affinité d'un échantillon plasmatique sur un support, en particulier un support d'agarose, sur lequel est greffé de l'héparine ou des dérivés ou mimétiques de l'héparine, qui ont la propriété de retenir le Facteur H et l'antithrombine III (ATIII). On précise que le Facteur H et l'AT III sont élués séparément, ce qui permet l'obtention d'une fraction d'éluat enrichie en Facteur H qui peut être utilisé comme produit de départ dans un procédé de purification du Facteur H comprenant une étape de formation de complexes entre ledit Facteur H et des aptamères anti-FH selon l'invention.

De manière générale, les conditions d'utilisation du support d'affinité pour réaliser le procédé de purification de l'invention sont très similaires aux conditions d'utilisation conventionnelles d'un support de chromatographie classique, par exemple du type support d'immuno-affinité sur lequel sont immobilisé des anticorps ligands. L'homme du métier peut par exemple se référer à l'ouvrage de Bailon et al. (Pascal Bailon, George K. Ehrlich, Wen-Jian Fung and Wolfgang Berthold, An Overview of Affinity Chromatography, Humana Press, 2000).

Toutefois, comme cela sera détaillé dans la suite de la description, les conditions de l'étape c) d'élution, étape au cours de laquelle le Facteur H est libéré à partir des complexes formés à l'étape a), sont très avantageuses, pour la purification du facteur H.

A l'étape a), un volume approprié de l'échantillon à purifier est mis en contact avec le support d'affinité. Des complexes sont formés entre (i) les aptamères anti-FH immobilisés sur ledit support d'affinité et (ii) le Facteur H contenu dans l'échantillon à purifier.

Dans certains modes de réalisation, les caractéristiques du procédé d'obtention des aptamères sont adaptées de manière à ce que les conditions de liaison des aptamères anti-FH au Facteur H soient favorisées en présence de magnésium. Ainsi, dans certains modes de réalisation de l'étape a), on utilise une solution tampon qui comprend du magnésium, préférentiellement sous la forme d'un sel de magnésium, de préférence un chlorure de magnésium ($MgCl_2$).

L'affinité pour le Facteur H d'un support de chromatographie sur lequel est immobilisé un aptamère anti-FH selon l'invention peut être substantiellement accrue lorsqu'on utilise à l'étape a) une solution tampon comprenant du chlorure de magnésium, par exemple une solution tampon de Tris-HCI 50mM à pH 7,5, 150 mM NaCl et 10 mM MgC12 par comparaison avec une solution tampon identique mais dépourvue de chlorure de magnésium.

Ainsi, dans certains modes de réalisation de l'étape a) du procédé de purification, on utilise une solution tampon comprenant une concentration finale en $MgCl_2$ d'au moins 1 mM. A l'étape a), on utilise avantageusement une solution tampon comprenant une concentration finale de MgC12 d'au plus 50 mM, ce qui englobe au plus 20 mM.

Dans des modes de réalisation préférés du procédé, on procède, à la fin de l'étape a), et préalablement à l'étape b) décrite en détail plus loin, à une ou plusieurs étapes de lavage du support d'affinité de manière à éliminer les substances qui ne sont pas retenues de manière spécifique, par exemple les substances simplement adsorbées sur ledit support. On peut utiliser un tampon de lavage conventionnel, bien connu de l'homme du métier.

Toutefois, dans certains modes de réalisation de la ou des étape(s) de lavage, on peut utiliser un tampon de lavage comprenant du NaCl, et/ou de l'éthylène glycol, et/ou du propylène glycol, et/ou de l'éthanol.

Dans certains modes de réalisation, l'utilisation d'une solution de lavage comprenant du NaCl n'entraîne pas d'altération dans la liaison spécifique du Facteur H cible aux aptamères anti-FH immobilisés. On a montré en particulier qu'une concentration finale de NaCl de 3M ne perturbe pas ladite liaison spécifique.

Ainsi, dans certains modes de réalisation de la ou des étapes de lavage préalables à l'étape b), on utilise une solution de lavage ayant une concentration finale en NaCl d'au moins 0,5 M, ce qui inclut au moins 1 M, 1 ,5 M, 2 M, 2,5 M et 3,0 M. La concentration finale de NaCl est avantageusement d'au plus 4M.

On a aussi montré que la capacité des aptamères anti-FH de l'invention à se lier au Facteur H cible n'est pas altérée lorsqu'on soumet les complexes formés entre les aptamères immobilisés et ledit Facteur H cible à un environnement de haute force ionique, ce qui est un résultat tout à fait inattendu. On rappelle en effet que l'on a recours couramment à un tampon de haute force ionique comme tampon pour l'élution de substances préalablement complexées à des ligands immobilisés d'un support d'affinité, y compris un support d'affinité comprenant des aptamères immobilisés.

Dans certains modes de réalisation, un ou plusieurs des aptamères anti-FH selon l'invention peuvent posséder des caractéristiques spécifiques et inattendues, concernant l'absence d'effet d'une haute force ionique sur leur capacité à se lier au Facteur H.

Dans certains modes de réalisation du procédé, l'utilisation d'une solution de lavage comprenant un alkylène glycol n'entraîne pas d'altération dans la liaison spécifique du Facteur H aux aptamères anti-FH immobilisés.

L'utilisation d'une solution de lavage comprenant de l'éthylène glycol n'entraîne pas d'altération dans la liaison spécifique du Facteur H aux aptamère anti-FH immobilisés.

Ainsi, dans certains modes de réalisation de la ou des étapes de lavage préalables à l'étape b), par exemple avec certains des aptamères nucléiques de l'invention, on peut utiliser une solution de lavage ayant une concentration finale en éthylène glycol d'au moins 0,5 M, ce qui inclut au moins 1 M, et 1 ,5 M. Avantageusement, on utilise une solution de lavage ayant une concentration finale en éthylène glycol d'au plus 3M.

Egalement, l'utilisation d'une solution de lavage comprenant du propylène glycol peut, avec au moins certains aptamères nucléiques de l'invention, ne pas entraîner d'altération dans la liaison spécifique du Facteur H aux aptamère anti-FH immobilisés.

Ainsi, dans certains modes de réalisation de la ou des étapes de lavage préalables à l'étape b), on pourrait avoir recours à une solution de lavage ayant une concentration finale en propylène glycol d'au moins 10 % (v/v), ce qui inclut au moins 15 %, 20 %, 25 %, 30 %, 35 %, 40 %, 45 % et 50 %. Avantageusement, on utilise une solution de lavage ayant une concentration finale en propylène glycol d'au plus 70 % (v/v).

Egalement, avec certains aptamères nucléiques de l'invention, l'utilisation d'une solution de lavage comprenant de l'éthanol pourrait ne pas entraîner d'altération dans la liaison spécifique du Facteur H aux aptamères anti-FH immobilisés.

Ainsi, dans certains modes de réalisation de la ou des étapes de lavage préalables à l'étape b), on pourrait utiliser une solution de lavage ayant une concentration finale en éthanol d'au moins 1 % (v/v), ce qui inclut au moins 1, 5 %, 2,0 %, 2,5 %, 3,0 %, 3,5 %, 4,0 %, 4,5 %, 5,0 %, 5,5 %, 6,0 %, 6,5 %, 7,0 %, 7,5 %, 8,0 %, 9,0 %, 9,5 % et 10,0 %. Avantageusement, on utilise dans ce cas une solution de lavage ayant une concentration finale en éthanol d'au plus 20,0 %.

Un tampon de lavage tel que défini ci-dessus entraîne des conditions drastiques d'élimination des substances qui ne sont pas retenues de manière spécifique sur les aptamères, tout en préservant la liaison spécifique du Facteur H aux aptamères immobilisés sur le support d'affinité. On rappelle qu'un tel avantage technique lié à l'élimination exclusive ou quasi-exclusive des substances non liées spécifiquement aux ligands immobilisés sur le support d'affinité ne peut être aisément réalisé avec un support d'affinité sur lequel sont immobilisés des anticorps.

L'étape b) consiste en une étape d'élution du Facteur H ayant formé des complexes avec les aptamères nucléiques anti-FH au cours de l'étape a). Un avantage spécifique attendu du procédé de purification ci-dessus est la possibilité de réaliser l'étape d'élution par mise en contact des complexes formés entre (i) les aptamères nucléiques anti-FH immobilisés sur ledit support d'affinité et (ii) le Facteur H, avec un agent chélateur des ions divalents, comme l'EDTA.

Cet avantage technique, qui est rendu possible, grâce aux caractéristiques du support d'affinité de l'invention, permet l'élution du facteur H sans nécessiter un quelconque recours à l'utilisation de conditions drastiques d'élution, susceptibles de dénaturer, au moins partiellement, le facteur H. Lesdites condition drastiques qui sont évitées englobent l'utilisation d'un pH acide pour l'étape d'élution, ce qui est couramment pratiqué pour les procédés de purification de protéines sur les supports d'affinité connus, et tout particulièrement sur les supports d'affinité comprenant des anticorps immobilisés.

Ainsi, dans certains modes de réalisation du procédé de purification ci-dessus, l'étape b) est réalisée par mise en contact du support d'affinité avec un tampon d'élution contenant un agent chélateur des ions divalents, de préférence l'EDTA.

A titre illustratif, le tampon d'élution peut contenir une concentration finale d'EDTA d'au moins 1 mM et d'au plus 500 mM.

L'expression " au moins 1 mM de EDTA" englobe au moins 2 mM, 3 mM, 4 mM, 5 mM, 6 mM, 7 mM, 8 mM, 9 mM, 10 mM, 15 mM, 20 mM ou au moins 30 mM de EDTA.

A l'étape c), on récupère le facteur H sous forme purifiée en collectant le liquide d'éluat obtenu à la fin de l'étape b).

A la fin de l'étape c), on obtient une composition liquide purifiée de la protéine de la coagulation d'intérêt. Ladite composition liquide purifiée peut ensuite être traitée de manière appropriée, selon toute technique connue de conditionnement et de conservation des protéines, y compris par flaconnage direct ou après dilution avec une solution adaptée, ou encore par lyophilisation, préférentiellement dans des conditions stériles et apyrogènes, puis conservation dans des conditions appropriées, à température ambiante, à 4 °C ou bien à basse température, selon le type de conditionnement choisi.

Comme cela a déjà été mentionné précédemment dans la présente description, le support d'affinité de l'invention peut, avec les cycles successifs d'utilisation pour la purification du Facteur H, subir une réduction de sa capacité d'absorption, par exemple du fait que l'étape c) d'élution ne permet pas systématiquement de libérer la totalité des molécules de protéine de la coagulation, ce qui réduit le nombre de sites aptamères libres pour les cycles ultérieurs de purification.

Comme pour la totalité des supports de chromatographie connus, il est donc nécessaire, à des moments appropriés, de réaliser une étape de régénération du support d'affinité, afin de libérer la totalité des molécules de Facteur H dudit support, et d'éliminer toute substance pouvant être liée au matériau solide du support d'affinité, en général par fixation non spécifique.

Ainsi, dans certains modes de réalisation, le procédé de purification de l'invention comprend une étape additionnelle d) de régénération du support d'affinité, par mise en contact dudit support d'affinité avec une solution de régénération.

Des tampons variés pour la régénération de support de chromatographie, en particulier de supports de chromatographie d'affinité sont bien connus par l'homme du métier, qui peuvent être utilisés à l'étape d) du procédé. L'homme

du métier peut se référer par exemple à l'ouvrage de Mohr et al. (Affinity Chromatography: Practical and Theoretical Aspects, Peter Mohr, Klaus Pommerening, Edition: illustrated, CRC Press, 1985).

A titre illustratif, l'étape d) de régénération du support d'affinité peut être réalisée par mise en contact dudit support avec une solution tampon de Tris 20 mM, Polyéthylène Glycol 5 %, NaCl 1 M, par exemple à pH 7,5.

Sans vouloir être lié par une quelconque théorie, le demandeur pense que le procédé de purification ci-dessus permet l'obtention d'un Facteur H cible à un très haut degré de pureté, éventuellement à une degré de pureté supérieur à 99,95 % en poids, par rapport au poids total des protéines contenues dans le produit final purifié.

Un autre avantage du procédé de purification ci-dessus, en particulier dans les modes de réalisation dans lesquels l'échantillon de départ consiste en un échantillon comprenant le Facteur H sous forme recombinante en mélange avec des protéines produites naturellement par le mammifère transgénique non humain, est que la composition finale comprenant la protéine humaine recombinante d'intérêt à haut degré de pureté est sensiblement exempte de protéines provenant dudit mammifère transgénique, et en particulier sensiblement exempte de protéines dudit mammifère, homologues au facteur H humain recombinant.

La présente invention est également relative à un aptamère nucléique se liant spécifiquement au facteur H, dont la capacité à se lier au Facteur H cible n'est pas altérée par une exposition à un environnement drastique, y compris un environnement provoquant l'inactivation ou la destruction physique des protéines, ou bien l'inactivation ou la destruction de micro-orgamismes, tels que les virus, les bactéries, les champignons ou les algues. Avantageusement, l'invention concerne un aptamère nucléique se liant sélectivement au Facteur H et dont la capacité à se lier au Facteur H n'est pas altérée par un environnement apte à inactiver, y compris détruire, les virus, tel qu'une solution fortement concentrée en NaOH. Une solution fortement concentrée en NaOH englobe une solution de NaOH supérieure à 0,05 M. Dans ce contexte spécifique, une solution de Na OH supérieure à 0,05 M englobe les solutions de Na OH supérieures à 0,1 M, 0,2 M, 0,3 M, et 0,4 M. Il peut s'agir par exemple d'une solution de NaOH d'environ 0,5 M.

Ces propriétés peuvent avantageusement être utilisées pour laver très efficacement un support d'affinité sur lequel est immobilisé un aptamère nucléique selon l'invention, ce qui permet d'obtenir une meilleure élimination des contaminants liés à la colonne de façon non spécifique. L'amélioration de l'efficacité du lavage permet d'augmenter la pureté du Facteur H que l'on cherche à purifier.

En particulier, ces propriétés peuvent être avantageusement utilisées pour sanitiser un support d'affinité, c'est-à-dire procéder à une étape d'inactivation ou de destruction des micro-organismes, et notamment des virus, susceptibles de contaminer ledit support d'affinité, sans altérer sensiblement la capacité dudit support d'affinité à lier sélectivement le Facteur H cible. Ces propriétés sont essentielles pour des supports d'affinité qui sont utilisés dans des procédés de purification du Facteur H destiné à une utilisation dans le domaine médical, humain ou vétérinaire.

Comme cela a été décrit précédemment dans la présente description et est aussi illustré dans les exemples, on peut dissocier les complexes formés entre les aptamères nucléiques de l'invention et le Facteur H par mise en contact desdits complexes avec une solution comprenant un chélateur d'ions divalents, par exemple de l'EDTA. Ainsi, selon une autre de leurs caractéristiques, les aptamères nucléiques de l'invention consistent en des aptamères nucléiques permettant la formation de complexes avec le facteur H cible, lesdits complexes pouvant être dissociés, avec libération du facteur H cible, par mise en contact desdits complexes avec un milieu comprenant un chélateur d'ions divalents, par exemple de l'EDTA.

Comme déjà précisé précédemment, un complexe entre un aptamère nucléique de l'invention et le Facteur H peut être dissocié en mettant en contact ledit complexe avec un milieu, y compris une solution tampon, comprenant une concentration finale d'EDTA d'au moins 1 mM et d'au plus 500 mM.

La présente invention a aussi pour objet une composition purifiée de Facteur H humain recombinant comprenant au moins 99,9 % en poids de Facteur H humain recombinant et qui est sensiblement exempte de protéines non humaines. La présente invention est aussi relative à une composition purifiée de Facteur H humain recombinant comprenant au moins 99,9 % en poids dudit Facteur H humain recombinant et au plus 0,1 % en poids de protéines non humaines, les pourcentages en poids étant exprimés par rapport au poids total en protéines de ladite composition purifiée.

Dans la composition purifiée ci-dessus, " au moins 99,9 % " englobe au moins 99,91 %, 99,92 %, 99,93 %, 99,94 %, 99,95 %, 99,96 %, 99,97 %, 99,98 % et 99,99 %.

Dans la composition purifiée ci-dessus, " au plus 0,1 % " englobe au plus 0,09 %, 0,08 %, 0,07 %, 0,06 %, 0,05 %, 0,04 %, 0,03 %, 0,02 % et 0,01 %.

La présente invention est aussi relative à une composition purifiée telle que définie ci- dessus, utilisable comme médicament.

L'invention concerne encore une composition pharmaceutique comprenant une composition purifiée de Facteur H humain recombinant telle que définie ci-dessus, en combinaison avec un ou plusieurs excipients pharmaceutiquement acceptables.

L'invention a aussi trait à une composition purifiée telle que définie ci-dessus pour le traitement des troubles liés à un déficit en Facteur H.

L'invention est également relative à l'utilisation d'une composition purifiée telle que définie ci-dessus pour la

fabrication d'un médicament pour le traitement des troubles liés à un déficit en Facteur H.

Les troubles liés à un déficit en Facteur H englobent le Syndrome hémolytique Urémique (SHU), y compris la forme typique du SHU et la forme atypique du SHU, la glomérulonéphrite de type II membranoproliférative.

## Modes de réalisation particuliers de supports d'affinité anti-FH

Dans des modes de réalisation spécifiques de l'invention, on utilise des supports d'affinité qui sont préparés par greffage chimique des aptamères anti-FH sur un support solide comprenant des fonctions acides carboxyliques activées, dans des conditions spécifiques de greffage permettant à la fois un haut rendement de greffage et le maintien de l'intégrité des aptamères anti-FH greffés.

Dans ces modes de réalisation, un support d'affinité selon l'invention est obtenu selon un procédé un procédé d'immobilisation d'aptamères anti-FH comprenant au moins une fonction amine réactive, par greffage sur un support solide présentant des fonctions acides carboxyliques activées, comprenant une étape de couplage covalent desdits acides nucléiques sur ledit support solide à un pH inférieur à 5.

Selon l'invention, un aptamère anti-FH comprend une fonction amine réactive lorsque ledit aptamère possède une fonction amine accessible au solvant et apte à réagir avec un groupe réactif approprié porté par une autre entité moléculaire. Une telle fonction amine englobe en particulier les amines primaires.

Des acides nucléiques possédant une fonction amine réactive sont bien connus dans l'état de la technique et sont classiquement utilisés pour leur couplage chimique à des supports ou à des substances marqueurs. Classiquement, il s'agit d'acides nucléiques qui ont été modifiés par l'ajout d'une fonction amine à leur extrémité 3' ou à leur extrémité 5'. Le plus fréquemment, la fonction amine est ajoutée à l'extrémité 5' de l'acide nucléique où son incorporation est plus aisée comme étape finale d'un procédé de synthèse d'un polynucléotide. Dans certains modes de réalisation, la fonction amine réactive et l'extrémité 5' ou 3' de l'acide nucléique sont séparées par une chaîne espaceur.

Selon l'invention, un aptamère anti-FH peut comprendre une fonction amine réactive à son extrémité 3' ou 5', ce qui signifie que la fonction amine réactive est couplée sur la partie nucléotidique dudit acide nucléique.

Selon d'autres modes de réalisation, un aptamère anti-FH peut comprendre une fonction amine réactive « du côté » de son extrémité 3' ou 5', ce qui signifie que ladite fonction amine n'est pas couplée directement sur la partie nucléotidique dudit aptamère anti-FH, mais est liée de manière covalente à une partie non-nucléotidique dudit aptamère anti-FH, par exemple une chaîne espaceur non-nucléotidique qui est interposée entre ladite fonction amine réactive et ladite extrémité de la partie nucléotidique dudit aptamère anti-FH.

Par « fonction acide carboxylique activée» ou « groupe acide carboxylique activé », on entend une fonction chimique dérivée de la fonction « acide carboxylique » apte à réagir avec un nucléophile. De manière plus spécifique, on entend par « fonction acide carboxylique activée » une fonction chimique dérivée de la fonction « acide carboxylique » apte à réagir avec une amine primaire de manière à former une liaison amidique. Les fonctions « acides carboxyliques activées » sont bien connues de l'homme du métier et englobent les fonctions chlorures d'acide, anhydrides mixtes et esters.

Dans certains modes de réalisation, les fonctions acides carboxyliques activées sont sous forme d'esters résultant de la réaction desdites fonctions acides carboxyliques avec un composé choisi parmi le groupe constitué par le 1-hydroxybenzotriazole (HOBt), le HOAt, le N-hydroxysuccinimide ou l'un de leurs dérivés.

Dans un mode de réalisation préféré, les groupes acides carboxyliques du support ont été activés par réaction avec le N-hydroxysuccinimide ou l'un de ses dérivés tels que le N-hydroxysulfosuccinimide.

Ceci signifie que les groupes « acides carboxyliques activés » du support solide correspondent à des groupes « esters de N-succinimidyle », ou encore appelés « esters de succinimidyle » ou « esters de N-hydroxysuccinimide », de formule suivante (I) où R représente la ramification du support solide à laquelle la fonction ester est fixée:

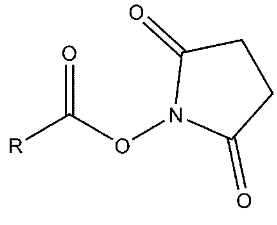

L'activation par le NHS ou le sulfo-NHS présente l'avantage de générer des esters activés réactifs vis-à-vis des amines primaires mais également suffisamment stables pour permettre le conditionnement et le stockage du support pré-activé obtenu.

Les supports solides présentant des fonctions « acides carboxyliques activées » sont bien connus dans l'état de la technique et nombre d'entre eux sont commercialisés. Les supports solides peuvent être également préparés

selon des méthodologies bien connues de l'homme du métier, par exemple en faisant réagir un support présentant initialement des fonctions acides carboxyliques à sa surface avec un agent chimique adapté permettant l'activation des fonctions acides carboxyliques en vue de la formation subséquente d'une liaison amidique. On pourra, en particulier, se référer aux méthodes classiques d'activation des fonctions acides carboxyliques utilisées en synthèse peptidique, en particulier par voie solide. A titre illustratif, on peut aussi utiliser, dans les conditions spécifiques de couplage prescrites par l'invention, la méthodologie d'activation par une combinaison de EDC (chlorhydrate de 1-Ethy1-3-[3-dimethylaminopropyl]carbodiimide) et de NHS, bien connue de l'homme du métier.

Les supports solides présentant des fonctions « acides carboxyliques activées » peuvent être de tout type. Ces supports englobent les supports classiquement utilisés pour la chromatographie, y compris les supports de silice et d'agarose, et qui ont été traités afin de présenter à leur surface des groupes acides carboxyliquesactivés. Les supports solides pré-activés englobent des gels de dextrane, d'agarose, d'amidon, des dérivés cellulosiques, ou encore des polymères synthétiques tels que des polyamides, du trisacryle, du sephacryle des dérivés de méthacrylate, des dérivés du polystyrène et des polyacrylamides, ou encore des supports minéraux tels que des supports de silice (notamment des supports de verre poreux) ou d'alumine, sur la surface desquels sont présents des groupes acides carboxyliques activés. De manière générale, les supports solides sur lesquels peuvent être immobilisés les ligands nucléiques selon l'invention englobent tout type de support ayant la structure et la composition retrouvée communément pour les supports de filtre, des membranes, etc. Les supports solides englobent notamment les résines, les résines ou gels pour colonne de chromatographie d'affinité, les billes de polymères, les billes magnétiques, les billes paramagnétiques, les matériaux supports de membrane filtrante, etc. Les supports solides englobent aussi notamment les matériaux à base de verre ou de métal, tels que l'acier, l'or, l'argent, l'aluminium, le cuivre, le silicium, le verre, la céramique. Les supports solides englobent aussi notamment des matériaux polymères, tels qu'un polyéthylène, un polypropylène, un polyamide, un fluorure de polyvinylidène, des dérivés de polyacrylamide et des combinaisons de ceux-ci.

Dans les modes de réalisation particuliers pour lesquels les fonctions acides carboxyliques du support solide sont activées par le NHS, ledit support solide peut être obtenu en faisant réagir un gel commercial présentant des fonctions acides carboxyliques libres avec le N-hydroxysuccinimide (NHS), éventuellement en présence d'un carbodiimide tel que le 1-éthyl-3-(3-diméthylaminopropyl)carbodiimide (EDC) .

On peut également utiliser un support solide commercial pré-activé par le NHS comme par exemple un gel « NHS Activated Sepharose 4 fast flow (GE) » commercialisé par la société General Electric Healthcare (Etats-Unis), un gel « HiTrap™ NHS-activated » commercialisé par la société General Electric Healthcare (Etats-Unis) ou encore un gel «NHS-Activated Agarose » commercialisé par la société Thermo Scientific Pierce.

Le procédé d'obtention d'un support d'affinité qui a été mis au point par le demandeur a consisté à réaliser la réaction de couplage des aptamères anti-FH sur un support pré-activé par le N-hydroxysuccinimide, dans des conditions de pH inférieur à 5.

Le succès de cette solution technique est tout à fait surprenante car à un pH acide, l'homme du métier se serait normalement attendu à ce que la réaction de couplage n'ait pas lieu du tout, ou à tout le moins ait lieu dans une si faible proportion qu'un très faible rendement de greffage soit obtenu.

Or, la réalisation de l'étape de couplage des aptamères anti-FH sur un support pré-activé par le NHS, dans des conditions de pH inférieur à 5, permet l'obtention d'un support greffé avec un rendement de greffage de 100 %, ou très proche de 100 %.

La réalisation de l'étape de couplage à un pH acide n'affecte pas l'intégrité fonctionnelle des aptamères atni-FH, alors que la grande sensibilité des acides nucléiques aux conditions de pH acide est bien connue par l'homme du métier. Les aptamères anti-FH greffés sur le support conservent leur intégrité chimique et physique, du fait que leur fonctionnalité est intacte.

De plus, le procédé ci-dessus aboutit à l'obtention de supports d'affinité possédant une forte densité d'acides nucléiques greffés et permet en conséquence la disponibilité de supports d'affinité possédant une grande capacité de capture de Facteur H cible, utilisables à l'échelle industrielle.

Les caractéristiques combinées de haute sélectivité vis-à-vis du facteur H cible et de grande capacité de capture dudit Facteur H cible illustrent la compatibilité d'un support d'affinité obtenu comme décrit ci-dessus avec une utilisation dans une étape de purification de Facteur H à l'échelle industrielle.

Dans ces applications industrielles, un avantage supplémentaire d'un support d'affinité préparé comme décrit ci-dessus est sa capacité de résistance à des traitements comprenant d'exposer le support à un pH fortement alcalin et/ou à des composés fortement polaires. Ainsi, les propriétés chromatographiques d'un support d'affinité selon l'invention ne sont pas altérées par un traitement par une solution ayant une concentration finale de NaOH de 0,5 M. On a également montré que les propriétés chromatographiques d'un support d'affinité selon l'invention ne sont pas altérées par un traitement par une solution ayant une concentration finale de propylène glycol de 50 % (vol/vol).

En conséquence, un support d'affinité selon l'invention n'est pas altéré par des traitements drastiques du type décrit ci-dessus, qui sont notamment destinés à éliminer les micro-organismes indésirables susceptibles de contaminer les préparations purifiées du ligand cible, en particulier les bactéries, les champignons et les virus.

Dans ces modes de réalisations particuliers, un support d'affinité pour la fixation sélective du facteur H peut être préparé selon un procédé comprenant les étapes suivantes :

a) fournir un support solide comprenant à sa surface des groupes acides carboxyliques activés,
b) fournir un aptamère nucléique anti-FH ou un composé de formule (I) comprenant au moins une fonction amine réactive, et
c) réaliser le couplage dudit aptamère nucléique anti-FH, ou dudit composé de formule (I), avec les groupes acides carboxyliques activés présents à la surface dudit support solide dans des conditions de pH inférieur à 5,

étant entendu que l'ordre des étapes a) et b) est indifférent.
Il va de soi que l'étape de couplage c) permet la création de liaisons amidiques entre le support solide et les ligands nucléiques, chaque liaison amidique résultant de la réaction entre une fonction acide carboxylique activée du support et une fonction amine primaire présente au niveau du ligand nucléique.
Selon l'invention, les conditions de couplage à un pH inférieur à 5 englobent des conditions de couplage à un pH inférieur à 5, inférieur à 4,9, inférieur à 4,8, inférieur à 4,7, inférieur à 4,6, inférieur à 4,5, inférieur à 4,3.
Dans certains modes de réalisation, le pH de l'étape de couplage est compris dans une gamme allant de 3 à 5, ce qui englobe un pH de 3,0, un pH de 3,1, un pH de 3,2, un pH de 3,3, un pH de 3,4, un pH de 3,5, un pH de 3,6, un pH de 3,7, un pH de 3,8, un pH de 3,9, un pH de 4, 0, un pH de 4,1, un pH de 4,2, un pH de 4,3, un pH de 4,4, un pH de 4,5, un pH de 4,6 un pH de 4,7, un pH de 4,8, un pH de 4,9 et un pH de 5,0.
De préférence, le pH de l'étape de couplage est inférieur à 4,5. Dans certains modes de réalisation, le pH de la réaction de couplage est compris dans une gamme allant 3,5 à 4,5.
L'étape de couplage peut être réalisée à un pH d'environ 4,2.
De préférence, le couplage est réalisé en présence d'un milieu tamponné aqueux présentant un pH inférieur à 5. Le milieu tamponné peut être préparé à partir de tout type d'acides et/ou de bases faibles, dans la mesure ou le ou les acides et bases faibles utilisées ne sont pas susceptibles de réagir au cours de la réaction de couplage. Comme cela est illustré dans les exemples, il peut s'agir d'une solution aqueuse d'acétate de sodium.
L'étape de couplage peut être effectuée indifféremment à basse température et à température ambiante. De manière remarquable, la mise en œuvre de la réaction à température ambiante n'induit pas une diminution du rendement de la réaction. De la même manière, la mise en œuvre de la réaction à basse température - typiquement à une température de 5 °C - n'induit pas une diminution substantielle de la vitesse de réaction.
Ainsi dans certains modes de réaction l'étape de couplage est effectuée à une température comprise dans une gamme allant de 0 °C à 50 °C.
De manière préférée, l'étape de couplage peut être réalisée à une température allant de 0 °C à 35 °C.
De manière pratique, la réaction de couplage pourra être réalisée à température ambiante, c'est-à-dire à une température allant de 15 °C à 35 °C, de préférence à une température allant de 15 °C à 25 °C. Néanmoins, l'étape de couplage pourra être réalisée à basse température, typiquement à une température allant de 0 °C à 8 °C, si les réactifs mis en jeu - en particulier les ligands nucléiques - présentent des groupements chimiques sensibles, en particulier, à l'hydrolyse.
La réaction de couplage étant particulièrement rapide, un avancement satisfaisant de la réaction est généralement obtenu au bout d'environ d'une heure, voire au bout de quelques minutes. En utilisant des techniques de suivi cinétiques adaptées, l'homme du métier sera à même de déterminer la durée optimale de la réaction. Il en est de même pour la température de réaction.
De manière générale, comme cela est illustré dans les exemples, l'étape de couplage peut être effectuée à un pH allant de 3,5 à 4,5, à température ambiante et sur une durée d'environ une heure.
Bien entendu, l'homme du métier peut, sur la base des conditions générales ci-dessus, adapter les conditions de la réaction de couplage afin de déterminer les conditions optimales adaptées dans chaque cas précis, sur la base de ses connaissances générales en chimie. A titre illustratif, l'homme du métier peut facilement prévoir que, pour l'obtention d'un rendement de couplage donné, l'abaissement de la température de réaction est susceptible de nécessiter une augmentation de la durée de l'étape de couplage.
Dans certains modes de réalisation du procédé d'immobilisation selon l'invention, la réaction de couplage peut être achevée en plaçant la combinaison support pré-activé/acides nucléiques dans des conditions de pH alcalin pendant une durée donnée.
Dans ces modes de réalisation, l'étape de couplage du procédé de l'invention comprend elle-même les deux étapes suivantes :

c1) faire réagir ledit acide nucléique avec les groupes acides carboxyliques activés présents à la surface dudit support solide, dans des conditions de pH inférieur à 5 et
c2) poursuivre la réaction de couplage dudit acide nucléique avec les groupes acides carboxyliques activés présents

à la surface dudit support solide, dans des conditions de pH supérieur à 7,5

Avantageusement, la phase finale de couplage à pH alcalin est réalisée à un pH d'au moins 7,5, ce qui englobe les pH d'au moins 8, et d'au moins 8,5. Les exemples illustrent la réalisation de l'étape c2) à un pH d'environ 9

L'étape c2)) peut être réalisée à température ambiante ou à une température basse. Par température basse pour l'étape finale de la réaction de couplage, on entend une température inférieure à 15 °C, y compris une température inférieure à 14 °C, 13 °C, 12 °C, 11 °C, 10 °C, 9 °C, 8 °C, 7 °C, 6 °C ou 5 °C.

La durée de la phase finale de l'étape de couplage est variable. Elle est comprise entre quelques minutes et quelques heures. De manière générale, la durée de l'étape c2) est inférieure à 9 heure ce qui englobe une durée inférieure à 8, 7, 6, 5, 4, 3, 2 et 1 heures. La durée l'étape c2) peut être d'environ 8 heures ou d'environ 3 heures, comme cela est illustré dans les exemples.

Pour ladite phase finale de l'étape de couplage, l'homme du métier peut aisément, sur la base des indications ci-dessus, déterminer les conditions optimales de combinaison de pH, température et durée, au cas-par-cas.

Dans des modes de réalisation avantageux, la réaction de couplage est suivie par une étape de neutralisation d) ou de blocage des groupes acides carboxyliques activés n'ayant pas réagi au cours de l'étape de couplage proprement dit. A titre illustratif, le blocage des fonctions acides carboxyliques activées et n'ayant pas réagi peut être réalisé par incubation du support greffé avec une solution de blocage comprenant 0,5 M d'éthanolamine, 0,5M de NaCl à pH 8,3 ou bien avec une solution de blocage à 0,1M Tris-HCl à pH 8,5, comme cela est recommandé notamment par le fabricant et décrit par ailleurs dans les exemples. La durée de l'étape de neutralisation ou de blocage est avantageusement d'au moins une heure à température basse. Elle peut être réalisée par exemple pendant une durée de 2h30 à la température de 4 °C, comme décrit dans les exemples.

En dernier lieu, le procédé selon l'invention comprend à l'issue de l'étape de couplage c) et/ou à l'issue de l'étape de blocage ou de neutralisation d) une ou plusieurs étapes de lavage e) dudit support dans des conditions classiques de manière à obtenir un support d'affinité prêt à l'emploi. A titre illustratif, la ou les étapes de lavage peuvent être réalisées avec une solution tampon de 0,1M Tris-HCl à un pH allant de 8 à 9, ou bien avec une solution tampon de 0,1M acétate, 0,5M NaCl à un pH allant de 4 à 5, comme cela est illustré dans les exemples. Dans certains modes de réalisation, une étape de lavage comprend successivement (i) un lavage par une solution tampon de 0,1M Tris-HCl à un pH allant de 8 à 9, suivi de (ii) un lavage par une solution tampon de 0,1M acétate, 0,5M NaCl à un pH allant de 4 à 5. Classiquement, on réalise une pluralité d'étapes de lavage, par exemple 3 étapes de lavage, comme cela est illustré dans les exemples.

Au vu de la description qui précède, le procédé d'immobilisation d'acides nucléiques selon l'invention peut être aussi défini comme un procédé comprenant les étapes suivantes :

a) fournir un support solide comprenant à sa surface des groupes acides carboxyliques activés, de préférence par le N-hydroxysuccinimide,
b) fournir un acide nucléique comprenant au moins une fonction amine primaire,
c) réaliser le couplage dudit acide nucléique avec les groupes acides carboxyliques activés présents à la surface dudit support solide dans des conditions de pH inférieur à 5, et
d) bloquer la réaction de couplage.

Le procédé d'immobilisation d'acides nucléiques selon l'invention peut être aussi défini comme un procédé comprenant les étapes suivantes :

a) fournir un support solide comprenant à sa surface des groupes acides carboxyliques activés, de préférence par le N-hydroxysuccinimide,
b) fournir un acide nucléique comprenant au moins une fonction amine primaire,
c) réaliser le couplage dudit acide nucléique avec les groupes acides carboxyliques activés présents à la surface dudit support solide dans des conditions de pH inférieur à5,
d) bloquer la réaction de couplage, et
e) réaliser une ou plusieurs étapes de lavage du support.

Comme cela a déjà été précisé précédemment, dans certains modes de réalisation, l'étape c) comprend elle-même les deux étapes suivantes :

c1) faire réagir ledit acide nucléique avec les groupes acides carboxyliques activés, de préférence par le N-hydroxysuccinimide, présents à la surface dudit support solide dans des conditions de pH inférieur à 5, et
c2) poursuivre la réaction de couplage dudit acide nucléique avec les groupes acides carboxyliques activés présents à la surface dudit support solide dans des conditions de pH supérieur à 7,5

**Procédés d'obtention d'aptamères anti-FH**

De manière générale, un aptamère anti-FH selon l'invention peut être obtenu selon un procédé basé sur les principes généraux de la technique appelée SELEX (Systematic Evolution of Ligands by Exponential Enrichment, qui a été initialement décrite notamment dans la demande PCT N° WO 1991/019813). Le procédé SELEX de sélection des aptamères consiste à mettre en présence une protéine avec une librairie combinatoire d'acides nucléiques, ADN ou ARN (en général $10^{15}$ molécules), les acides nucléiques ne se liant pas à la cible sont éliminés, les acides nucléiques se liant à la cible sont isolés et amplifiés par PCR. Le procédé est répété jusqu'à ce que la solution soit suffisamment enrichie avec les acides nucléiques ayant une bonne affinité pour la protéine d'intérêt (Tuerk et Gold, " Systematic évolution of ligands by exponential enrichment : RNA ligands to bacteriophage T4 DNA polymerase " (1990) Science, 249(4968) :505-10 et Ellington et Szostak, " In vitro sélection of RNA molécules that bind spécifie ligands ", (1990) Nature Aug 30;346(6287):818-22). D'autres exemples de procédé SELEX sont donnés dans les documents EP 0 786 469, EP 668 931, EP 1 695 978, EP 1 493 825 dont les enseignements peuvent être repris dans la réalisation du procédé de sélection d'un aptamère nucléique utilisé selon l'invention. Certaines variantes du procédé SELEX comprennent des étapes de contre-sélection d'aptamères précédemment sélectionnés par liaison à la protéine cible d'intérêt. La ou les étapes de contre-sélection peuvent consister en une étape dans laquelle une collection d'aptamères, qui a été précédemment sélectionnée avec la protéine cible d'intérêt, est mise en contact avec des molécules non-cibles, de manière à éliminer de la collection d'aptamères de départ ceux qui se lient aux molécules non-cibles. La mise en oeuvre d'une telle étape de contre-sélection, dans un procédé d'obtention d'aptamères nucléiques, est susceptible d'accroître la spécificité ou l'affinité des aptamères sélectionnés à la fin du procédé.

Un premier procédé pour l'obtention d'un aptamère anti-FH peut consister en un procédé comprenant les étapes suivantes :

a) fournir un mélange, ou collection, d'acides nucléiques,

b) mettre en contact le mélange d'acides nucléiques fourni à l'étape a), ou le mélange d'acides nucléiques fourni à l'étape d) lorsque l'étape b) est répétée, avec un facteur H purifié, dans des conditions permettant la formation de complexes entre (i) des acides nucléiques dudit mélange et (ii) le facteur H purifié,

c) réaliser une séparation entre (i) le ou les acides nucléiques ayant formé des complexes avec le facteur H et (ii) les acides nucléiques n'ayant pas formé de complexes avec le facteur H, et récupérer le ou les acides nucléiques ayant formé des complexes avec le facteur H,

d) amplifier les acides nucléiques ayant formé des complexes avec le facteur H, de manière à obtenir un mélange, ou collection, d'acides nucléiques se liant au facteur H,

e) réitérer les étapes b) à d) un nombre de fois suffisant pour l'obtention d'une collection d'acides nucléiques ayant la capacité de liaison désirée au facteur H, et

f) collecter le ou les acides nucléiques se liant au facteur H obtenus à la dernière occurrence de l'étape e).

Dans certains modes de réalisation, les caractéristiques du procédé sont adaptées pour l'obtention d'aptamères anti-FH se liant sélectivement au Facteur H natif et ne se liant pas à des formes de Facteur H ayant subi une dégradation, par exemple des formes protéolysées de facteur H. Dans ces modes de réalisation, on utilise, à l'étape b) du procédé, une composition de Facteur H purifié comprenant au moins 70 % en poids de Facteur H natif non dégradé, ce qui englobe les compositions de Facteur H purifié comprenant au moins 75 %, 80 %, 85 %, 90 % en poids de Facteur H natif non dégradé, par rapport au poids total de Facteur H (Facteur H intègre et Facteur H dégradé) contenu dans ladite composition de Facteur H.

Dans certains modes de réalisation du procédé, on utilise des compositions distinctes de Facteur H purifié, en particulier au moins deux compositions distinctes de Facteur H purifié, pour des occurrences distinctes de l'étape b) du procédé. A titre illustratif, une occurrence donnée de l'étape b) est réalisée avec une première composition de Facteur H purifié et l'occurrence suivante de l'étape b) est réalisée avec une seconde composition de Facteur H purifié. Dans ces modes de réalisation, plusieurs étapes b) successives exécutées au cours des cycles successifs de réalisation des étapes b) à e), peuvent être réalisées avec la même composition de Facteur H purifié, ou bien avec des compositions distinctes de Facteur H purifié, comme cela est illustré dans les exemples.

Ainsi, dans certains modes de réalisation du procédé d'aptamères anti-FH, on combine au moins deux caractéristiques, parmi les caractéristiques suivantes :

(i) deux occurrences successives de l'étape b) sont réalisées avec la même composition de Facteur H purifié, et

(ii) deux occurrences successives de l'étape b) sont réalisées avec des compositions distinctes de Facteur H purifié.

Dans certains modes de réalisation du procédé, on peut faire varier la quantité d'aptamères nucléiques anti-FH utilisée à l'étape b) dans deux occurrences distinctes de réalisation de l'étape b).

Dans certains modes de réalisation du procédé, on peut faire varier la quantité de Facteur H purifié utilisée à l'étape b) dans deux occurrences distinctes de réalisation de l'étape b).

Dans les modes de réalisation dans lesquels les quantités respectives d'aptamères anti-FH et de Facteur FH purifié varient du même ordre de grandeur, alors la variation conjointe de la quantité d'aptamères anti-FH et de la quantité de Facteur FH purifié ne modifie pas le rapport molaire des deux "réactifs", d'une occurrence de l'étape b) à une autre.

Dans les modes de réalisation dans lesquels les quantités respectives d'aptamères anti-FH et de Facteur FH purifié varient avec des ordres de grandeur distincts, alors la variation conjointe de la quantité d'aptamères anti-FH et de la quantité de Facteur FH purifié modifie le rapport molaire des deux "réactifs", d'une occurrence de l'étape b) à une autre.

Dans certains modes de réalisation du procédé d'aptamères anti-FH, on combine au moins deux caractéristiques, parmi les caractéristiques suivantes :

(i) deux occurrences successives de l'étape b) sont réalisées avec la même quantité d'aptamères anti-FH et la même quantité de Facteur H purifié,
(ii) deux occurrences successives de l'étape b) sont réalisées avec une quantité distincte d'aptamères anti-FH et une quantité distincte de Facteur H purifié et dans un rapport molaire aptamères anti-FH/Facteur H purifié identique, et
(iii) deux occurrences successives de l'étape b) sont réalisées avec une quantité distincte d'aptamères anti-FH et une quantité distincte de Facteur H purifié et dans un rapport molaire aptamères anti-FH/Facteur H purifié distinct.

Dans certains modes de réalisation du procédé d'obtention d'aptamères anti-FH de l'invention, la durée de l'étape b) de mise en contact des aptamères anti-FH avec le Facteur H purifié peut être identique ou bien distincte, pour deux occurrences successives du procédé.

Les différents modes de réalisation de l'étape b) du procédé d'obtention d'aptamères anti-FH ci-dessus sont illustrés à l'exemple 1.

Sans vouloir être lié par une quelconque théorie, le demandeur pense que l'utilisation de compositions distinctes de Facteur H purifié, à des occurrences distinctes de l'étape b) du procédé, favorise la sélection d'aptamères anti-FH aptes à se lier à du Facteur H provenant d'échantillons d'origine variée.

Selon un premier exemple, l'utilisation de Facteur H humain purifié d'origine plasmatique et de Facteur H humain recombinant purifié, à des occurrences distinctes de l'étape b) du procédé, favorise la sélection d'aptamères anti-FH aptes à se lier à la fois à du Facteur H humain plasmatique et à du Facteur H humain recombinant.

Selon un second exemple, l'utilisation de préparations distinctes de Facteur H humain purifié ayant une faible teneur en Facteur H dégradé, en particulier en Facteur H non protéolysé, à des occurrences distinctes de l'étape b) du procédé, favorise la sélection d'aptamères anti-FH aptes à se lier sélectivement au Facteur H natif non dégradé, lesdits aptamères ne se liant pas ou peu à des formes dégradées du Facteur H, par exemples aux fragments protéolytiques de 37 kDa et de 110 kDa générés après clivage du Facteur H humain natif entre les résidus $Arg_{223}$ et $Arg_{324}$.

Dans certains modes de réalisation, on peut faire varier la durée de l'étape c) de séparation entre (i) le ou les acides nucléiques ayant formé des complexes avec le facteur H et (ii) les acides nucléiques n'ayant pas formé de complexes avec le facteur H. L'étape d) comprend une ou plusieurs étapes successives de lavage avec une solution tampon appropriée. La sélectivité du procédé pour l'obtention d'aptamères anti-FH ayant une forte affinité pour le Facteur FH cible est accrue avec (i) une durée croissante de la ou des étape(s) de lavage réalisées à l'étape c) et avec (ii) un nombre croissant d'étapes de lavage réalisées à l'étape c).

Selon encore d'autres modes de réalisation, un procédé d'obtention d'un aptamère anti-FH peut comprendre une ou plusieurs étapes de sélection de ceux des aptamères permettant la formation de complexes avec le Facteur H en présence d'une solution tampon comprenant un sel de magnésium, par exemple du $MgCl_2$. L'étape de sélection de ces aptamères peut consister à réaliser au moins une occurrence de l'étape b) du procédé avec un milieu, y compris une solution tampon, comprenant une concentration finale en $MgCl_2$ d'au moins 1 mM, ce qui englobe au moins 2 mM, 3mM, 4 mM, 5 mM, 6 mM, 7 mM, 8 mM ou 9 mM. Selon une première alternative, l'étape de sélection avec une solution tampon comprenant un sel de magnésium, par exemple du $MgCl_2$ est réalisée pour seul cycle de sélection (étapes a) à d)). Selon une seconde alternative, l'étape de sélection avec une solution tampon comprenant un sel de magnésium, par exemple du $MgCl_2$, est réalisée pour une pluralité de cycles de sélection, soit de cycles successifs, soit de cycles non successifs. Selon une troisième alternative, l'étape de sélection avec une solution tampon comprenant un sel de magnésium, par exemple du $MgCl_2$, est réalisée pour chaque cycle de sélection.

Dans certains modes de réalisation, on utilise à l'étape b) une solution tampon comprenant du chlorure de magnésium, par exemple une solution tampon de Tris-HCl 50mM à pH 7,5, 150 mM NaCl et 10 mM MgC12 par comparaison avec une solution tampon identique mais dépourvue de chlorure de magnésium.

Selon encore d'autres modes de réalisation, un procédé d'obtention d'un aptamère anti- FH peut être du type favorisant la sélection de ceux des aptamères permettant la formation de complexes avec le Facteur H, lesdits complexes pouvant être dissociés, avec libération du Facteur H, par mise en contact desdits complexes avec un milieu comprenant

un chélateur d'ions divalents, par exemple de l'EDTA.

Dans ces modes de réalisation particuliers, on utilise à l'étape c) une solution tampon comprenant un agent chélatant des ions divalents, par exemple l'EDTA. On peut utiliser à l'étape c) une solution tampon d'élution comprenant de l'EDTA à la concentration finale d'au moins 1 mM et d'au plus 500 mM. La réalisation de l'étape c) en présence d'EDTA favorise la sélection d'aptamères nucléiques anti-FH lesquels, lorsqu'ils sont impliqués dans des complexes avec le Facteur H cible, libèrent ledit facteur H cible lorsque lesdits complexes sont mis en contact avec une solution tampon d'élution comprenant de l'EDTA, en particulier de l'EDTA à une concentration finale d'au moins 1 mM et d'au plus 500 mM.

Ainsi, dans certains modes de réalisation du procédé d'obtention d'aptamères anti-FH, au moins une occurrence de l'étape c), avantageusement une pluralité d'occurrences de l'étape c), ou bien la totalité des occurrences de l'étape c), est réalisée avec une solution tampon comprenant de l'EDTA, notamment à une concentration finale d'EDTA définie ci-dessus.

Dans certains modes de réalisation du procédé, la spécificité des aptamères anti-FH vis-à-vis du Facteur H cible peut encore être accrue en mettant en œuvre une ou plusieurs étapes de contre-sélection. Dans certains modes de réalisation, on met en œuvre au moins une étape de contre-sélection avant la première occurrence d'une étape de sélection (étapes a) à d) du procédé). Dans certains modes de réalisation, on met en œuvre au moins une étape de sélection qui est interposée entre deux occurrences d'une étape de sélection du procédé. Dans certains modes de réalisation, on met en œuvre au moins une étape de contre-sélection après la dernière occurrence d'une étape de sélection du procédé.

Par exemple, On peut ajouter à l'un quelconque des modes de réalisation du procédé décrits ci-dessus, une ou plusieurs étapes d'incubation des aptamères obtenus à la fin d'une occurrence de l'étape d) avec un Facteur H non cible, par exemple un Facteur H non humain lorsque l'on veut obtenir des aptamères nucléiques spécifiques du Facteur H humain. Dans ces modes de réalisation, on inclut, à la fin d'une occurrence de l'étape c) ou de l'étape d), une étape de mise en contact des acides nucléiques obtenus avec un Facteur H non cible et on récupère les aptamères anti-FH n'ayant pas formé de complexes avec ledit Facteur H non cible. Les aptamères sélectionnés n'ayant pas formé de complexes avec le Facteur H non cible peuvent être directement utilisés comme aptamères anti-FH selon l'invention. Alternativement, Les aptamères sélectionnés n'ayant pas formé de complexes avec le Facteur H non cible sont soumis à un ou plusieurs cycles de sélection additionnels, par réitération des étapes a) à d). Selon une première alternative, l'étape de contre-sélection avec un Facteur H non cible est réalisée à la fin d'un seul cycle de sélection (étapes a) à d)). Selon une seconde alternative, l'étape de contre-sélection avec un Facteur H non cible est réalisée à la fin d'une pluralité de cycles de sélection, soit de cycles successifs, soit de cycles non successifs. Selon une troisième alternative, l'étape de contre-sélection avec un Facteur H non cible est réalisée à la fin de chaque cycle de sélection du procédé.

On utilise les modes de réalisation ci-dessus en particulier pour l'obtention d'aptamères anti-FH humain produits chez un animal transgénique, dans lesquelles on réalise une ou plusieurs étapes de contre-sélection avec du Facteur H naturel ou recombinant dudit animal transgénique.

La spécificité et/ou l'affinité des aptamères anti-FH peut aussi être accrue en ajoutant à l'un quelconque des modes de réalisation du procédé décrit ci-dessus une ou plusieurs étapes de contre-sélection vis-à-vis du matériau utilisé pour retenir les acides nucléiques ayant formé des complexes avec le facteur H, utilisé à l'étape c) du procédé. Les exemples illustrent la mise en œuvre d'un procédé d'obtention d'aptamères anti-FH pour lequel on utilise une membrane de nitrocellulose à l'étape c), ledit procédé comprenant systématiquement, à partir de la première réitération de la séquence d'étapes a)-d), et éventuellement aussi à chaque réitération suivante, une étape de contre-sélection par élimination des aptamères nucléiques se liant à la membrane de nitrocellulose. Dans ces modes de réalisation, les aptamères obtenus à la fin d'au moins une occurrence de l'étape c) ou de l'étape d) sont mis en contact avec le matériau utilisé pour retenir les acides nucléiques, par exemple une membrane de nitrocellulose, puis les aptamères qui ne sont pas liés audit matériau (i) soit sont utilisés directement comme aptamères anti-FH, (ii) soit sont soumis à un ou plusieurs cycles de sélection additionnels, par réitération des étapes a) à d).

Les caractéristiques des aptamères anti-FH, et en particulier leur inaptitude à se lier à des protéines autres que le Facteur H, peuvent aussi être améliorées par des étapes de contre-sélection par élimination des aptamères se liant à des protéines autres que le Facteur H cible. Pour l'obtention d'aptamères anti-FH utilisables pour détecter ou purifier du Facteur H plasmatique, il est avantageux d'inclure dans le procédé une ou plusieurs étapes de contre-sélection vis-à-vis d'une ou plusieurs protéines plasmatiques autres que le Facteur H, ou vis-à-vis de mélanges de protéines plasmatiques dépourvus de Facteur H. De telles étapes de contre-sélection favorisent notamment l'obtention d'aptamères anti-FH ne se liant pas aux protéines du complément, en particulier ne se liant pas à la protéine C3 du complément. Les exemples illustrent un procédé comprenant des étapes de contre-sélection vis-à-vis de deux mélanges de protéines plasmatiques humaines, respectivement du sérum humain épuisé en Facteur H et un cryosurnageant plasmatique humain épuisé en Facteur H.

Dans les modes de réalisation ci-dessus, on inclut, à la fin d'une occurrence de l'étape c) ou de l'étape d), une étape de mise en contact des acides nucléiques obtenus avec une composition comprenant un mélange de protéines

plasmatiques et on récupère les aptamères anti-FH n'ayant pas formé de complexes avec lesdites protéines plasmatiques. Les aptamères sélectionnés n'ayant pas formé de complexes avec lesdites protéines plasmatiques peuvent être directement utilisés comme aptamères anti-FH selon l'invention. Alternativement, les aptamères sélectionnés n'ayant pas formé de complexes avec lesdites protéines plasmatiques sont soumis à un ou plusieurs cycles de sélection additionnels, par réitération des étapes a) à d). Selon une première alternative, l'étape de contre-sélection avec une composition comprenant un mélange de protéines plasmatiques est réalisée à la fin d'un seul cycle de sélection (étapes a) à d)). Selon une seconde alternative, l'étape de contre-sélection avec une composition comprenant un mélange de protéines plasmatiques est réalisée à la fin d'une pluralité de cycles de sélection, soit de cycles successifs, soit de cycles non successifs. Selon une troisième alternative, l'étape de contre-sélection avec une composition comprenant un mélange de protéines plasmatiques est réalisée à la fin de chaque cycle de sélection du procédé.

Selon encore d'autres modes de réalisation, un procédé d'obtention d'un aptamère anti-FH peut être du type de l'un quelconque des procédés décrits ci-dessus, auquel peut être ajoutée une étape de sélection de ceux des aptamères dont la capacité à se lier à la protéine cible n'est pas altérée par la présence d'un alkylène glycol ou d'un polyalkylène glycol.

Selon encore d'autres modes de réalisation, un procédé d'obtention d'un aptamère anti-FH peut être du type de l'un quelconque des procédés décrits ci-dessus, auquel peut être ajoutée une étape de sélection de ceux des aptamères dont la capacité à se lier à la protéine cible n'est pas altérée par la présence d'éthylène glycol. L'étape de sélection de ces aptamères peut être réalisée avec un milieu, y compris une solution tampon, comprenant une concentration finale d'éthylène glycol d'au moins 0,5 M, ce qui inclut au moins 1 M, et 1 ,5 M.

Selon encore d'autres modes de réalisation, un procédé d'obtention d'un aptamère anti-FH peut être du type de l'un quelconque des procédés décrits ci-dessus, auquel peut être ajoutée une étape de sélection de ceux des aptamères dont la capacité à se lier à la protéine cible n'est pas altérée par la présence de propylène glycol. L'étape de sélection de ces aptamères peut être réalisée avec un milieu, y compris une solution tampon, comprenant une concentration finale de propylène glycol d'au moins 10 % (v/v), ce qui inclut au moins 15 %, 20 %, 25 %, 30 %, 35 %, 40 %, 45 % et 50 %.

Selon encore d'autres modes de réalisation, un procédé d'obtention d'un aptamère anti-FH peut être du type de l'un quelconque des procédés décrits ci-dessus, auquel peut être ajoutée une étape de sélection de ceux des aptamères dont la capacité à se lier à la protéine cible n'est pas altérée par la présence d'éthanol. L'étape de sélection de ces aptamères peut être réalisée avec un milieu, y compris une solution tampon, comprenant une concentration finale en éthanol d'au moins 1 % (v/v), ce qui inclut au moins 1 ,5 %, 2,0 %, 2,5 %, 3,0 %, 3,5 %, 4,0 %, 4,5 %, 5,0 %, 5,5 %, 6,0 %, 6,5 %, 7,0 %, 7,5 %, 8,0 %, 9,0 %, 9,5 % et 10,0 %.

Tableau 1 des séquences

| SEQ ID N° | Type | Désignation |
|---|---|---|
| 1 | Acide nucléique | Région 5' commune des aptamères |
| 2 | Acide nucléique | Région 3' commune des aptamères |
| 3 à 42 | Acide nucléique | séquences des régions centrales des aptamères de séquences SEQ ID N° 117 à 156 (Famille 1) |
| 43 à 60 | Acide nucléique | séquences des régions centrales des aptamères de séquences SEQ ID N° 159 à 176 (Famille 2) |
| 61 à 64 | Acide nucléique | séquences des régions centrales des aptamères de séquences SEQ ID N° 177 à 180 (Famille 3) |
| 65 à 66 | Acide nucléique | séquences des régions centrales des aptamères de séquences SEQ ID N° 181 à 182 (Famille 4) |
| 67 à 71 | Acide nucléique | séquences des régions centrales des aptamères de séquences SEQ ID N° 183 à 187 (famille 5) |
| 72 à 116 | Acide nucléique | séquences des régions centrales des aptamères de séquences SEQ ID N° 188 à 232 |
| 117 à 158 | Acide nucléique | aptamères (Famille 1) |
| 159 à 176 | Acide nucléique | aptamères (Famille 2) |
| 177 à 180 | Acide nucléique | aptamères (Famille 3) |
| 181 à 182 | Acide nucléique | aptamères (Famille 4) |

(suite)

| SEQ ID N° | Type | Désignation |
|---|---|---|
| 183 à 187 | Acide nucléique | aptamères (Famille 5) |
| 188 à 232 | Acide nucléique | autres aptamères anti-FH |
| 233 | Acide nucléique | séquence consensus de Famille 1 |
| 234 | Acide nucléique | séquence consensus de famille 1 retrouvée dans SEQ ID N°3 |
| 235 | Acide nucléique | séquence consensus de Famille 2 |
| 236 | Acide nucléique | séquence consensus de Famille 3 |
| 237 | Acide nucléique | séquence consensus de Famille 4 |
| 238 | Acide nucléique | séquence consensus de Famille 5 |
| 239 | Acide nucléique | Aptamère MaptH1.1 N13-48 |
| 240 | Acide nucléique | Aptamère MaptH1.1 N19-58 |
| 241 | Acide nucléique | Aptamère MaptH1.1 N19-53 |
| 242 | Acide nucléique | Aptamère MaptH1.1 N19-48 |
| 243 | Acide nucléique | Aptamère MaptH1.1 N22-45 |

La présente invention est en outre illustrée par les exemples suivants.

## EXEMPLES

### Exemple 1 : Obtention d'aptamères nucléiques anti-FH

#### 1.1. Facteur H cible

On a utilisé deux préparations de Facteur H humain purifié, respectivement :

- une composition de Facteur H purifié commercialisée par la Société Calbiochem (San Diego, USA) sous la référence 341274
- une composition de Facteur H purifié préparé par le demandeur.

#### 1.2. Mélange ou collection d'acides nucléiques

On a utilisé une banque aléatoire d'acides désoxyribonucléiques (ADN) ayant la séquence générique 5'-(SEQ ID N°1) - $(N)_{40}$ - (SEQ ID N°2)-3', dans laquelle $(N)_{40}$ représente une séquence aléatoire de 40 nucléotides de longueur. Les séquences SEQ ID N°1 et SEQ ID N°2 sont des séquences fixes qui sont utilisées pour l'amplification des séquences se liant au facteur H, à chaque cycle de sélection de la méthode SELEX.

Pour les besoins (i) de régénérer le brin sens à la fin de chaque cycle de sélection de la méthode SELEX et (ii) de tester la sélectivité et/ou l'affinité des aptamères anti-FH obtenus à la fin de chaque cycle de sélection de la méthode SELEX, les acides nucléiques sélectionnés ont été couplés à la biotine (masse moléculaire de 569,61), la biotine étant liée à l'extrémité 5' de l'acide nucléique par une chaîne espaceur consistant en un polyéthylène glycol de type PEG(C18) (Masse moléculaire de 344,3) commercialisé par la Société Sigma Aldrich (Saint Louis, MO, USA).

#### 1.3. Tampon de sélection

Comme tampon de sélection, on a utilisé un tampon 50 mM Tris HCl à pH 7,5, 150 mM NaCl, 10 mM $MgCl_2$.

#### 1.4. Tampon d'élution

Comme tampon d'élution, on a utilisé un tampon 50 mM Tris HCl à pH 7,5, 150 mM NaCl, 50 mM EDTA.

**1.5. Sélection d'aptamères nucléiques anti-FH**

Pour la sélection des aptamères anti-FH, on a incubé la banque d'ADN (environ $10^{15}$ séquences aléatoires) avec le Facteur H purifié dans le tampon de sélection (voir § 1.3 ci-dessus).
Le mélange banque d'ADN/Facteur H est ensuite filtré sur une membrane de nitrocellulose du type MF-Millipore Membrane Filter (référence HAWP02500) commercialisée par la Société Millipore
Les complexes ADN/Facteur H qui ont été formés sont retenus sur la membrane filtrante de nitrocellulose. Les ADNs qui n'ont pas formé de complexes avec le Facteur H traversent la membrane filtrante de nitrocellulose et sont éliminés.
On réalise un ou plusieurs lavages de la membrane filtrante avec le tampon de sélection.
Les ADNs retenus sur le filtre de nitrocellulose sont élués avec le tampon d'élution (voir § 1.4. ci-dessus).
Les ADNs récupérés après élution sont amplifiés par PCR en utilisant une paire d'amorces hybridant respectivement avec les séquences SEQ ID N° 1 et SEQ ID N°2.
Puis, le brin sens est régénéré par chromatographie d'affinité sur des billes magnétiques sur lesquelles est immobilisée de la streptavidine.

*1.6.1. Contre-sélection vis-à-vis de la nitrocellulose*

Pour réaliser une étape de contre-sélection vis-à-vis de la nitrocellulose, les ADNs qui ont été élués à l'issue d'un cycle de sélection sont incubés au contact de la membrane de nitrocellulose.
Les ADNs non retenus sur la membrane de nitrocellulose sont amplifiés puis utilisés pour réaliser le cycle de sélection suivant.

*1.6.2. Contre-sélection avec un mélange de protéines plasmatiques dépourvu de Facteur H*

Pour réaliser une étape de contre-sélection avec un mélange de protéines plasmatiques, on a utilisé respectivement (i) du sérum humain dépourvu de Facteur H ou (ii) du cryosurnageant de plasma dépourvu en Facteur H.
Avant l'étape de sélection avec le facteur H, Les ADNs ont été incubés avec le mélange de protéines plasmatiques dépourvu en Facteur H, dans le tampon de sélection utilisé précédemment dans les conditions opératoires suivantes :
On a filtré la solution résultante sur une membrane de nitrocellulose et on a récupéré dans le filtrat les ADNs non retenus, qui n'ont pas formé de complexes avec les protéines plasmatiques.
Les ADNs non retenus vont alors suivre le cycle de sélection avec le facteur H décrit précédemment.

*1.6.3. Description détaillée de la méthode de sélection*

On a réitéré les cycles de sélection, selon les conditions opératoires détaillées dans le Tableau 2 ci-dessous.

Tableau 2

| Cycle n° | ADN (pmoles) | FH (pmoles) | Type de FH | Temps d'incubation (minutes) | Nombres et durée des lavages | Contre-sélection nitrocellulose | autre contre-sélection |
|---|---|---|---|---|---|---|---|
| 1 | 2000 | 1000 | LFB | 60 | 1 (1 min) | non | non |
| 2 | 400 | 200 | LFB | 60 | 2 (2min) | oui | non |
| 3 | 400 | 200 | Calbiochem | 45 | 2 (2min) | oui | non |
| 4 | 200 | 100 | Calbiochem | 45 | 3 (3min) | oui | non |
| 5 | 200 | 100 | LFB | 30 | 3 (3min) | oui | non |
| 6 | 200 | 100 | LFB | 30 | 4 (4min) | oui | non |
| 7 | 200 | 50 | Calbiochem | 15 | 4 (13min) | oui | non |
| 8 | 200 | 50 | Calbiochem | 15 | 5 (20 min) | oui | sérum humain déplété en FH |
| 9 | 200 | 50 | LFB | 10 | 5 (20 min) | oui | sérum humain déplété en FH |
| 10 | 200 | 50 | LFB | 10 | 5 (20 min) | oui | cryosurnageant déplété en FH |
| 11 | 200 | 50 | Calbiochem | 10 | 5 (20 min) | oui | cryosurnageant déplété en FH |

Comme indiqué dans le Tableau 2, on a fait varier les quantités absolues, respectivement d'ADN et de Facteur H, d'un cycle à l'autre de la méthode SELEX. Toutefois, un rapport molaire ADNs/FH identique a été utilisé pour les cycles n° 1 à 6 (rapport de 2) et pour les cycles n°7 à 11 (rapport de 4) respectivement.
Les ADNs et le Facteur H ont été incubés pendant des durées décroissantes, avec les cycles de sélection successifs.
On a augmenté le nombre d'étapes de lavage, avec les cycles de sélection successifs. De plus, on a augmenté progressivement la durée totale des lavages.

**Exemple 2 : Caractéristiques de liaison au Facteur H**

**2.1. Conditions expérimentales**

Appareil utilisé: Biacore T100
Puce : Le facteur H (LFB) a été immobilisé en utilisant la chimie NHS/EDC (chip CM5, GE) à 5813 RU en flow cell 2-1 (FC2-1). Aucune protéine n'est fixée sur la flow cell n°1 (FC1) mais cette flow cell a subi le même traitement que la flow cell n°2. L'échantillon injecté passe sur la FC1 et la FC2 afin de soustraire rigoureusement le bruit de fond dû à des interactions non spécifiques.
Tampon de course et dilution des échantillons : Tris 50 mM, NaCl 150 mM, MgC12 10 mM, pH 7.5
Flux : 30μL/min, injection pendant 60 sec, dissociation pendant 60 sec
Signal : Signal sur FC2 avec soustraction du signal sur FC1
Régénération :EDTA 50 mM pH 5 dans de l'eau, 2 fois 60 sec

**2.2. Résultats**

Les résultats sont représentés sur les figures 1 et 2.
L'évolution de la population d'aptamères sélectionnée après chaque cycle de réalisation du procédé SELEX est évaluée par mesure de fixation avec la technique Biacore®. On a mesuré la capacité de la population d'aptamères à se lier au Facteur H cible.

***A. Caractérisation de la banque d'ADN de départ***

Dans un premier temps, il a été vérifié que la banque d'acides nucléiques ADN de départ ne présente pas d'affinité détectable avec le facteur H.
Dans un premier essai, le facteur H a été immobilisé sur le support Biacore® qui consiste en une puce du type CM5 commercialisée par la Société GE Healthcare, qui comprend un support d'or sur lequel sont immobilisées des molécules de dextran carboxyméthylées, par la méthode NHS avec EDC (un carbodiimide).La banque d'ADN a été injectée à des concentrations de 0,25 μM, 0.5 μM et 1 μM. Aucune fixation de la banque d'ADN n'est observée sur le facteur H, quelle que soit sa concentration utilisée (Figure 1A).
Dans un second essai, la banque a été immobilisée sur une puce streptavidine et le facteur H a été injecté à des concentrations de 200 nM et 400 nM. Aucune fixation du Facteur H n'est observée sur le support sur lequel sont immobilisés les acides nucléiques de la banque d'ADN de départ (Figure 1B).
Les résultats montrent que la banque d'ADN de départ n'a donc pas d'affinité détectable pour le Facteur H, avant le début du processus de sélection d'aptamères nucléiques par le procédé SELEX.

***B.Caractérisation des aptamères nucléiques anti-facteur H***

*B.1. Affinité pour le Facteur H*

On a caractérisé l'aptitude des ensembles d'acides nucléiques sélectionnés à se lier au Facteur H. La capacité de fixation au Facteur H des ensembles d'acides nucléiques obtenus à la fin des $5^{ème,}$ $6^{ème}$ et $7^{ème}$ cycles de réalisation du procédé SELEX a été mesurée par la technique Biacore®.
Les résultats sont représentés sur la Figure 2.
La Figure 2A montre les résultats de mesure de la capacité de fixation des ensembles d'acides nucléiques obtenus à l'issue des cycles 5 à 9 (SHT5 à SHT9)au Facteur H immobilisé sur un support. Les résultats montrent que l'affinité des aptamères nucléiques augmente avec le nombre de cycles de sélection. On note que les résultats de la figure 1 indiquent une affinité des acides nucléiques obtenus à l'issue du cycle n°8, supérieure à celle des acides nucléiques obtenus à l'issue du cycle n°9..
La Figure 2B montre les résultats de mesure de la capacité de fixation du Facteur H aux aptamère nucléiques

obtenus à l'issue du cycle n° 6, qui sont immobilisés sur un support. Courbe n° 1 (courbe inférieure) : solution tampon de sélection exempte d'acides nucléiques. Courbe n° 2 : facteur H humain plasmatique à la concentration finale de 400 nM. Courbe n° 3 : Facteur H humain plasmatique à la concentration de 200 nM.

Par ailleurs, le Tableau 3 ci-dessous présente les résultats de liaison sélective d'une variété d'aptamères nucléiques selon l'invention au Facteur H humain. Les aptamères testés ont été sélectionnés à l'issue du cycle n°7 du procédé Selex décrit à l'exemple 1.

Tableau 3

| Nom séquence | Séquence | Stabilité de liaison au Facteur H (RU) |
|---|---|---|
| FAM1-1 | GGGTCAATGCCAGGTCTCCGGTCTCGAGATGGGGTCTGGTGGTTATACGCTCTCAGT GATCGGCTCGCAAGCAGTC (SEQ ID N° 117) | 19,2* |
| FAM1-2 | GGGTCAATGCCAGGTCTCACCGTCATCGAGAAGGGTCTGGTGGTTATACGTTCTCGT GATCGGCTCGCAAGCAGTC (SEQ ID N° 118) | 47 |
| FAM 1-3 | GGGTCAATGCCAGGTCTCGGACCGCGATGGAGAGGGGTCTGGTGGTTATACGCTAT GCATCGGCTCGCAAGCAGTC (SEQ ID N° 119) | 20,95 |
| FAM 1-4 | GGGTCAATGCCAGGTCTCGGGCCGCGATGGAGAGGGGTCTGGTGGTTATACGCTAT GCATCGGCTCGCAAGCAGTC (SEQ ID N°157) | 26,2 |
| FAM1-5 | GGGTCAATGCCAGGTCTCGCAGAGGTGCGAGGGGCGGTGGGGTCTGGTGGTTATAC GCATCGGCTCGCAAGCAGTC (SEQ ID N° 124) | 21,25 |
| FAM1-6 | GGGTCAATGCCAGGTCTCGGCAGGGAGGAGGAATCGGGACAGGCGGTTATACGGA TTCATCGGCTCGCAAGCAGTC (SEQ ID N° 128) | 30 |
| FAM1-7 | GGGTCAATGCCAGGTCTCGGGGGGCCGGTTATACGGGTCAGGCGGTTATACGGTAT TCATCGGCTCGCAAGCAGTC (SEQ ID N° 135) | 15,45 |
| FAM 1-8 | GGGTCAATGCCAGGTCTCGGGGTGCGAGGGTCTGGTGGTTATACGTCGTCACCTCTA GATCGGCTCGCAAGCAGTC (SEQID N° 150) | 35,95 |
| FAM1-9 | GGGTCAATGCCAGGTCTCGAGGGGCTTTTGGGAGGGACTGGTGGTTATACGTCCCA ATATCGGCTCGCAAGCAGTC (SEQ ID N° 152) | 57,8 |
| FAM1-10 | GGGTCAATGCCAGGTCTCGCGGTGGACGGGGCTGGCGGTTATACGGTCCTTACGCG GTATCGGCTCGCAAGCAGTC (SEQ ID N° 153) | 73,5 |
| FAM1-11 | GGGTCAATGCCAGGTCTCCGGGGCAGGCGGTTATACGGGAGAACGGTATGGGGGG ACTATCGGCTCGCAAGCAGTC (SEQ ID N° 138) | 16,9 |
| FAM1-12 | GGGTCAATGCCAGGTCTCACCGGGTCAGGCGGTTATACGGGTGAGGGCAGGTACAT ACATCGGCTCGCAAGCAGTC (SEQ ID N° 158) | 18,2 |
| FAM1-13 | GGGTCAATGCCAGGTCTCGGGCACGGGTCAGGCGGTTATACGGTGCCCATTGTTCTT TATCGGCTCGCAAGCAGTC [SEQ ID N° 142) | 30,65 |
| FAM2-1 | GGGTCAATGCCAGGTCTCCCGGTTACCGCGCTAAGCTTGGCGATGGTGTTAGTGGCT GATCGGCTCGCAAGCAGTC [SEQ ID N° 159) | 29,65 |
| FAM2-2 | GGGTCAATGCCAGGTCTCCCGGTTACTGCCGTAGTTGTCTTACGGTGGTGTTAGTGG CATCGGCTCGCAAGCAGTC (SEQ ID N° 161) | 22,4 |
| FAM2-3 | GGGTCAATGCCAGGTCTCCCGGTTACCTCCATTGGTGGTGTTAGTGGCTTTGTAGGA TATCGGCTCGCAAGCAGTC [SEQ ID N° 164) | 18,25 |

(suite)

| Nom séquence | Séquence | Stabilité de liaison au Facteur H (RU) |
|---|---|---|
| FAM2-4 | GGGTCAATGCCAGGTCTCCCGGTTACCAGTTGGTGTTTGTGGCTTTGCACGTCTGCAT ATCGGCTCGCAAGCAGTC (SEQ ID N° 165) | 27,55 |
| FAM2-5 | GGGTCAATGCCAGGTCTCCCGGTTACCAACTATGATAGTATGGTGTTTGTGGCATGA TCGGCTCGCAAGCAGTC (SEQ ID N° 172) | 22,25 |
| FAM2-6 | GGGTCAATGCCAGGTCTCCCGGTTACCATCGAGGACGTCGCTTGGTGTTTGTGGCAT GATCGGCTCGCAAGCAGTC (SEQ ID N° 174) | 22,05 |
| FAM2-7 | GGGTCAATGCCAGGTCTCCCGGTTACCAAGATCGTTGTTCTTGGTGTTAGTGGCATTC ATCGGCTCGCAAGCAGT (SEQ ID N° 175)C | 22,05 |
| FAM3 | GGGTCAATGCCAGGTCTCTGAGGCGCAGATGTGGAGGCTTTTACAGGCGGTGCGGA ACATCGGCTCGCAAGCAGTC (SEQ ID N° 177) | 11,7 |
| FAM4 | GGGTCAATGCCAGGTCTCCAAAGGGGGGGGTTGGGGGACCGTCCGTTGTTGATCTC ACATCGGCTCGCAAGCAGTC (SEQ ID N° 181) | 25,85 |
| IND1 | GGGTCAATGCCAGGTCTCGGTGGGAGGTGAGGTCGTTGGACGGTGGCAGGGGATT TTGATCGGCTCGCAAGCAGTC (SEQ ID N° 193) | 20,1 |
| * : valeur d'unités de résonance arbitraires (RU) au pic du signal. | | |

Les résultats du Tableau 3 ci-dessus montrent que la totalité des aptamères nucléiques testés se lient sélectivement au Facteur H.

<u>**Exemple 3 : Obtention d'un support d'affinité**</u>

- Tampon de greffage : Acétate de sodium 100mM, pH = 4,2
- Préparation de 1595 µl d'aptamère à 2,5 g/l dans tampon de greffage soit : 4 mg d'aptamère anti-FH.
- Pour le greffage, on a utilisé respectivement :

  a) pour la réalisation d'un premier support d'affinité, un aptamère anti-FH comprenant un polynucléotide choisi parmi les séquences SEQ ID N° 117 à 158 lié à son extrémité 5' à la chaîne espaceur 11-(trifluoroacetamido)-3,6,9-trioxaundecan-1-yl-[(2-cyanoethyl)-(N,N-diisopropyl) (espaceur C11 hydrophile proposé par la société Link Technologies)
  b) pour la réalisation d'un second support d'affinité, un aptamère comprenant le polynucléotide choisi parmi les séquences SEQ ID N° 117 à 158 lié à son extrémité 5' à une chaîne espaceur 12-(4-Monométhoxytritylamino)dodécyl-[(2-cyanoéthyl)-(N,N-diisopropyl)] (espaceur C12) et lié à son extrémité 3' à un oligo-dT
  c) pour la réalisation d'un troisième support d'affinité, un aptamère comprenant le polynucléotide choisi parmi les séquences SEQ ID N° 117 à 158 lié à son extrémité 5' à une chaîne espaceur 6-(Trifluoroacétylamino)héxyl-[(2-cyanoéthyl)-(N,N-diisopropyl)] (espaceur C6)

- Préparation d'1ml de gel comprenant des groupes acides carboxyliques activés par le NHS à savoir le gel pré-activé «NHS Activated Sepharose 4 fast flow (GE) » en réalisant un lavage avec HCl 1mM, puis un lavage avec le tampon de greffage.
- On a vérifié que le pH dans la préparation d'aptamère dans la solution tampon était de 4,2
- On a mélangé la préparation d'aptamère avec 1ml de gel pré-activé. Le gel pré-activé a été incubé en présence des aptamères sous agitation pendant 48h (+/- 2H) à 4 °C
- On a ajouté un demi volume réactionnel (797µl) de Tampon borate 200mM pH=9 en agitant, puis on a incubé pendant 8h sous agitation à 4 °C

- On a récupéré le surnageant et on a dosé la quantité d'aptamères non greffés
- On a ajouté 2ml de Tris-HCL 0,1M pH = 8.5 sous agitation pendant 2h30 à 4 °C pour bloquer la réaction de couplage.
- On a réalisé 3 cycles d'ajout /agitation /élimination du surnageant comprenant : 1) 1ml de Tris-HCl 0,1M pH = 8.5 puis 2) 1ml d'acétate de sodium à 0,1M NaCl 0,5 M, pH = 4,0, afin d'obtenir un support d'affinité prêt à l'emploi.

Les résultats montrent que la solution résultante obtenue après la réalisation du greffage chimique ne comprend pas de quantité détectable d'aptamère nucléique, c'est-à-dire, dans les conditions d'analyse utilisées, comprend une quantité d'aptamère inférieure à 0,08mg/ml

Il peut être déduit de ces résultats que le rendement de greffage est de 100 %, ou très proche de 100 %.

Une analyse plus fine des caractéristiques du support d'affinité a permis de définir les caractéristiques suivantes :

- Capacité de greffage ($C_{greffage}$) de l'aptamère :6 mg/mL de gel
- Rendement de greffage : supérieur à 98%
- Concentration finale du gel en aptamère anti-FH ($C_{finale\ du\ gel}$) : supérieur à 6mg/mL

**Exemple 4 : Purification d'un Facteur H plasmatique.**

**A. Matériels et Méthodes**

Le support d'affinité a été réalisé à partir d'un matériau de support solide consistant en une matrice sur laquelle on a greffé de la streptavidine (streptavidin-agarose - Novagen®
On a introduit un volume de 1 ml de gel dans une conteneur consistant en une colonne (i.d. 1 1 mm). On a lavé le gel avec de l'eau purifiée, pour éliminer le solvant de stockage
La sortie de la colonne conditionnée (" packée ") (Hauteur de lit de gel = 1cm) est connectée à un détecteur d'absorbance équipé d'un filtre UV à 254 nm et d'un enregistreur. Des aptamères nucléiques anti-FH humain biotinylés MaptH1.1 (SEQ ID N° 144) sont solubilisés dans de l'eau purifiée à la concentration finale de 0,5 mg/0,187 ml, soit une concentration molaire finale de 0,1 mM. La solution d'aptamères nucléiques a été activée à 95°C selon le cycle standard, pour l'immobilisation des aptamères sur le matériau support solide.

La solution d'aptamères nucléiques a été préalablement diluée par 4,8 ml d'eau purifiée puis 1 ,5 ml de tampon $Me^{++}$ (5 x concentré).

Le détecteur d'absorbance est ajusté à 1 AUFS (Absorbance Unit Full Scale) et on enregistre la DO à 254 nm de cette solution à 0,575 UA254. La solution d'aptamères nucléiques biotinylés est injectée sur le gel streptavidin-agarose pré-conditionnée (" prépackée ") et mise en recirculation avec une pompe péristaltique à un débit de 2,5 ml/minute, soit un temps de contact sur le gel de 24 secondes (entrée/sortie E/S). Dans ces conditions, la DO à 254 nm se stabilise rapidement à 0,05 UA254, soit une valeur de 91% de couplage théorique, c'est-à-dire 0,455 mg d'aptamères nucléiques par millilitre de gel.

Un lavage avec un tampon 10 mM CaCl2 + 4 mM MgCl2 puis en NaCl 2M est réalisé, afin d'éliminer les aptamères nucléiques qui ne sont pas fixées spécifiquement aux molécules de streptavidine greffées sur le matériau de support solide.

**B. Résultats**

Une solution de Facteur H humain plasmatique purifié qui a été préalablement ajustée à 50 mM MgCl2, NaCl 50mM, et pH 7,5, est injectée sur le gel Aptamère-agarose (support d'affinité) avec une pompe péristaltique à un débit de 0,5 ml/minute soit un temps de contact avec le support d'affinité de 10 minutes (E/S).

Après injection, le gel est lavé en tampon tris 50 mM + NaCl 2M + 50 mM MgCl2 + à pH 7,5.

Un volume de 10 ml de solution non adsorbé est collecté.

Le Facteur H plasmatique est élué par un tampon tris 50 mM + EDTA 500 mM à pH 8,0. La collecte du pic d'élution est réalisée suivant le profil de DO.

La figure 3 illustre un profil de chromatographie du Facteur H humain plasmatique avec un suivi en continu des valeurs d'absorbance (D.O.) à 254 nanomètres.

Sur la Figure 3, de la gauche vers la droite de la figure, le pic n° 1 de la courbe d'absorption, après le moment de l'injection, illustre la fraction qui n'est pas retenue sur le support d'affinité. Le pic n° 2 de la courbe illustre la fraction de Facteur H qui est éluée.

Les résultats de la Figure 3 montrent que la fraction non retenue par le support d'affinité représente 26% du matériel de départ absorbant à 254 nanomètres. Les résultats de la Figure 3 montrent que la fraction de Facteur H humain plasmatique qui est retenue sur le support d'affinité, puis éluée, représente 74% du matériel de départ absorbant

à 254 nanomètres.

L'échantillon de Facteur H humain plasmatique de départ, le contenu de la fraction non retenue sur le support d'affinité, et le contenu de la fraction d'éluat ont été analysés sur gel d'électrophorèse SDS PAGE selon le protocole suivant : Gels NOVEX (Invitrogen) 10 puits, 4-12%, Bis-Tris; tampon de migration MES, migration à 200V pendant 50min. Coloration AgNO3 (kit GE).

Les résultats d'analyse sur gel d'électrophorèse sont représentés sur la Figure 5A.

Les résultats de la Figure 5A montrent que la solution de Facteur H humain plasmatique de départ purifié contient encore des impuretés visualisées en nitrate d'argent e. En revanche, les résultats de la Figure 5A montrent que la fraction d'élution comprend un matériel protéique migrant selon une seule bande malgré la grande sensibilité connu de la révélation au nitrate d'argent..

Les résultats d'analyse sur gel d'électrophorèse de la Figure 5A montrent que le support d'affinité sur lequel sont immobilisés des aptamères nucléiques MaptH1.1 est apte à purifier le Facteur H humain plasmatique et à améliorer très significativement le niveau de pureté d'un produit purifié par des approches traditionelles.

## Exemple 5 : Purification d'un Facteur H recombinant

Dans l'exemple 5, on a utilisé un support d'affinité qui a été préparé comme décrit à l'exemple 4.

On a utilisé une préparation de Facteur H humain consistant en un surnageant de culture de cellules recombinées produisant le Facteur H humain recombinant. Un surnageant de culture cellulaire de Facteur H humain recombinant qui a été préalablement ajusté à Tris 50mM, NaCl 50mM, 10mMMgCl2 et et pH 7,5, est injecté sur le gel Aptamère-agarose (support d'affinité) avec une pompe péristaltique à un débit de 0,5 ml/minute soit un temps de contact avec le support d'affinité de 20 minutes (E/S).

Après injection, le gel est lavé en tampon tris 50 mM + NaCl 2 M + 10 mM MgCl2 + à pH 7,5.

Un volume de 10 ml de solution non adsorbé est collecté.

Le Facteur H humain recombinant est élué par un tampon tris 50 mM + EDTA 500 mM à pH 8,0. La collecte du pic d'élution est réalisée suivant le profil de DO.

La figure 4 illustre un profil de chromatographie du Facteur H humain recombinant avec un suivi en continu des valeurs d'absorbance (D.O.) à 254 nanomètres.

Sur la Figure 4, de la gauche vers la droite de la figure, le pic n° 1 de la courbe d'absorption, après le moment de l'injection, illustre la fraction qui n'est pas retenue sur le support d'affinité. Le pic n° 2 de la courbe illustre la fraction de Facteur H recombinant qui est éluée. Le pic n° 3 illustre la fraction résultant d'une étape de régénération du support d'affinité.

Les résultats de la Figure 4 montrent que la fraction non retenue par le support d'affinité représente 95% du matériel de départ absorbant à 254 nanomètres. Les résultats de la Figure 4 montrent que la fraction de Facteur H humain recombinant qui est retenue sur le support d'affinité, puis éluée, représente 2% du matériel de départ absorbant à 254 nanomètres. Enfin, les résultats de la Figure 4 montrent que 3% du matériel de départ absorbant à 254 nanomètres a été retenu de manière non spécifique sur le support d'affinité.

L'échantillon de Facteur H humain recombinant de départ, le contenu de la fraction non retenue sur le support d'affinité, et le contenu de la fraction d'éluat ont été analysés sur gel d'électrophorèse SDS PAGE selon le protocole suivant : Gels NOVEX (Invitrogen) 10 puits, 4-12%, Bis-Tris; tampon de migration MES, migration à 200V pendant 50min. Coloration AgNO3 (kit GE).

Les résultats d'analyse sur gel d'électrophorèse sont représentés sur la Figure 5B.

Les résultats de la Figure 5B montrent que la solution de Facteur H humain recombinant de départ est très hétérogène et comprend une grande variété de protéines cellulaires de poids moléculaires distincts. Les résultats de la Figure 5B montrent aussi une grande hétérogénéité des entités protéiques contenues dans la fraction non retenue. En revanche, les résultats de la Figure 5B montrent que la fraction d'élution est constitué d'un produit très pure qui comprend un matériel protéique migrant selon une seule bande et ceux malgré la sensibilité de la révélation au nitrate d'argent., Les résultats d'analyse sur gel d'électrophorèse de la Figure 5B montrent que le support d'affinité sur lequel sont immobilisés des aptamères nucléiques MaptH1.1 est apte à purifier le Facteur H humain recombinant, à partir d'un échantillon de surnageant de culture cellulaire complexe comprenant une grande variété de protéines plasmatiques et permet d'obtenir en une seule étape un très haut niveau de pureté.

## Exemple 6 : Sélection d'aptamères nucléiques anti-FH optimisés

### A. Matériels et Méthodes

Appareil utilisé: Biacore T100

Puce : Le facteur H (LFB) a été immobilisé en utilisant la chimie NHS/EDC (chip CM5, GE) à 5813 RU en flow cell 2-1

(FC2-1). Aucune protéine n'est fixée sur la flow cell n°1 (FC1) mais cette flow cell a subi le même traitement que la flow cell n°2. L'échantillon injecté passe sur la FC1 et la FC2 afin de soustraire rigoureusement le bruit de fond dû à des interactions non spécifiques.
Tampon de course et dilution des échantillons : Tris 50 mM, NaCl 50 mM, MgCl2 50 mM, pH 7.5
Flux : 30μL/min, injection pendant 60 sec, dissociation pendant 60 sec
Signal : Signal sur FC2 avec soustraction du signal sur FC1
Régénération :EDTA 500 mM pH 8 dans de l'eau, 2 fois 60 sec

**B. Résultats**

Une pluralité d'aptamères nucléiques dérivés de l'aptamère nucléique MaptH1.1 de séquence SEQ ID N° 144 ont été synthétisés, respectivement les aptamères suivants : (i) MaptH1.1 N13-48 (SEQ ID N° 239), (ii) MaptH1.1 N19-58 (SEQ ID N° 240), (iii) MaptH1.1 N19-53 (SEQ ID N° 241) ; (iv) MaptH1.1 N19-48 (SEQ ID N° 242) et (v) MaptH1.1 N22-45 (SEQ ID N° 243). Les séquences de ces aptamères nucléiques sont représentées dans le tableau 4 ci-dessous.

Tableau 4

| Ref | SEQ ID N0 | Séquence |
|---|---|---|
| MaptH1.1 N13-48 | 239 | caggtctcgg gcacgggtca ggcggttata cggtgccc |
| MaptH1.1 N19-58 | 240 | gg gcacgggtca ggcggttata cggtgcccat tgttcttt |
| MaptH1.1 N19-53 | 241 | gg gcacgggtca ggcggttata cggtgcccat tgt |
| MaptH1.1 N19-48 | 242 | gg gcacgggtca ggcggttata cggtgccc |
| MaptH1.1 N22-45 | 243 | cacgggtca ggcggttata cggtg |

L'aptamère MaptH1.1 N13-48 est un acide nucléique consistant en la séquence allant du nucléotide en position 13 jusqu'au nucléotide en position 48 de l'aptamère nucléique Mapt H1.1 de séquence SEQ ID N° 144.
L'aptamère MaptH1.1 N19-58 est un acide nucléique consistant en la séquence allant du nucléotide en position 19 jusqu'au nucléotide en position 58 de l'aptamère nucléique Mapt H1.1 de séquence SEQ ID N° 144.
L'aptamère MaptH1.1 19-53 est un acide nucléique consistant en la séquence allant du nucléotide en position 19 jusqu'au nucléotide en position 53 de l'aptamère nucléique Mapt H1.1 de séquence SEQ ID N° 144.
L'aptamère MaptH1.1 N19-48 est un acide nucléique consistant en la séquence allant du nucléotide en position 19 jusqu'au nucléotide en position 48 de l'aptamère nucléique Mapt H1.1 de séquence SEQ ID N° 144.
L'aptamère MaptH1.1 N22-45 est un acide nucléique consistant en la séquence allant du nucléotide en position 22 jusqu'au nucléotide en position 45 de l'aptamère nucléique Mapt H1.1 de séquence SEQ ID N° 144.
L'aptitude de chacun des aptamères ci-dessus à se lier au Facteur H humain recombinant a été testée par mesure de fixation des aptamères sur un support obtenu conformément à la Section Matériels et Méthodes, selon la technique Biacore®.
Les résultats sont représentés sur la Figure 7.
Les résultats de la Figure 7 montrent que tous les aptamères dérivés de l'aptamère nucléique MaptH1.1 de séquence SEQ ID N° 144 se fixent au Facteur H humain immobilisé sur le support.
En particulier, il est observé que l'aptamère nucléique MaptH1.1 N22-45, qui est l'aptamère nucléique ayant la longueur la plus réduite, a conservé l'aptitude de l'aptamère parent MaptH1.1 à se fixer au Facteur H humain, bien que son affinité pour le Facteur H humain soit plus faible que pour l'aptamère nucléique parent MaptH1.1. En conséquence, les résultats montrent que la partie de l'aptamère nucléique MaptH1.1 de séquence SEQ ID N° 144 allant du nucléotide en position 22 jusqu'au nucléotide en position 45 est suffisante pour conférer audit aptamère nucléique la capacité à se lier au Facteur H humain.
Ces résultats sont confirmés par le fait que tous les autres aptamères nucléiques, respectivement les aptamères (i) MaptH1.1 N13-48 (SEQ ID N° 239), (ii) MaptH1.1 N19-58 (SEQ ID N° 240), (iii) MaptH1.1 N19-53 (SEQ ID N° 241) et (iv) MaptH1.1 N19-48 (SEQ ID N° 242), comprennent tous la partie de l'aptamère parent MaptH1.1 allant du nucléotide en position 22 jusqu'au nucléotide en position 45 et ont tous conservé une bonne capacité de liaison au Facteur H humain.
Par ailleurs, une affinité croissante avec le Facteur H humain est mesurée pour la succession des aptamères nucléiques MaptH1.1 N22-45 (SEQ ID N° 243), MaptH1.1 N19-48 (SEQ ID N° 242), MaptH1.1 N19-53 (SEQ ID N° 241),

MaptH1.1 N19-58 (SEQ ID N° 240) et MaptH1.1 N13-48 (SEQ ID N° 239).

Ainsi, parmi les aptamères nucléiques comprenant la région de l'aptamère parent MaptH1.1 allant du nucléotide en position 22 jusqu'au nucléotide en position 45 qui ont été testés, c'est l'aptamère nucléique MaptH1.1 N13-48 (SEQ ID N° 239) qui possède la plus forte affinité pour le Facteur H humain. L'aptamère MaptH1.1 N13-48 (SEQ ID N° 239) peut aussi être désigné « MaptH1.1CSO » aux fins de la présente description.

**Revendications**

1. Aptamère nucléique se liant au Facteur H et comprenant la séquence SEQ ID NO : 243.

2. Aptamère nucléique selon la revendication 1, qui est de formule (III) suivante :

$$5'\text{-}[N1]x\text{-}[SEQ\ ID\ N°\ 243]\text{-}[N2]y\text{-}3'\ (III),$$

dans laquelle :

- " x " est un entier égal à 0 ou 1, et
- " y " est un entier égal à 0 ou 1,
- N1 est un acide nucléique ayant de 1 à 100 nucléotides de longueur, et
- N2 est un acide nucléique ayant de 1 à 100 nucléotides de longueur.

3. Aptamère nucléique selon l'une des revendications 1 et 2, choisi dans le groupe constitué par les acides nucléiques de séquence SEQ ID N° 144 et 239 à 243.

4. Aptamère nucléique selon l'une des revendications 1 à 3, **caractérisé en ce qu'**il se lie à un Facteur H choisi parmi un Facteur H plasmatique, un Facteur H recombinant et un Facteur H transgénique.

5. Aptamère nucléique selon l'une des revendications 1 à 4, **caractérisé en ce qu'**il se lie spécifiquement à un Facteur H choisi parmi un Facteur H humain et un Facteur H non humain.

6. Aptamère nucléique selon l'une des revendications 1 à 5, **caractérisé en ce qu'**il est un aptamère désoxyribonucléotidique.

7. Composé de formule (II) suivante :

$$[IMM]_x\text{-}[SPAC]_y\text{-}[APT]\ (II),$$

dans laquelle :

- [IMM] signifie un composé d'immobilisation sur un support,
- [SPAC] signifie une chaîne espaceur,
- [APT] signifie un aptamère nucléique se liant spécifiquement au Facteur H tel que défini dans l'une des revendications 1 à 6,
- x est un entier égal à 0 ou 1,
- y est un entier égal à 0 ou 1.

8. Support d'affinité pour la fixation sélective du Facteur H, comprenant un matériau support solide sur lequel sont

immobilisés des aptamères nucléiques selon l'une quelconque des revendications 1 à 6, ou un composé de formule (II) selon la revendication 7.

**9.** Procédé d'obtention d'un support d'affinité pour la fixation sélective du Facteur H comprenant les étapes suivantes :

a) fournir un support solide comprenant à sa surface des groupes acides carboxyliques activés,
b) fournir des aptamères nucléiques selon l'une des revendications 1 à 6, ou un composé de formule (II) selon la revendication 7, comprenant au moins une fonction amine réactive, et
c) réaliser le couplage desdits aptamères nucléiques, ou dudit composé de formule (II), avec les groupes acides carboxyliques activés présents à la surface dudit support solide dans des conditions de pH inférieur à 5,

étant entendu que l'ordre des étapes a) et b) est indifférent.

**10.** Procédé pour immobiliser un Facteur H sur un support, comprenant une étape au cours de laquelle on met en contact un échantillon contenant le Facteur H avec un support d'affinité selon la revendication 8.

**11.** Procédé pour la purification d'un Facteur H comprenant les étapes suivantes :

a) mettre en contact un échantillon contenant un Facteur H avec un support d'affinité selon la revendication 8, afin de former des complexes entre (i) les aptamères nucléiques immobilisés sur ledit support d'affinité et (ii) ledit Facteur H,
b) libérer le Facteur H à partir des complexes formés à l'étape a), et
c) récupérer le Facteur H sous forme purifiée.

**12.** Procédé selon l'une des revendications 10 et 11, **caractérisé en ce que** le Facteur H est choisi parmi un Facteur H plasmatique, un Facteur H recombinant et un Facteur H transgénique.

**13.** Procédé selon l'une des revendications 9 à 12, **caractérisé en ce que** le Facteur H est choisi parmi un Facteur H humain et un Facteur H non humain.

**Patentansprüche**

**1.** Nukleinsäure-Aptamer, das an Faktor H bindet und die Sequenz SEQ ID NO: 243 umfasst.

**2.** Nukleinsäure-Aptamer gemäß Anspruch 1, das die folgende Formel (III) besitzt:

5'-[N1]x-[SEQ ID NO: 243]-[N2]y-3' (III)

wobei:

- "x" eine ganze Zahl gleich 0 oder 1 ist, und
- "y" eine ganze Zahl gleich 0 oder 1 ist,
- N1 eine Nukleinsäure mit einer Länge von 1 bis 100 Nukleotiden ist, und
- N2 eine Nukleinsäure mit einer Länge von 1 bis 100 Nukleotiden ist.

**3.** Nukleinsäure-Aptamer gemäß einem der Ansprüche 1 und 2, ausgewählt aus der Gruppe, bestehend aus den Nukleinsäuren der Sequenz SEQ ID NO: 144 und 239 bis 243.

**4.** Nukleinsäure-Aptamer gemäß einem der Ansprüche 1 bis 3, **dadurch gekennzeichnet, dass** es an einen Faktor H bindet, ausgewählt aus einem plasmatischen Faktor H, einem rekombinanten Faktor H und einem transgenen Faktor H.

**5.** Nukleinsäure-Aptamer gemäß einem der Ansprüche 1 bis 4, **dadurch gekennzeichnet, dass** es spezifisch an einen Faktor H bindet, ausgewählt aus einem humanen Faktor H und einem nichthumanen Faktor H.

**6.** Nukleinsäure-Aptamer gemäß einem der Ansprüche 1 bis 5, **dadurch gekennzeichnet, dass** es ein Desoxyribonukleinsäure-Aptamer ist.

**7.** Verbindung der folgenden Formel (II):

$$[IMM]_x\text{-}[SPAC]_y\text{-}[APT]\ (II).$$

wobei:

- [IMM] eine Verbindung zur Immobilisierung auf einem Träger bedeutet,
- [SPAC] eine Spacer-Kette bedeutet,
- [APT] ein Nukleinsäure-Aptamer bedeutet, das spezifisch an Faktor H bindet, wie es in einem der Ansprüche 1 bis 6 definiert ist,
- x eine ganze Zahl gleich 0 oder 1 ist,
- y eine ganze Zahl gleich 0 oder 1 ist.

**8.** Affinitätsträger für die selektive Bindung von Faktor H, umfassend ein festes Trägermaterial, auf dem Nukleinsäure-Aptamere gemäß einem der Ansprüche 1 bis 6 oder eine Verbindung der Formel (II) gemäß Anspruch 7 immobilisiert werden.

**9.** Verfahren zum Erhalt eines Trägers für die selektive Bindung von Faktor H, umfassend die folgenden Schritte:

a) Bereitstellen eines festen Trägers, umfassend aktivierte Carboxylsäuregruppen auf seiner Oberfläche,
b) Bereitstellen von Nukleinsäure-Aptameren gemäß einem der Ansprüche 1 bis 6 oder einer Verbindung der Formel (II) gemäß Anspruch 7, umfassend wenigstens eine reaktive Aminfunktion, und
c) Durchführung der Kopplung besagter Nukleinsäure-Aptamere oder besagter Verbindung der Formel (II) mit den aktiven Carboxylsäuregruppen, die auf besagtem festen Träger vorhanden sind, unter Bedingungen eines pH-Werts unter 5,

wobei einzusehen ist, dass die Reihenfolge der Schritte a) und b) beliebig ist.

**10.** Verfahren zur Immobilisierung eines Faktors H auf einem Träger, umfassend einen Schritt, in dessen Verlauf eine Probe, die den Faktor H enthält, mit einem Affinitätsträger gemäß Anspruch 8 in Kontakt gebracht wird.

**11.** Verfahren zur Reinigung eines Faktors H, umfassend die folgenden Schritte:

a) Inkontaktbringen einer Probe, die einen Faktor H enthält, mit einem Affinitätsträger gemäß Anspruch 8, um Komplexe zwischen (i) den Nukleinsäure-Aptameren, die auf besagtem Affinitätsträger immobilisiert sind, und (ii) besagtem Faktor H zu bilden,
b) Freisetzen des Faktors H aus den in Schritt a) gebildeten Komplexen, und
c) Wiedergewinnen des Faktors H in gereinigter Form.

**12.** Verfahren gemäß einem der Ansprüche 10 und 11, **dadurch gekennzeichnet, dass** der Faktor H aus einem plasmatischen Faktor H, einem rekombinanten Faktor H und einem transgenen Faktor H ausgewählt ist.

**13.** Verfahren gemäß einem der Ansprüche 9 bis 12, **dadurch gekennzeichnet, dass** der Faktor H aus einem humanen Faktor H und einem nichthumanen Faktor H ausgewählt ist.

## Claims

**1.** A nucleic aptamer which binds to Factor H and which comprises SEQ ID NO:243.

**2.** The nucleic aptamer according to claim 1, which is of formula (III) as follows:

$$5'\text{-}[N1]x\text{-}[SEQ\ ID\ NO{:}243]\text{-}[N2]y\text{-}3'\ (III),$$

wherein:

- x is an integer equal to 0 or 1, and
- y is an integer equal to 0 or 1,
- N1 is a nucleic acid having a length of 1 to 100 nucleotides, and
- N2 is a nucleic acid having a length of 1 to 100 nucleotides.

**3.** The nucleic aptamer according to one of claims 1 and 2, selected from the group consisting of nucleic acids of SEQ ID NO:144 and SEQ ID NO:239 to 243.

**4.** The nucleic aptamer according to any one of claims 1 to 3, **characterized in that** it binds to a Factor H selected from a plasma Factor H, a recombinant Factor H and a transgenic Factor H.

**5.** The nucleic aptamer according to any one of claims 1 to 4, **characterized in that** it specifically binds to a Factor H selected from a human Factor H and a non-human Factor H.

**6.** The nucleic aptamer according to any one of claims 1 to 5, **characterized in that** it is a deoxyribonucleotide aptamer.

**7.** A compound of formula (II) as follows:

$$[IMM]_x\text{-}[SPAC]_y\text{-}[APT] \qquad (II)$$

wherein:

- [IMM] means a compound for immobilization on a support,
- [SPAC] means a spacer chain,
- [APT] means a nucleic acid which specifically binds to Factor H as defined in any one of claims 1 to 6,
- x is an integer equal to 0 or 1,
- y is an integer equal to 0 or 1.

**8.** An affinity support for the selective binding of Factor H, comprising a solid support material on which nucleic aptamers of any one of claims 1 to 6, or a compound of formula (II) of claim 7, are immobilized.

**9.** A method for obtaining an affinity support for the selective binding of Factor H, comprising the following steps:

a) providing a solid support comprising activated carboxylic acid groups at its surface,
b) providing nucleic aptamers of any one of claims 1 to 6, or a compound of formula (II) of claim 7, comprising at least one reactive amine function, and
c) coupling said nucleic aptamers, or said compound of formula (II), with the activated carboxylic acid groups present at the surface of said solid support under pH conditions below 5,

wherein steps a) and b) may be in any order.

**10.** A method for immobilizing a Factor H on a support, comprising a step wherein a sample containing Factor H is contacted with an affinity support of claim 8.

**11.** A method for purifying a Factor H, comprising the following steps:

a) contacting a sample containing a Factor H with an affinity support of claim 8, so as to form complexes between (i) the nucleic aptamers immobilized on said affinity support and (ii) said Factor H,
b) releasing the Factor H from the complexes formed in step a), and
c) recovering the Factor H in purified form.

**12.** The method according to one of claims 10 and 11, **characterized in that** the Factor H is selected from a plasma Factor H, a recombinant Factor H and a transgenic Factor H.

**13.** The method according to one of claims 9 to 12, **characterized in that** the Factor H is selected from a human Factor

H and a non-human Factor H.

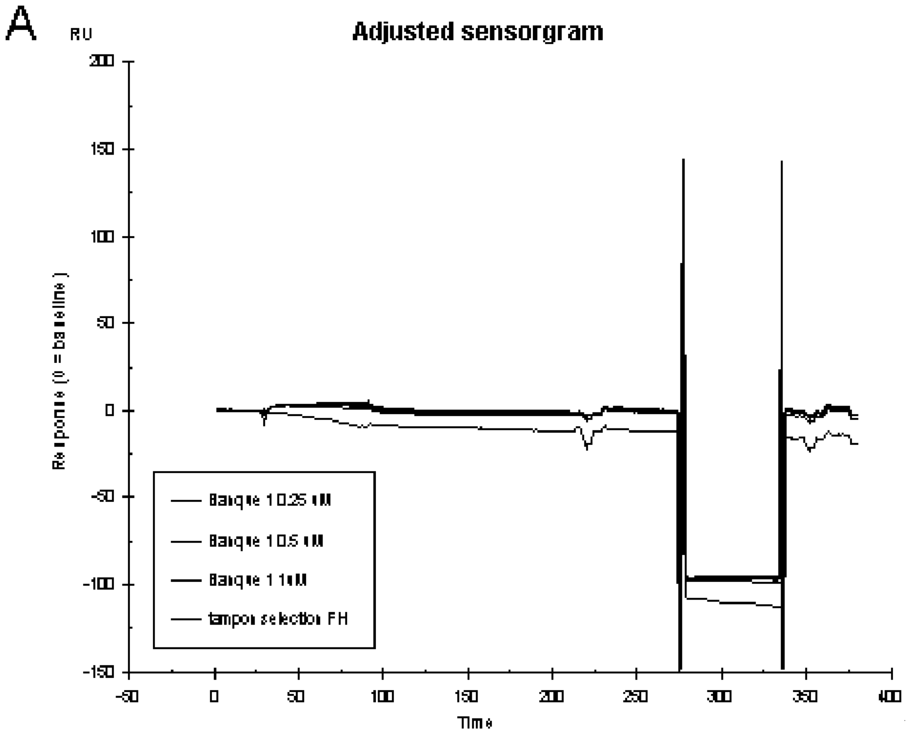


**Figure 1A**

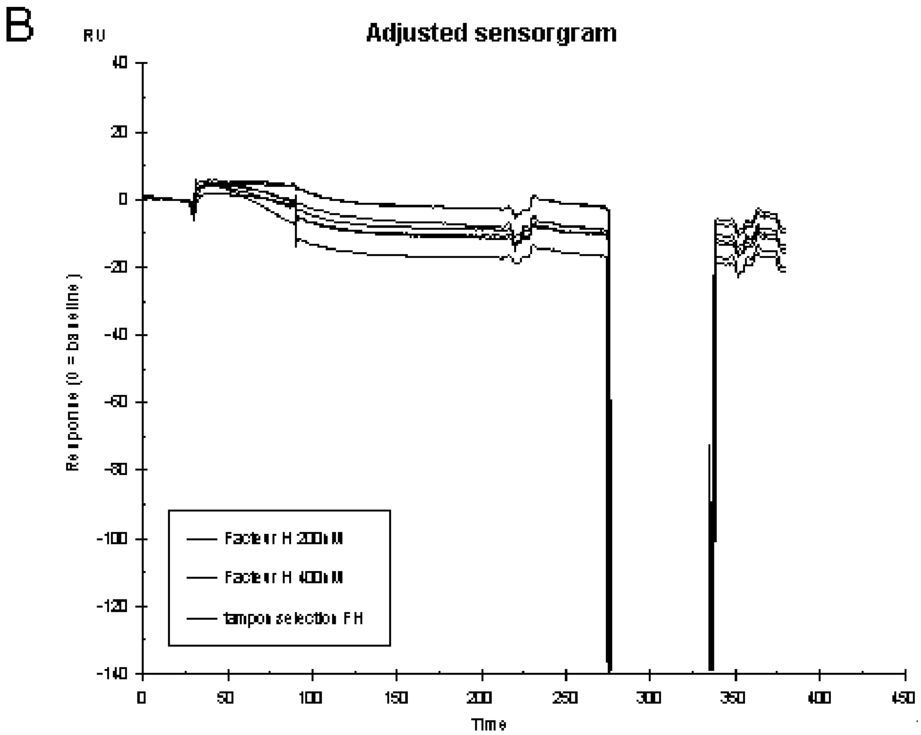


**Figure 1B**

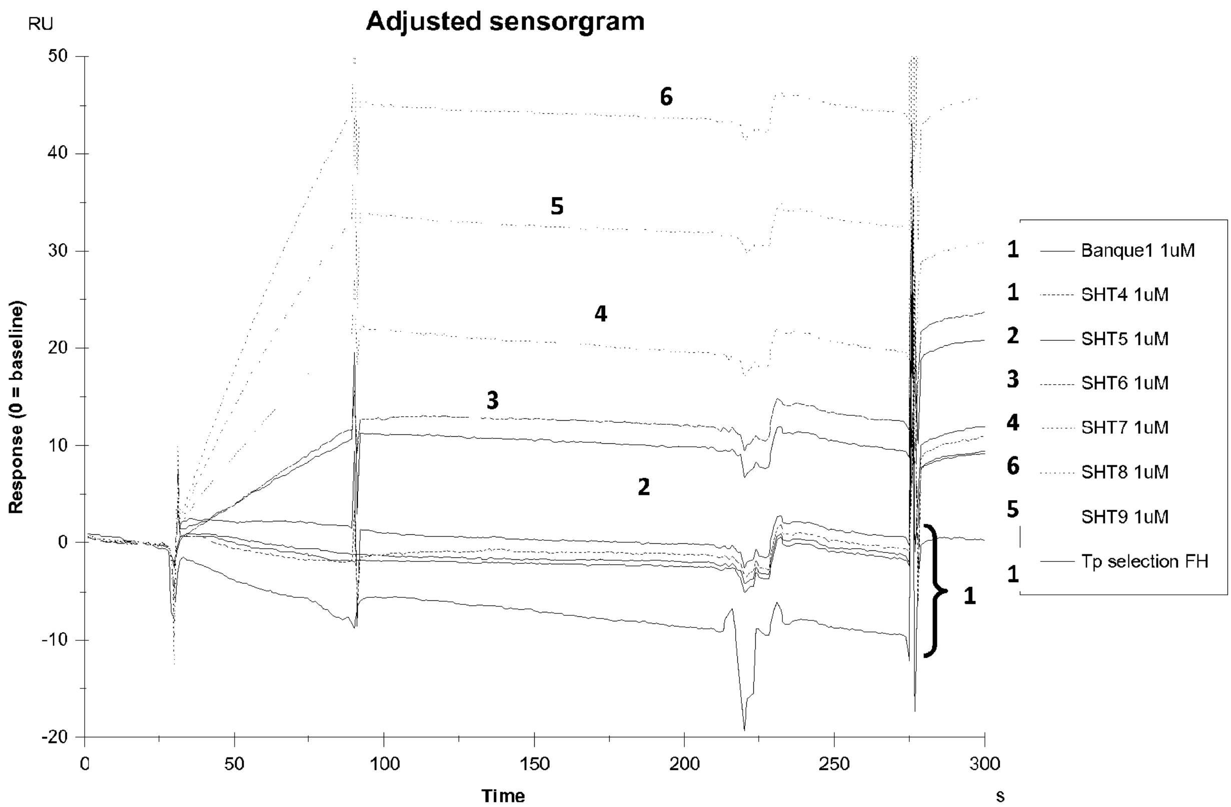
RU
Adjusted sensorgram
50
40
30
20
10
0
-10
-20
Response (0 = baseline)
6
5
4
3
2
1
0
50
100
150
200
250
300
Time
s
1 Banque1 1uM
1 SHT4 1uM
2 SHT5 1uM
3 SHT6 1uM
4 SHT7 1uM
6 SHT8 1uM
5 SHT9 1uM
1 Tp selection FH

Figure 2A

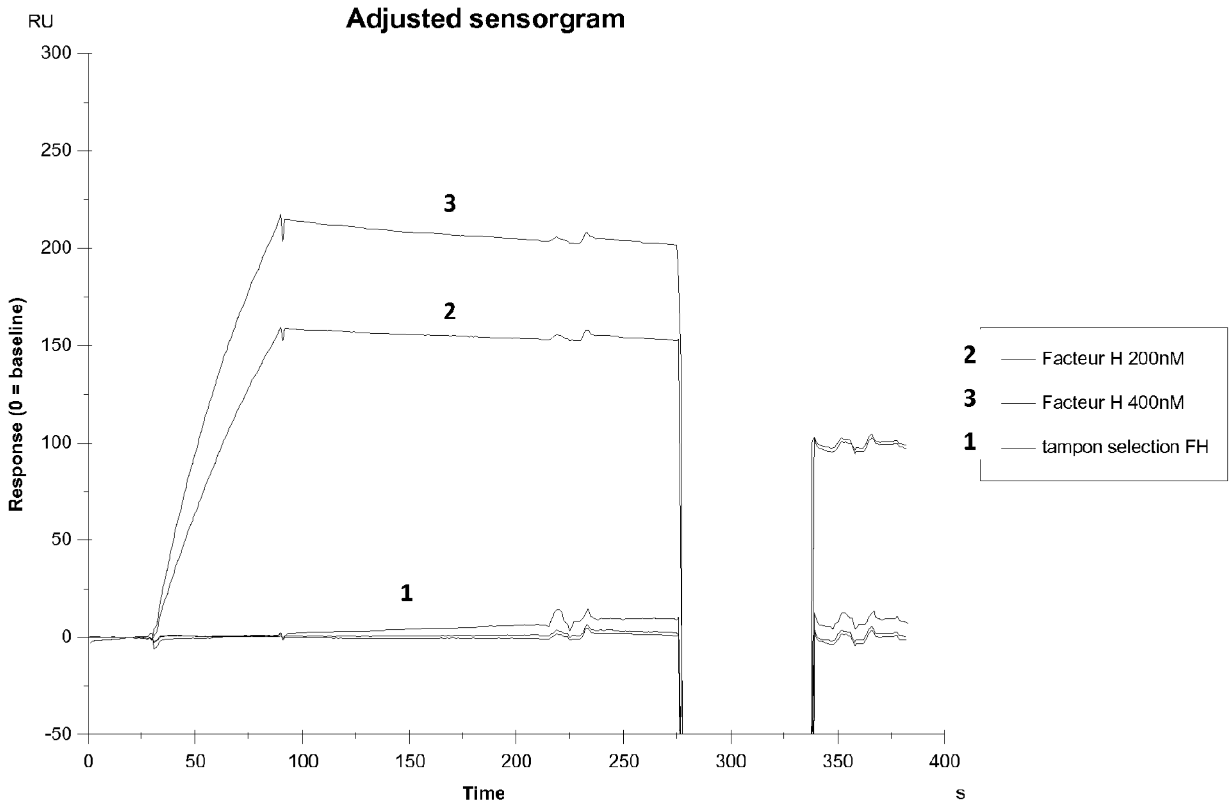
Adjusted sensorgram
RU
300
250
200
150
100
50
0
-50
Response (0 = baseline)
0
50
100
150
200
250
300
350
400
Time
s
3
2
1
2 Facteur H 200nM
3 Facteur H 400nM
1 tampon selection FH

**Figure 2B**

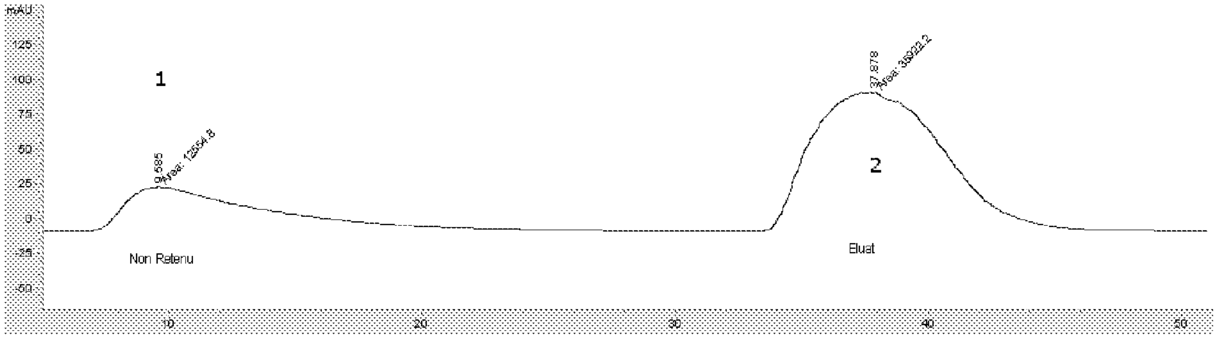


**Figure 3**

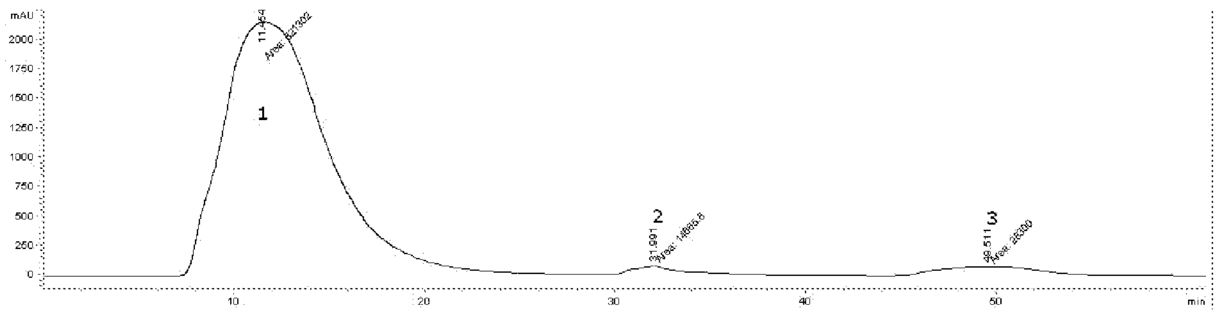


**Figure 4**

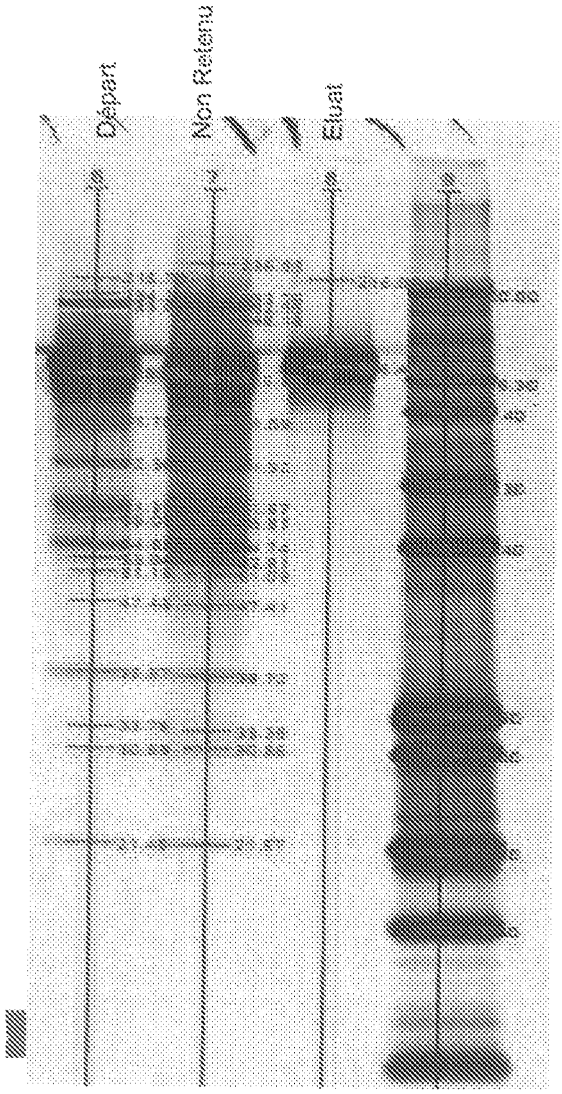


1 2 3 4

**Figure 5A**

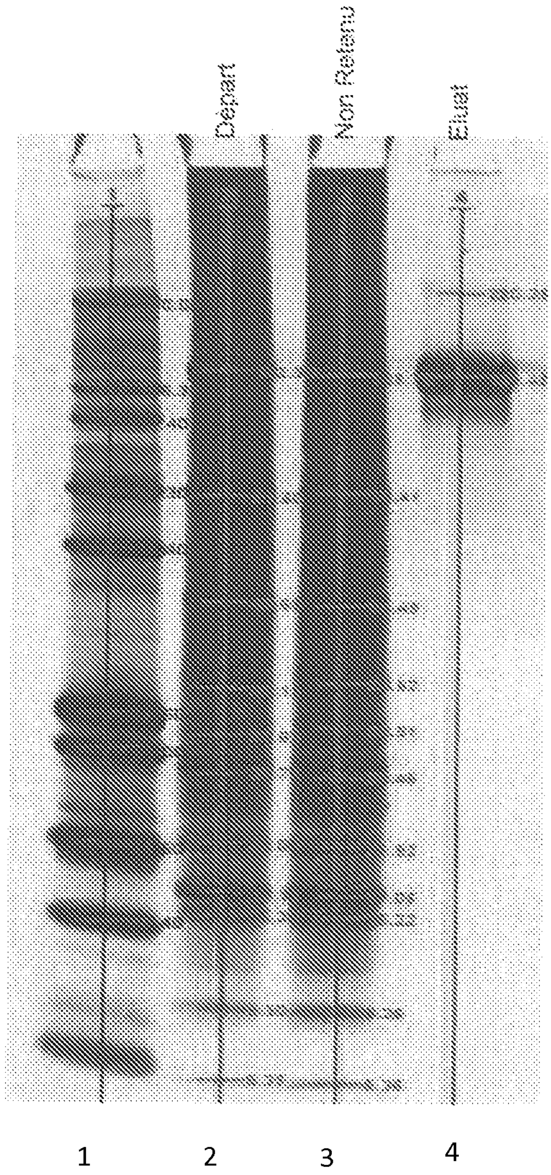


Figure 5B

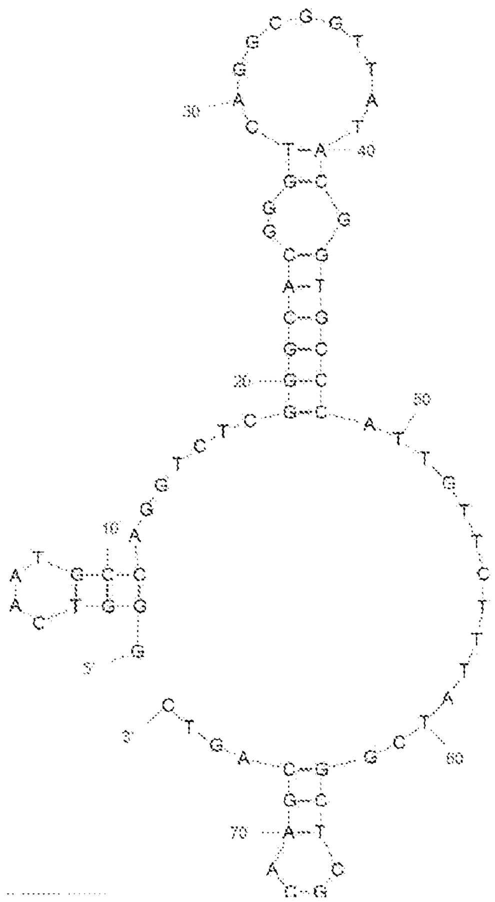


Figure 6A

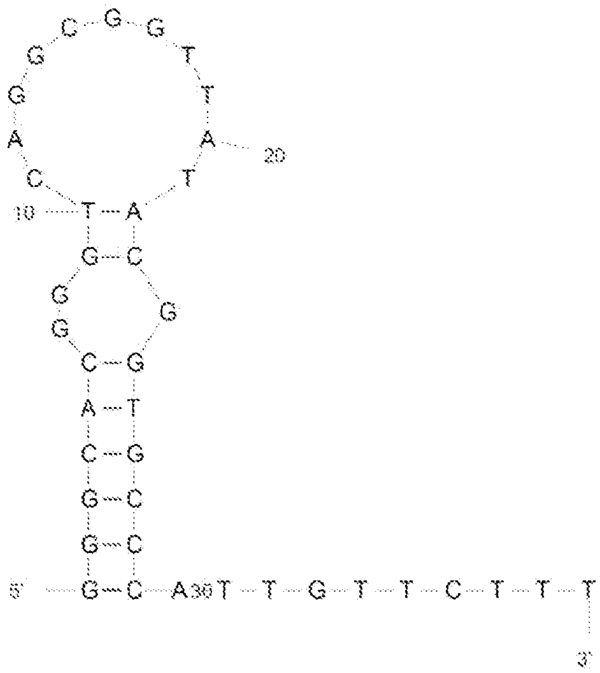


Figure 6B

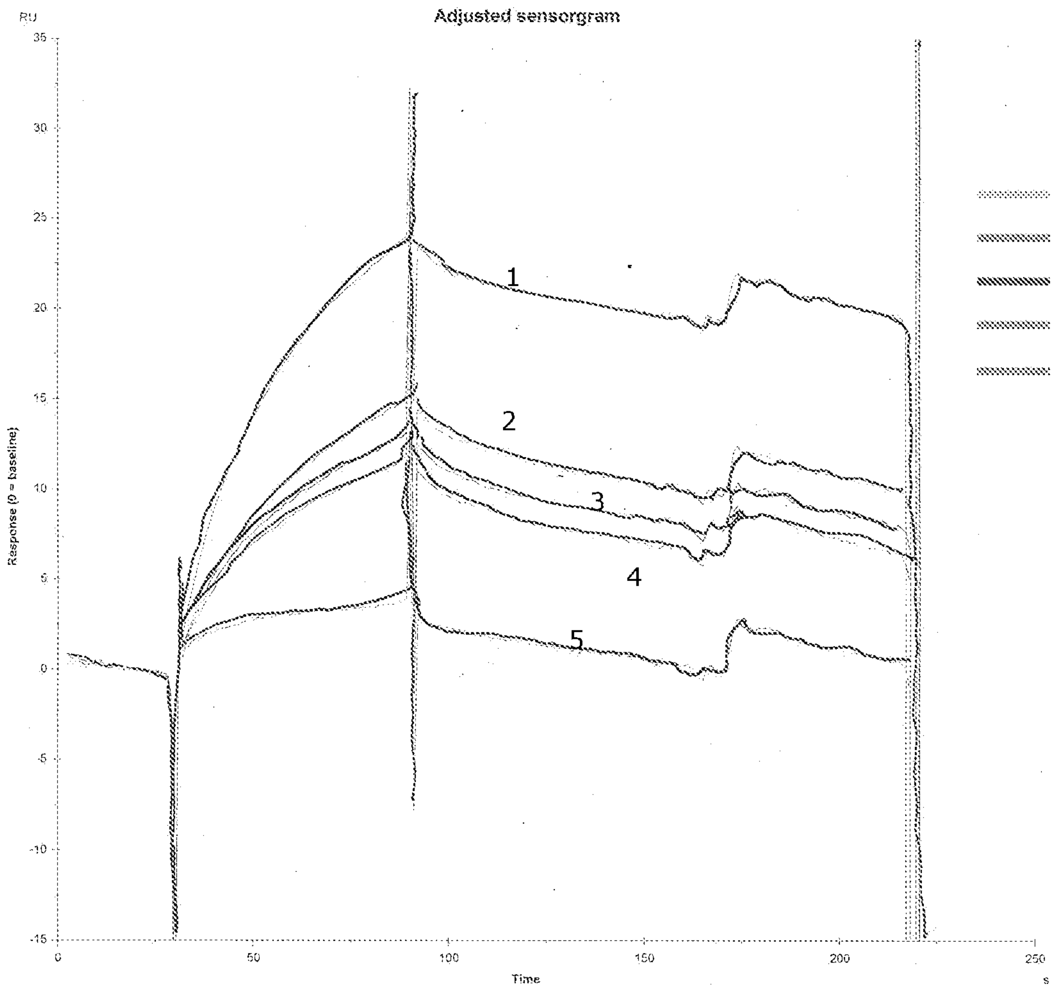
Adjusted sensorgram
RU
35
30
25
20
15
10
5
0
-5
-10
-15
Response (0 = baseline)
0
50
100
150
200
250
Time
s
1
2
3
4
5

Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2894145
- WO 2007038995 A
- WO 2008113589 A
- WO 2007066017 A
- WO 2011058284 A
- WO 2011058285 A
- EP 1972693 A
- WO 2008038696 A
- WO 2008025830 A
- WO 20070322359 A
- EP 0527063 A
- US 7045676 B
- EP 1739170 A
- WO 1991019813 A
- EP 0786469 A
- EP 668931 A
- EP 1695978 A
- EP 1493825 A
- FR 1160862


### Littérature non-brevet citée dans la description


- **HERMANSON GT.** Bioconjugate techniques. Académie Press, 1996, 239-242
- **SAMOSZUK M. K. et al.** *Antibody, Immunoconjugates Radiopharm.,* 1989, vol. 2 (1), 37-46
- **ROMIG et al.** *J Chomatogr B Biomed Sci ApI,* 1999, vol. 731 (2), 275-284
- **RAVELET et al.** *J Chromatogr A,* 2006, vol. 1 17 (1), 1-10
- **CONNOR et al.** *J Chromatogr A,* 2006, vol. 1 1 1 (2), 1 15-1 19
- **CHO et al.** *Electrophoresis,* 2004, vol. 25 (21 - 22), 3730-3739
- **ZHAO et al.** *Anal Chem,* 2008, vol. 80 (10), 3915-3920
- **MICHAUD et al.** *Anal Chem,* 2003, vol. 76, 1015-1020
- **BRUMBT et al.** *Anal Chem,* vol. 77, 1993-1998
- **PASCAL BAILON ; GEORGE K. EHRLICH ; WEN-JIAN FUNG ; WOLFGANG BERTHOLD.** An Overview of Affinity Chromatography. Humana Press, 2000
- **MOHR et al.** Affinity Chromatography: Practical and Theoretical Aspects. CRC Press, 1985
- **TUERK ; GOID.** Systematic évolution of ligands by exponential enrichment : RNA ligands to bacteriophage T4 DNA polymerase. *Science,* 1990, vol. 249 (4968), 505-10
- **ELLINGTON ; SZOSTAK.** In vitro sélection of RNA molécules that bind spécifie ligands. *Nature,* 30 Août 1990, vol. 346 (6287), 818-22

**SEQUENCE LISTING**

<110> LFB Biotechnologies

<120> Aptamères anti-FH, procédé pour leur obtention et utilisations

<130> PR94164

<150> FR 1160862
<151> 2011-11-28

<160> 243

<170> PatentIn version 3.5

<210> 1
<211> 18
<212> DNA
<213> artificial sequence

<220>
<223> aptamer

<400> 1
gggtcaatgc caggtctc 18

<210> 2
<211> 18
<212> DNA
<213> artificial sequence

<220>
<223> aptamer

<400> 2
atcggctcgc aagcagtc 18

<210> 3
<211> 40
<212> DNA
<213> artificial sequence

<220>
<223> aptamere

<400> 3
cggtctcgag atggggtctg gtggttatac gctctcagtg 40

<210> 4
<211> 40

<212> DNA
<213> artificial sequence

<220>
<223> aptamere

<400> 4
accgtcatcg agaagggtct ggtggttata cgttctcgtg 40

<210> 5
<211> 40
<212> DNA
<213> artificial sequence

<220>
<223> aptamere

<400> 5
ggaccgcgat ggagaggggt ctggtggtta tacgctatgc 40

<210> 6
<211> 40
<212> DNA
<213> artificial sequence

<220>
<223> aptamere

<400> 6
gggccgcgat ggagaggggt ctggtggtta tacgctatgt 40

<210> 7
<211> 40
<212> DNA
<213> artificial sequence

<220>
<223> aptamere

<400> 7
cggcagcaga tggacagggt caggtggtta tacgtgtggc 40

<210> 8
<211> 40
<212> DNA
<213> artificial sequence

<220>
<223> aptamere

<400> 8
gcgatgacca ataggggtca ggtggttata cgctttggat 40

<210> 9
<211> 38
<212> DNA
<213> artificial sequence

<220>
<223> aptamere

<400> 9
gggggcggcc gcagtagggg tctggtggtt atacgcgt 38

<210> 10
<211> 40
<212> DNA
<213> artificial sequence

<220>
<223> aptamere

<400> 10
gcagaggtgc gaggggcggt ggggtctggt ggttatacgc 40

<210> 11
<211> 40
<212> DNA
<213> artificial sequence

<220>
<223> aptamere

<400> 11
acgtcgagtt gtatggtggg gtcaggtggt tatacgcagc 40

<210> 12
<211> 40
<212> DNA
<213> artificial sequence

<220>
<223> aptamere

<400> 12
aaggccgaca cactgggggg tctggtggtt atacgcccgt 40

<210> 13
<211> 40
<212> DNA
<213> artificial sequence

<220>
<223> aptamere

<400> 13
gcatgatact actggggtct ggtggttata cgcaggtagg 40

<210> 14
<211> 40
<212> DNA
<213> artificial sequence

<220>
<223> aptamere

<400> 14
gaggaacgtg gggtcaggtg gttatacgca gggtaccggg 40

<210> 15
<211> 40
<212> DNA
<213> artificial sequence

<220>
<223> aptamere

<400> 15
ggcagggagg aggaatcggg acaggcggtt atacggattc 40

<210> 16
<211> 40
<212> DNA
<213> artificial sequence

<220>
<223> aptamere

<400> 16
cggaaagagt atggaacggg tcaggcggtt ataaggttac 40

<210> 17
<211> 40
<212> DNA
<213> artificial sequence

<220>
<223> aptamere

<400> 17
cacggcgtat gcgtagatcg atgggtctgg cggttatacg 40

<210> 18
<211> 40
<212> DNA
<213> artificial sequence

<220>
<223> aptamere

<400> 18
acaagagctg tattagtcgg gtctggcggt tatacggact 40

<210> 19
<211> 40
<212> DNA
<213> artificial sequence

<220>
<223> aptamere

<400> 19
agggcggatt gcggtacggt tgggtctggt ggttatacga 40

<210> 20
<211> 40
<212> DNA
<213> artificial sequence

<220>
<223> aptamere

<400> 20
tgcggagcat tacattcacg ggtctggcgg ttatacggtg 40

<210> 21
<211> 40
<212> DNA
<213> artificial sequence

<220>
<223> aptamere

<400> 21
ggggggccgg ttatacgggt caggcggtta tacggtattc 40

<210> 22
<211> 40
<212> DNA
<213> artificial sequence

<220>
<223> aptamere

<400> 22
gaggggctgg ggtcacaggg gtcggtgtgg ttattattct 40

<210> 23
<211> 40
<212> DNA
<213> artificial sequence

<220>
<223> aptamere

<400> 23
gggacaggcg gttatacgga gagttctggt gtagggttgg 40

<210> 24
<211> 40
<212> DNA
<213> artificial sequence

<220>
<223> aptamere

<400> 24
ggggcaggcg gttatacgga gagcttctta catgggccct 40

<210> 25
<211> 40
<212> DNA

<213> artificial sequence

<220>
<223> aptamere

<400> 25
gggtcaggcg gttatacgga gacaccttgc tgtgttaggc 40

<210> 26
<211> 40
<212> DNA
<213> artificial sequence

<220>
<223> aptamere

<400> 26
cggggcaggc ggttatacgg gagaacggta tggggggact 40

<210> 27
<211> 40
<212> DNA
<213> artificial sequence

<220>
<223> aptamere

<400> 27
cggggtctgg tggttatacg ctgagatggg tgctacagag 40

<210> 28
<211> 40
<212> DNA
<213> artificial sequence

<220>
<223> aptamere

<400> 28
accgggtcag gcggttatac gggtgggggc aggtacatac 40

<210> 29
<211> 40
<212> DNA
<213> artificial sequence

<220>
<223> aptamere

<400> 29
acaggggtca gtggaagtta tagactggga aggcatacaa 40

<210> 30
<211> 40
<212> DNA
<213> artificial sequence

<220>

<223> aptamere

<400> 30
gggcacgggt caggcggtta tacggtgccc attgttcttt 40

<210> 31
<211> 40
<212> DNA
<213> artificial sequence

<220>
<223> aptamere

<400> 31
acgggtcagg cggttatacg gtgtggcttg atggtgactt 40

<210> 32
<211> 40
<212> DNA
<213> artificial sequence

<220>
<223> aptamere

<400> 32
gggtcaggcg gttatacgga ggcctcgctg aacccagcta 40

<210> 33
<211> 39
<212> DNA
<213> artificial sequence

<220>
<223> aptamere

<400> 33
gcaacgggtc aggcggttat acggttcgta tcctggcga 39

<210> 34
<211> 40
<212> DNA
<213> artificial sequence

<220>
<223> aptamere

<400> 34
aagtggggtc tggtggttat acgcggttgt ggtgattgac 40

<210> 35
<211> 40
<212> DNA
<213> artificial sequence

<220>
<223> aptamere

<400> 35

gcgggtctgg tggttatacg tcgaatttgt taaattgcca 40

<210> 36
<211> 40
<212> DNA
<213> artificial sequence

<220>
<223> aptamere

<400> 36
aatcagaagg gtctggtggt tatacgttca tgtattgggt 40

<210> 37
<211> 40
<212> DNA
<213> artificial sequence

<220>
<223> aptamere

<400> 37
ggatggggtc aggtggttat acgctctgag tggttttggt 40

<210> 38
<211> 40
<212> DNA
<213> artificial sequence

<220>
<223> aptamere

<400> 38
ggggtgcgag ggtctggtgg ttatacgtcg tcacctctag 40

<210> 39
<211> 40
<212> DNA
<213> artificial sequence

<220>
<223> aptamere

<400> 39
tgggggaggg agggtctggg ggttatacgt cactagcaaa 40

<210> 40
<211> 40
<212> DNA
<213> artificial sequence

<220>
<223> aptamere

<400> 40
gaggggcttt tgggagggac tggtggttat acgtcccaat 40

<210> 41

<211> 40
<212> DNA
<213> artificial sequence

<220>
<223> aptamere

<400> 41
gcggtggacg gggctggcgg ttatacggtc cttacgcggt 40

<210> 42
<211> 40
<212> DNA
<213> artificial sequence

<220>
<223> aptamere

<400> 42
gcggctgtgg ggtctggtgg ttatacgcac atacgcgctg 40

<210> 43
<211> 40
<212> DNA
<213> artificial sequence

<220>
<223> aptamere

<400> 43
ccggttaccg cgctaagctt ggcgatggtg ttagtggctg 40

<210> 44
<211> 40
<212> DNA
<213> artificial sequence

<220>
<223> aptamere

<400> 44
ccggttaccg ttacgttgga cgaggaatgg tgttcgtggc 40

<210> 45
<211> 40
<212> DNA
<213> artificial sequence

<220>
<223> aptamere

<400> 45
ccggttactg ccgtagttgt cttacggtgg tgttagtggc 40

<210> 46
<211> 40
<212> DNA
<213> artificial sequence

<220>
<223> aptamere

<400> 46
ccggttactt ccgtagctgt cttacggtgg tgttagtggc 40

<210> 47
<211> 40
<212> DNA
<213> artificial sequence

<220>
<223> aptamere

<400> 47
ccggttaccg cattagtggt gtttgtggcg ttgagatggc 40

<210> 48
<211> 40
<212> DNA
<213> artificial sequence

<220>
<223> aptamere

<400> 48
ccggttacct ccattggtgg tgttagtggc tttgtaggat 40

<210> 49
<211> 40
<212> DNA
<213> artificial sequence

<220>
<223> aptamere

<400> 49
ccggttacca gttggtgttt gtggctttgc acgtctgcat 40

<210> 50
<211> 40
<212> DNA
<213> artificial sequence

<220>
<223> aptamere

<400> 50
ccggttacca gttggtgttt gtggctttgc acgtgtgcat 40

<210> 51
<211> 43
<212> DNA
<213> artificial sequence

<220>
<223> aptamere

<400> 51
ccggttacca gttggtgttg ttagtggctt tgcacgtctg cat 43

<210> 52
<211> 40
<212> DNA
<213> artificial sequence

<220>
<223> aptamere

<400> 52
ccggttacca actatggtgt tagtggcatc gatcgggatc 40

<210> 53
<211> 38
<212> DNA
<213> artificial sequence

<220>
<223> aptamere

<400> 53
ccggttacct ttggtgtttg tggcatgggc gtcggggc 38

<210> 54
<211> 40
<212> DNA
<213> artificial sequence

<220>
<223> aptamere

<400> 54
ccggttaccg tttggtgttt gtggctgtga cttggatacc 40

<210> 55
<211> 40
<212> DNA
<213> artificial sequence

<220>
<223> aptamere

<400> 55
ccggttacca cattggtgtt tgtggcagtg ttattccccg 40

<210> 56
<211> 38
<212> DNA
<213> artificial sequence

<220>
<223> aptamere

<400> 56
ccggttacca actatgatag tatggtgttt gtggcatg 38

<210> 57
<211> 38
<212> DNA
<213> artificial sequence

<220>
<223> aptamere

<400> 57
ccggttacca actatgatag tatggtgttt gtggcgtg 38

<210> 58
<211> 40
<212> DNA
<213> artificial sequence

<220>
<223> aptamere

<400> 58
ccggttacca tcgaggacgt cgcttggtgt ttgtggcatg 40

<210> 59
<211> 40
<212> DNA
<213> artificial sequence

<220>
<223> aptamere

<400> 59
ccggttacca agatcgttgt tcttggtgtt agtggcattc 40

<210> 60
<211> 40
<212> DNA
<213> artificial sequence

<220>
<223> aptamere

<400> 60
ccggttaccc agtctcctgc ggtgtttgtg gcatagggca 40

<210> 61
<211> 40
<212> DNA
<213> artificial sequence

<220>
<223> aptamere

<400> 61
tgaggcgcag atgtggaggc ttttacaggc ggtgcggaac 40

<210> 62
<211> 40
<212> DNA

<213> artificial sequence

<220>
<223> aptamere

<400> 62
tgaggcgcag atgtggaggc ttttaccggc ggtgcggaac 40

<210> 63
<211> 40
<212> DNA
<213> artificial sequence

<220>
<223> aptamere

<400> 63
tggggcgcag atgtggaggc ttttacaggc ggtgcggaac 40

<210> 64
<211> 40
<212> DNA
<213> artificial sequence

<220>
<223> aptamere

<400> 64
ggggggggcg gtgccgggag gctttcatca ggctgtgcgt 40

<210> 65
<211> 40
<212> DNA
<213> artificial sequence

<220>
<223> aptamere

<400> 65
caaagggggg ggttggggga ccgtccgttg ttgatctcac 40

<210> 66
<211> 40
<212> DNA
<213> artificial sequence

<220>
<223> aptamere

<400> 66
ccaggggggg ggtgatatag gggaccgtcc gtattggtag 40

<210> 67
<211> 40
<212> DNA
<213> artificial sequence

<220>

<223> aptamere

<400> 67
ggcgaattgg ttctgtgggg ggttggggcg gcaattcgat 40

<210> 68
<211> 41
<212> DNA
<213> artificial sequence

<220>
<223> aptamere

<400> 68
gagcgtgcca aagggttttg tggggggggt ggggcggcaa a 41

<210> 69
<211> 40
<212> DNA
<213> artificial sequence

<220>
<223> aptamere

<400> 69
ttgccatgtg gtctttgtgg gttcgggtta gggtagggtt 40

<210> 70
<211> 39
<212> DNA
<213> artificial sequence

<220>
<223> aptamere

<400> 70
gttgttcttt gtgggtgcgg gttagggtag ggggattag 39

<210> 71
<211> 40
<212> DNA
<213> artificial sequence

<220>
<223> aptamere

<400> 71
ggcaaaccgt cttagtgggt gtggggttag ggttgggggt 40

<210> 72
<211> 40
<212> DNA
<213> artificial sequence

<220>
<223> aptamere

<400> 72

ggctgcaagg gattggatca gatacggggg ggacagctcg 40

<210> 73
<211> 40
<212> DNA
<213> artificial sequence

<220>
<223> aptamere

<400> 73
ggcatgcgga ccaccaatca gcccgggcgt cgggcggcca 40

<210> 74
<211> 40
<212> DNA
<213> artificial sequence

<220>
<223> aptamere

<400> 74
cgcaggaagg ccggaggtta taacggcaac tcttcagcca 40

<210> 75
<211> 40
<212> DNA
<213> artificial sequence

<220>
<223> aptamere

<400> 75
tatgccccat cccgcgaaca ccgtcagtaa cgcaactgtt 40

<210> 76
<211> 40
<212> DNA
<213> artificial sequence

<220>
<223> aptamere

<400> 76
cgcgctcggt ctagggaata cggccgccca attcgttacc 40

<210> 77
<211> 40
<212> DNA
<213> artificial sequence

<220>
<223> aptamere

<400> 77
ggtgggaggt gaggtcgttg gacggtggca ggggattttg 40

<210> 78

<211> 40
<212> DNA
<213> artificial sequence

<220>
<223> aptamere

<400> 78
gtggaggggt tgggcgtggc ccaggggagg gtggcttgtt 40

<210> 79
<211> 40
<212> DNA
<213> artificial sequence

<220>
<223> aptamere

<400> 79
tgcgttgggg gggtgcgcgg gggcaacgtg gcacgtgagg 40

<210> 80
<211> 40
<212> DNA
<213> artificial sequence

<220>
<223> aptamere

<400> 80
gtggtacgcg tattggcggt agtggagttt gaggcacgag 40

<210> 81
<211> 40
<212> DNA
<213> artificial sequence

<220>
<223> aptamere

<400> 81
gtgctgctgt agtaggccat gtggagggtt ccagggaggt 40

<210> 82
<211> 40
<212> DNA
<213> artificial sequence

<220>
<223> aptamere

<400> 82
gggggactgg agggtatacg ccgaggaggt tcgggggttg 40

<210> 83
<211> 40
<212> DNA
<213> artificial sequence

<220>
<223> aptamere

<400> 83
acgtggaggg tgccagggag gtgtgagaat aggacgcgct 40

<210> 84
<211> 40
<212> DNA
<213> artificial sequence

<220>
<223> aptamere

<400> 84
accggggaga aggagccgga cctggggcgc gcgggcgtcg 40

<210> 85
<211> 40
<212> DNA
<213> artificial sequence

<220>
<223> aptamere

<400> 85
gggtccaggg catcctgcgt aggtggcggg cttatgttac 40

<210> 86
<211> 40
<212> DNA
<213> artificial sequence

<220>
<223> aptamere

<400> 86
gggcagccct ccttctcagt cggtgttggt gatgtgacta 40

<210> 87
<211> 40
<212> DNA
<213> artificial sequence

<220>
<223> aptamere

<400> 87
tggccccgat gtcctggtaa gatggatggt ctgggatggt 40

<210> 88
<211> 40
<212> DNA
<213> artificial sequence

<220>
<223> aptamere

<400> 88
cctgccgcgg tgtgtgggtt attctggtta ttttggtggc 40

<210> 89
<211> 40
<212> DNA
<213> artificial sequence

<220>
<223> aptamere

<400> 89
ggccgggcgg gtgtgggggg tactgagggt aagtagcggc 40

<210> 90
<211> 40
<212> DNA
<213> artificial sequence

<220>
<223> aptamere

<400> 90
gcggtggccg acggttacct atggtgttag ttgtagaaca 40

<210> 91
<211> 40
<212> DNA
<213> artificial sequence

<220>
<223> aptamere

<400> 91
ggattcaggt ggccgaacgg tgttgtgttg agtacggtgg 40

<210> 92
<211> 39
<212> DNA
<213> artificial sequence

<220>
<223> aptamere

<400> 92
caagggagcg agagttggcg cgtctacaca taccctgac 39

<210> 93
<211> 40
<212> DNA
<213> artificial sequence

<220>
<223> aptamere

<400> 93
ggacgcaggg gtggacgcta taagccatgg atagttcagt 40

<210> 94
<211> 40
<212> DNA
<213> artificial sequence

<220>
<223> aptamere

<400> 94
gtcgacacgg gggggatatc gctatcgcaa tctgatcgct 40

<210> 95
<211> 40
<212> DNA
<213> artificial sequence

<220>
<223> aptamere

<400> 95
aaggcggggc ggtactgtac tctcgtaata ctgttccccg 40

<210> 96
<211> 40
<212> DNA
<213> artificial sequence

<220>
<223> aptamere

<400> 96
caaagggggg ggttggggga ccgtccgttg ttgatctcac 40

<210> 97
<211> 40
<212> DNA
<213> artificial sequence

<220>
<223> aptamere

<400> 97
ccaggggggg ggtgatatag gggaccgtcc gtattggtag 40

<210> 98
<211> 40
<212> DNA
<213> artificial sequence

<220>
<223> aptamere

<400> 98
tatgggtgca cttgtcacgt accttgggac tttcaagggt 40

<210> 99
<211> 40
<212> DNA

<213> artificial sequence

<220>
<223> aptamere

<400> 99
ggggggaata gtcaagtatt gagcgggact ttcgttgccc 40

<210> 100
<211> 40
<212> DNA
<213> artificial sequence

<220>
<223> aptamere

<400> 100
acgggggtat aatgttcatt gaatcaggcc gcccgtgtgt 40

<210> 101
<211> 40
<212> DNA
<213> artificial sequence

<220>
<223> aptamere

<400> 101
acgcggggat aaaagtctgt ctattggtga aggcacgcca 40

<210> 102
<211> 40
<212> DNA
<213> artificial sequence

<220>
<223> aptamere

<400> 102
cgggcggtag aagttggctc gtcggttagg tgggaagggt 40

<210> 103
<211> 40
<212> DNA
<213> artificial sequence

<220>
<223> aptamere

<400> 103
cactggcgaa gagacgttgg cttcgttgcg gcggcccagt 40

<210> 104
<211> 40
<212> DNA
<213> artificial sequence

<220>

<223> aptamere

<400> 104
gtcgtggtgc cgaagggaga cgaattcgat tgccggtggc 40

<210> 105
<211> 40
<212> DNA
<213> artificial sequence

<220>
<223> aptamere

<400> 105
gggcggtgga agtggggaga aagtgggtat tgtggccagt 40

<210> 106
<211> 40
<212> DNA
<213> artificial sequence

<220>
<223> aptamere

<400> 106
ggcagaacgg gacatattgg aatgcctctg tgtgtttagt 40

<210> 107
<211> 40
<212> DNA
<213> artificial sequence

<220>
<223> aptamere

<400> 107
ctgaacggtg gaattatgtc ggtgcgtgta cctattcggg 40

<210> 108
<211> 40
<212> DNA
<213> artificial sequence

<220>
<223> aptamere

<400> 108
ggacaccagc acgtagggga gtatatatca atggggggct 40

<210> 109
<211> 40
<212> DNA
<213> artificial sequence

<220>
<223> aptamere

<400> 109

cgacgcgaga gccagggcgc ggtgtcttct tgtggggtag 40

<210> 110
<211> 40
<212> DNA
<213> artificial sequence

<220>
<223> aptamere

<400> 110
tgtgagcgca gcactgtgta cccccctatt gttatctgga 40

<210> 111
<211> 40
<212> DNA
<213> artificial sequence

<220>
<223> aptamere

<400> 111
gggattttga cgcgcacgtg ggaccacccc tgcctagttc 40

<210> 112
<211> 40
<212> DNA
<213> artificial sequence

<220>
<223> aptamere

<400> 112
aaacccgatc cacgccccct gtagtgatcc taagatgatc 40

<210> 113
<211> 40
<212> DNA
<213> artificial sequence

<220>
<223> aptamere

<400> 113
aacgccccgc ccactatgcg ccgtatatgg accagatcct 40

<210> 114
<211> 40
<212> DNA
<213> artificial sequence

<220>
<223> aptamere

<400> 114
agcacccggg gcctgaaaga aggcgaggta atatggactt 40

<210> 115

<211> 40
<212> DNA
<213> artificial sequence

<220>
<223> aptamere

<400> 115
caccgcctga acgaacaaga aagcgagaac caggagctac 40

<210> 116
<211> 39
<212> DNA
<213> artificial sequence

<220>
<223> aptamere

<400> 116
gctgcgcgct tctgatgtac tgaaggggtc cgggagtac 39

<210> 117
<211> 76
<212> DNA
<213> artificial sequence

<220>
<223> aptamere

<400> 117

**gggtcaatgc caggtctccg gtctcgagat ggggtctggt ggttatacgc tctcagtgat 60**

**cggctcgcaa gcagtc 76**

<210> 118
<211> 76
<212> DNA
<213> artificial sequence

<220>
<223> aptamere

<400> 118

**gggtcaatgc caggtctcac cgtcatcgag aagggtctgg tggttatacg ttctcgtgat 60**

**cggctcgcaa gcagtc 76**

<210> 119
<211> 76
<212> DNA
<213> artificial sequence

<220>
<223> aptamere

<400> 119

gggtcaatgc caggtctcgg accgcgatgg agaggggtct ggtggttata cgctatgcat 60

cggctcgcaa gcagtc 76

<210> 120
<211> 76
<212> DNA
<213> artificial sequence

<220>
<223> aptamere

<400> 120

gggtcaatgc caggtctcgg gccgcgatgg agaggggtct ggtggttata cgctatgtat 60

cggctcgcaa gcagtc 76

<210> 121
<211> 76
<212> DNA
<213> artificial sequence

<220>
<223> aptamere

<400> 121

gggtcaatgc caggtctccg gcagcagatg gacagggtca ggtggttata cgtgtggcat 60

cggctcgcaa gcagtc 76

<210> 122
<211> 76
<212> DNA
<213> artificial sequence

<220>
<223> aptamere

<400> 122

gggtcaatgc caggtctcgc gatgaccaat aggggtcagg tggttatacg ctttggatat 60

cggctcgcaa gcagtc 76

<210> 123
<211> 74
<212> DNA
<213> artificial sequence

<220>

<223> aptamere

<400> 123

gggtcaatgc caggtctcgg gggcggccgc agtaggggtc tggtggttat acgcgtatcg 60

gctcgcaagc agtc 74

<210> 124
<211> 76
<212> DNA
<213> artificial sequence

<220>
<223> aptamere

<400> 124

gggtcaatgc caggtctcgc agaggtgcga ggggcggtgg ggtctggtgg ttatacgcat 60

cggctcgcaa gcagtc 76

<210> 125
<211> 76
<212> DNA
<213> artificial sequence

<220>
<223> aptamere

<400> 125

gggtcaatgc caggtctcac gtcgagttgt atggtggggt caggtggtta tacgcagcat 60

cggctcgcaa gcagtc 76

<210> 126
<211> 76
<212> DNA
<213> artificial sequence

<220>
<223> aptamere

<400> 126

gggtcaatgc caggtctcaa ggccgacaca ctgggggtc tggtggttat acgcccgtat 60

cggctcgcaa gcagtc 76

<210> 127
<211> 76
<212> DNA
<213> artificial sequence

<220>
<223> aptamere

<400> 127

gggtcaatgc caggtctcgc atgatactac tggggtctgg tggttatacg caggtaggat 60

cggctcgcaa gcagtc 76

<210> 128
<211> 76
<212> DNA
<213> artificial sequence

<220>
<223> aptamere

<400> 128

gggtcaatgc caggtctcga ggaacgtggg gtcaggtggt tatacgcagg gtaccgggat 60

cggctcgcaa gcagtc 76

<210> 129
<211> 76
<212> DNA
<213> artificial sequence

<220>
<223> aptamere

<400> 129

gggtcaatgc caggtctcgg cagggaggag gaatcgggac aggcggttat acggattcat 60

cggctcgcaa gcagtc 76

<210> 130
<211> 76
<212> DNA
<213> artificial sequence

<220>
<223> aptamere

<400> 130

gggtcaatgc caggtctccg gaaagagtat ggaacgggtc aggcggttat aaggttacat 60

cggctcgcaa gcagtc 76

<210> 131
<211> 76
<212> DNA
<213> artificial sequence

<220>
<223> aptamere

<400> 131

gggtcaatgc caggtctcca cggcgtatgc gtagatcgat gggtctggcg gttatacgat 60

cggctcgcaa gcagtc 76

<210> 132
<211> 76
<212> DNA
<213> artificial sequence

<220>
<223> aptamere

<400> 132

gggtcaatgc caggtctcac aagagctgta ttagtcgggt ctggcggtta tacggactat 60

cggctcgcaa gcagtc 76

<210> 133
<211> 76
<212> DNA
<213> artificial sequence

<220>
<223> aptamere

<400> 133

gggtcaatgc caggtctcag ggcggattgc ggtacggttg ggtctggtgg ttatacgaat 60

cggctcgcaa gcagtc 76

<210> 134
<211> 76
<212> DNA
<213> artificial sequence

<220>
<223> aptamere

<400> 134

gggtcaatgc caggtctctg cggagcatta cattcacggg tctggcggtt atacggtgat 60

cggctcgcaa gcagtc 76

<210> 135
<211> 76
<212> DNA

<213> artificial sequence

<220>
<223> aptamere

<400> 135

```
gggtcaatgc caggtctcgg ggggccggtt atacgggtca ggcggttata cggtattcat        60

cggctcgcaa gcagtc                                                        76
```

<210> 136
<211> 76
<212> DNA
<213> artificial sequence

<220>
<223> aptamere

<400> 136

```
gggtcaatgc caggtctcga ggggctgggg tcacaggggt cggtgtggtt attattctat        60

cggctcgcaa gcagtc                                                        76
```

<210> 137
<211> 76
<212> DNA
<213> artificial sequence

<220>
<223> aptamere

<400> 137

```
gggtcaatgc caggtctcgg gacaggcggt tatacggaga gttctggtgt agggttggat        60

cggctcgcaa gcagtc                                                        76
```

<210> 138
<211> 76
<212> DNA
<213> artificial sequence

<220>
<223> aptamere

<400> 138

```
gggtcaatgc caggtctcgg ggcaggcggt tatacggaga gcttcttaca tgggccctat        60

cggctcgcaa gcagtc                                                        76
```

<210> 139

<211> 76
<212> DNA
<213> artificial sequence

<220>
<223> aptamere

<400> 139

gggtcaatgc caggtctcgg gtcaggcggt tatacggaga caccttgctg tgttaggcat 60

cggctcgcaa gcagtc 76

<210> 140
<211> 76
<212> DNA
<213> artificial sequence

<220>
<223> aptamere

<400> 140

gggtcaatgc caggtctccg gggcaggcgg ttatacggga gaacggtatg gggggactat 60

cggctcgcaa gcagtc 76

<210> 141
<211> 76
<212> DNA
<213> artificial sequence

<220>
<223> aptamere

<400> 141

gggtcaatgc caggtctccg gggtctggtg gttatacgct gagatgggtg ctacagagat 60

cggctcgcaa gcagtc 76

<210> 142
<211> 76
<212> DNA
<213> artificial sequence

<220>
<223> aptamere

<400> 142

gggtcaatgc caggtctcac cgggtcaggc ggttatacgg gtgggggcag gtacatacat 60

cggctcgcaa gcagtc 76

<210> 143
<211> 76
<212> DNA
<213> artificial sequence

<220>
<223> aptamere

<400> 143

gggtcaatgc caggtctcac aggggtcagt ggaagttata gactgggaag gcatacaaat 60

cggctcgcaa gcagtc 76

<210> 144
<211> 76
<212> DNA
<213> artificial sequence

<220>
<223> aptamere

<400> 144

gggtcaatgc caggtctcgg gcacgggtca ggcggttata cggtgcccat tgttctttat 60

cggctcgcaa gcagtc 76

<210> 145
<211> 76
<212> DNA
<213> artificial sequence

<220>
<223> aptamere

<400> 145

gggtcaatgc caggtctcac gggtcaggcg gttatacggt gtggcttgat ggtgacttat 60

cggctcgcaa gcagtc 76

<210> 146
<211> 76
<212> DNA
<213> artificial sequence

<220>
<223> aptamere

<400> 146

gggtcaatgc caggtctcgg gtcaggcggt tatacggagg cctcgctgaa cccagctaat 60

cggctcgcaa gcagtc 76

<210> 147
<211> 75
<212> DNA
<213> artificial sequence

<220>
<223> aptamere

<400> 147

```
gggtcaatgc caggtctcgc aacgggtcag gcggttatac ggttcgtatc ctggcgaatc        60

ggctcgcaag cagtc                                                          75
```

<210> 148
<211> 76
<212> DNA
<213> artificial sequence

<220>
<223> aptamere

<400> 148

```
gggtcaatgc caggtctcaa gtggggtctg gtggttatac gcggttgtgg tgattgacat        60

cggctcgcaa gcagtc                                                         76
```

<210> 149
<211> 76
<212> DNA
<213> artificial sequence

<220>
<223> aptamere

<400> 149

```
gggtcaatgc caggtctcgc gggtctggtg gttatacgtc gaatttgtta aattgccaat        60

cggctcgcaa gcagtc                                                         76
```

<210> 150
<211> 76
<212> DNA
<213> artificial sequence

<220>
<223> aptamere

<400> 150

gggtcaatgc caggtctcaa tcagaagggt ctggtggtta tacgttcatg tattgggtat 60

cggctcgcaa gcagtc 76

<210> 151
<211> 76
<212> DNA
<213> artificial sequence

<220>
<223> aptamere

<400> 151

gggtcaatgc caggtctcgg atggggtcag gtggttatac gctctgagtg gttttggtat 60

cggctcgcaa gcagtc 76

<210> 152
<211> 76
<212> DNA
<213> artificial sequence

<220>
<223> aptamere

<400> 152

gggtcaatgc caggtctcgg ggtgcgaggg tctggtggtt atacgtcgtc acctctagat 60

cggctcgcaa gcagtc 76

<210> 153
<211> 76
<212> DNA
<213> artificial sequence

<220>
<223> aptamere

<400> 153

gggtcaatgc caggtctctg ggggagggag ggtctggggg ttatacgtca ctagcaaaat 60

cggctcgcaa gcagtc 76

<210> 154
<211> 76
<212> DNA
<213> artificial sequence

<220>
<223> aptamere

<400> 154

gggtcaatgc caggtctcga ggggcttttg ggagggactg gtggttatac gtcccaatat 60

cggctcgcaa gcagtc 76

<210> 155
<211> 76
<212> DNA
<213> artificial sequence

<220>
<223> aptamere

<400> 155

gggtcaatgc caggtctcgc ggtggacggg gctggcggtt atacggtcct tacgcggtat 60

cggctcgcaa gcagtc 76

<210> 156
<211> 76
<212> DNA
<213> artificial sequence

<220>
<223> aptamere

<400> 156

gggtcaatgc caggtctcgc ggctgtgggg tctggtggtt atacgcacat acgcgctgat 60

cggctcgcaa gcagtc 76

<210> 157
<211> 76
<212> DNA
<213> artificial sequence

<220>
<223> aptamere

<400> 157

gggtcaatgc caggtctcgg gccgcgatgg agaggggtct ggtggttata cgctatgcat 60

cggctcgcaa gcagtc 76

<210> 158
<211> 76
<212> DNA
<213> artificial sequence

<220>

<223> aptamere

<400> 158

gggtcaatgc caggtctcac cgggtcaggc ggttatacgg gtgagggcag gtacatacat 60

cggctcgcaa gcagtc 76

<210> 159
<211> 76
<212> DNA
<213> artificial sequence

<220>
<223> aptamere

<400> 159

gggtcaatgc caggtctccc ggttaccgcg ctaagcttgg cgatggtgtt agtggctgat 60

cggctcgcaa gcagtc 76

<210> 160
<211> 76
<212> DNA
<213> artificial sequence

<220>
<223> aptamere

<400> 160

gggtcaatgc caggtctccc ggttaccgtt acgttggacg aggaatggtg ttcgtggcat 60

cggctcgcaa gcagtc 76

<210> 161
<211> 76
<212> DNA
<213> artificial sequence

<220>
<223> aptamere

<400> 161

gggtcaatgc caggtctccc ggttactgcc gtagttgtct tacggtggtg ttagtggcat 60

cggctcgcaa gcagtc 76

<210> 162
<211> 76
<212> DNA
<213> artificial sequence

<220>
<223> aptamere

<400> 162

gggtcaatgc caggtctccc ggttacttcc gtagctgtct tacggtggtg ttagtggcat 60

cggctcgcaa gcagtc 76

<210> 163
<211> 76
<212> DNA
<213> artificial sequence

<220>
<223> aptamere

<400> 163

gggtcaatgc caggtctccc ggttaccgca ttagtggtgt ttgtggcgtt gagatggcat 60

cggctcgcaa gcagtc 76

<210> 164
<211> 76
<212> DNA
<213> artificial sequence

<220>
<223> aptamere

<400> 164

gggtcaatgc caggtctccc ggttacctcc attggtggtg ttagtggctt tgtaggatat 60

cggctcgcaa gcagtc 76

<210> 165
<211> 76
<212> DNA
<213> artificial sequence

<220>
<223> aptamere

<400> 165

gggtcaatgc caggtctccc ggttaccagt tggtgtttgt ggctttgcac gtctgcatat 60

cggctcgcaa gcagtc 76

<210> 166
<211> 76
<212> DNA
<213> artificial sequence

<220>
<223> aptamere

<400> 166

gggtcaatgc caggtctccc ggttaccagt tggtgtttgt ggctttgcac gtgtgcatat 60

cggctcgcaa gcagtc 76

<210> 167
<211> 79
<212> DNA
<213> artificial sequence

<220>
<223> aptamere

<400> 167

gggtcaatgc caggtctccc ggttaccagt tggtgttgtt agtggctttg cacgtctgca 60

tatcggctcg caagcagtc 79

<210> 168
<211> 76
<212> DNA
<213> artificial sequence

<220>
<223> aptamere

<400> 168

gggtcaatgc caggtctccc ggttaccaac tatggtgtta gtggcatcga tcgggatcat 60

cggctcgcaa gcagtc 76

<210> 169
<211> 74
<212> DNA
<213> artificial sequence

<220>
<223> aptamere

<400> 169

gggtcaatgc caggtctccc ggttaccttt ggtgtttgtg gcatgggcgt cggggcatcg 60

gctcgcaagc agtc 74

<210> 170
<211> 76
<212> DNA
<213> artificial sequence

<220>
<223> aptamere

<400> 170

gggtcaatgc caggtctccc ggttaccgtt tggtgtttgt ggctgtgact tggataccat 60

cggctcgcaa gcagtc 76

<210> 171
<211> 76
<212> DNA
<213> artificial sequence

<220>
<223> aptamere

<400> 171

gggtcaatgc caggtctccc ggttaccaca ttggtgtttg tggcagtgtt attccccgat 60

cggctcgcaa gcagtc 76

<210> 172
<211> 74
<212> DNA
<213> artificial sequence

<220>
<223> aptamere

<400> 172

gggtcaatgc caggtctccc ggttaccaac tatgatagta tggtgtttgt ggcatgatcg 60

gctcgcaagc agtc 74

<210> 173
<211> 74
<212> DNA
<213> artificial sequence

<220>
<223> aptamere

<400> 173

gggtcaatgc caggtctccc ggttaccaac tatgatagta tggtgtttgt ggcgtgatcg 60

gctcgcaagc agtc 74

<210> 174
<211> 76
<212> DNA

<213> artificial sequence

<220>
<223> aptamere

<400> 174

gggtcaatgc caggtctccc ggttaccatc gaggacgtcg cttggtgttt gtggcatgat 60

cggctcgcaa gcagtc 76

<210> 175
<211> 76
<212> DNA
<213> artificial sequence

<220>
<223> aptamere

<400> 175

gggtcaatgc caggtctccc ggttaccaag atcgttgttc ttggtgttag tggcattcat 60

cggctcgcaa gcagtc 76

<210> 176
<211> 76
<212> DNA
<213> artificial sequence

<220>
<223> aptamere

<400> 176

gggtcaatgc caggtctccc ggttacccag tctcctgcgg tgtttgtggc atagggcaat 60

cggctcgcaa gcagtc 76

<210> 177
<211> 76
<212> DNA
<213> artificial sequence

<220>
<223> aptamere

<400> 177

gggtcaatgc caggtctctg aggcgcagat gtggaggctt ttacaggcgg tgcggaacat 60

cggctcgcaa gcagtc 76

<210> 178
<211> 76

<212> DNA
<213> artificial sequence

<220>
<223> aptamere

<400> 178

gggtcaatgc caggtctctg aggcgcagat gtggaggctt ttaccggcgg tgcggaacat 60

cggctcgcaa gcagtc 76

<210> 179
<211> 76
<212> DNA
<213> artificial sequence

<220>
<223> aptamere

<400> 179

gggtcaatgc caggtctctg gggcgcagat gtggaggctt ttacaggcgg tgcggaacat 60

cggctcgcaa gcagtc 76

<210> 180
<211> 75
<212> DNA
<213> artificial sequence

<220>
<223> aptamere

<400> 180

gggtcaatgc caggtctcgc tgcgcgcttc tgatgtactg aaggggtccg ggagtacatc 60

ggctcgcaag cagtc 75

<210> 181
<211> 76
<212> DNA
<213> artificial sequence

<220>
<223> aptamere

<400> 181

gggtcaatgc caggtctcca aagggggggg ttgggggacc gtccgttgtt gatctcacat 60

cggctcgcaa gcagtc 76

<210> 182
<211> 76
<212> DNA
<213> artificial sequence

<220>
<223> aptamere

<400> 182

gggtcaatgc caggtctccc aggggggggg tgatataggg gaccgtccgt attggtagat 60

cggctcgcaa gcagtc 76

<210> 183
<211> 76
<212> DNA
<213> artificial sequence

<220>
<223> aptamere

<400> 183

gggtcaatgc caggtctcgg cgaattggtt ctgtgggggg ttggggcggc aattcgatat 60

cggctcgcaa gcagtc 76

<210> 184
<211> 77
<212> DNA
<213> artificial sequence

<220>
<223> aptamere

<400> 184

gggtcaatgc caggtctcga gcgtgccaaa gggttttgtg gggggggtgg ggcggcaaaa 60

tcggctcgca agcagtc 77

<210> 185
<211> 76
<212> DNA
<213> artificial sequence

<220>
<223> aptamere

<400> 185

gggtcaatgc caggtctctt gccatgtggt ctttgtgggt tcgggttagg gtagggttat 60

cggctcgcaa gcagtc 76

<210> 186
<211> 75
<212> DNA
<213> artificial sequence

<220>
<223> aptamere

<400> 186

gggtcaatgc caggtctcgt tgttctttgt gggtgcgggt tagggtaggg ggattagatc 60

ggctcgcaag cagtc 75

<210> 187
<211> 76
<212> DNA
<213> artificial sequence

<220>
<223> aptamere

<400> 187

gggtcaatgc caggtctcgg caaaccgtct tagtgggtgt ggggttaggg ttgggggtat 60

cggctcgcaa gcagtc 76

<210> 188
<211> 76
<212> DNA
<213> artificial sequence

<220>
<223> aptamere

<400> 188

gggtcaatgc caggtctcgg ctgcaaggga ttggatcaga tacggggggg acagctcgat 60

cggctcgcaa gcagtc 76

<210> 189
<211> 76
<212> DNA
<213> artificial sequence

<220>
<223> aptamere

<400> 189

gggtcaatgc caggtctcgg catgcggacc accaatcagc ccgggcgtcg ggcggccaat 60

cggctcgcaa gcagtc 76

<210> 190
<211> 76
<212> DNA
<213> artificial sequence

<220>
<223> aptamere

<400> 190

gggtcaatgc caggtctccg caggaaggcc ggaggttata acggcaactc ttcagccaat 60

cggctcgcaa gcagtc 76

<210> 191
<211> 76
<212> DNA
<213> artificial sequence

<220>
<223> aptamere

<400> 191

gggtcaatgc caggtctcta tgccccatcc cgcgaacacc gtcagtaacg caactgttat 60

cggctcgcaa gcagtc 76

<210> 192
<211> 76
<212> DNA
<213> artificial sequence

<220>
<223> aptamere

<400> 192

gggtcaatgc caggtctccg cgctcggtct agggaatacg gccgcccaat tcgttaccat 60

cggctcgcaa gcagtc 76

<210> 193
<211> 76
<212> DNA
<213> artificial sequence

<220>
<223> aptamere

<400> 193

gggtcaatgc caggtctcgg tgggaggtga ggtcgttgga cggtggcagg ggattttgat 60

cggctcgcaa gcagtc 76

<210> 194
<211> 76
<212> DNA
<213> artificial sequence

<220>
<223> aptamere

<400> 194

gggtcaatgc caggtctcgt ggaggggttg ggcgtggccc aggggagggt ggcttgttat 60

cggctcgcaa gcagtc 76

<210> 195
<211> 76
<212> DNA
<213> artificial sequence

<220>
<223> aptamere

<400> 195

gggtcaatgc caggtctctg cgttgggggg gtgcgcgggg gcaacgtggc acgtgaggat 60

cggctcgcaa gcagtc 76

<210> 196
<211> 76
<212> DNA
<213> artificial sequence

<220>
<223> aptamere

<400> 196

gggtcaatgc caggtctcgt ggtacgcgta ttggcggtag tggagtttga ggcacgagat 60

cggctcgcaa gcagtc 76

<210> 197
<211> 76
<212> DNA
<213> artificial sequence

<220>

<223> aptamere

<400> 197

gggtcaatgc caggtctcgt gctgctgtag taggccatgt ggagggttcc agggaggtat 60

cggctcgcaa gcagtc 76

<210> 198
<211> 76
<212> DNA
<213> artificial sequence

<220>
<223> aptamere

<400> 198

gggtcaatgc caggtctcgg gggactggag ggtatacgcc gaggaggttc gggggttgat 60

cggctcgcaa gcagtc 76

<210> 199
<211> 76
<212> DNA
<213> artificial sequence

<220>
<223> aptamere

<400> 199

gggtcaatgc caggtctcac gtggagggtg ccagggaggt gtgagaatag gacgcgctat 60

cggctcgcaa gcagtc 76

<210> 200
<211> 75
<212> DNA
<213> artificial sequence

<220>
<223> aptamere

<400> 200

gggtcaatgc caggtctcac cggggagaag gagccggacc tggggcgcgc gggcgtcgat 60

cggctcgcaa gcagt 75

<210> 201
<211> 76
<212> DNA
<213> artificial sequence

<220>
<223> aptamere

<400> 201

gggtcaatgc caggtctcgg gtccagggca tcctgcgtag gtggcgggct tatgttacat 60

cggctcgcaa gcagtc 76

<210> 202
<211> 76
<212> DNA
<213> artificial sequence

<220>
<223> aptamere

<400> 202

gggtcaatgc caggtctcgg gcagccctcc ttctcagtcg gtgttggtga tgtgactaat 60

cggctcgcaa gcagtc 76

<210> 203
<211> 76
<212> DNA
<213> artificial sequence

<220>
<223> aptamere

<400> 203

gggtcaatgc caggtctctg gccccgatgt cctggtaaga tggatggtct gggatggtat 60

cggctcgcaa gcagtc 76

<210> 204
<211> 76
<212> DNA
<213> artificial sequence

<220>
<223> aptamere

<400> 204

gggtcaatgc caggtctccc tgccgcggtg tgtgggttat tctggttatt ttggtggcat 60

cggctcgcaa gcagtc 76

<210> 205
<211> 76
<212> DNA

<213> artificial sequence

<220>
<223> aptamere

<400> 2

gggtcaatgc caggtctcgg ccgggcgggt gtggggggta ctgagggtaa gtagcggcat 60

cggctcgcaa gcagtc 76

<210> 206
<211> 76
<212> DNA
<213> artificial sequence

<220>
<223> aptamere

<400> 206

gggtcaatgc caggtctcgc ggtggccgac ggttacctat ggtgttagtt gtagaacaat 60

cggctcgcaa gcagtc 76

<210> 207
<211> 76
<212> DNA
<213> artificial sequence

<220>
<223> aptamere

<400> 207

gggtcaatgc caggtctcgg attcaggtgg ccgaacggtg ttgtgttgag tacggtggat 60

cggctcgcaa gcagtc 76

<210> 208
<211> 75
<212> DNA
<213> artificial sequence

<220>
<223> aptamere

<400> 208

gggtcaatgc caggtctcca agggagcgag agttggcgcg tctacacata ccctgacatc 60

ggctcgcaag cagtc 75

<210> 209

<211> 76
<212> DNA
<213> artificial sequence

<220>
<223> aptamere

<400> 209

gggtcaatgc caggtctcgg acgcaggggt ggacgctata agccatggat agttcagtat 60

cggctcgcaa gcagtc 76

<210> 210
<211> 76
<212> DNA
<213> artificial sequence

<220>
<223> aptamere

<400> 210

gggtcaatgc caggtctcgt cgacacgggg gggatatcgc tatcgcaatc tgatcgctat 60

cggctcgcaa gcagtc 76

<210> 211
<211> 76
<212> DNA
<213> artificial sequence

<220>
<223> aptamere

<400> 211

gggtcaatgc caggtctcaa ggcggggcgg tactgtactc tcgtaatact gttccccgat 60

cggctcgcaa gcagtc 76

<210> 212
<211> 76
<212> DNA
<213> artificial sequence

<220>
<223> aptamere

<400> 212

gggtcaatgc caggtctcca aagggggggg ttgggggacc gtccgttgtt gatctcacat 60

cggctcgcaa gcagtc 76

<210> 213
<211> 76
<212> DNA
<213> artificial sequence

<220>
<223> aptamere

<400> 213

gggtcaatgc caggtctccc aggggggggg tgatataggg gaccgtccgt attggtagat 60

cggctcgcaa gcagtc 76

<210> 214
<211> 76
<212> DNA
<213> artificial sequence

<220>
<223> aptamere

<400> 214

gggtcaatgc caggtctcta tgggtgcact tgtcacgtac cttgggactt tcaagggtat 60

cggctcgcaa gcagtc 76

<210> 215
<211> 76
<212> DNA
<213> artificial sequence

<220>
<223> aptamere

<400> 215

gggtcaatgc caggtctcgg ggggaatagt caagtattga gcgggacttt cgttgcccat 60

cggctcgcaa gcagtc 76

<210> 216
<211> 76
<212> DNA
<213> artificial sequence

<220>
<223> aptamere

<400> 216

gggtcaatgc caggtctcac gggggtataa tgttcattga atcaggccgc ccgtgtgtat 60

cggctcgcaa gcagtc 76

<210> 217
<211> 76
<212> DNA
<213> artificial sequence

<220>
<223> aptamere

<400> 217

gggtcaatgc caggtctcac gcggggataa aagtctgtct attggtgaag gcacgccaat 60

cggctcgcaa gcagtc 76

<210> 218
<211> 76
<212> DNA
<213> artificial sequence

<220>
<223> aptamere

<400> 218

gggtcaatgc caggtctccg ggcggtagaa gttggctcgt cggttaggtg ggaagggtat 60

cggctcgcaa gcagtc 76

<210> 219
<211> 76
<212> DNA
<213> artificial sequence

<220>
<223> aptamere

<400> 219

gggtcaatgc caggtctcca ctggcgaaga gacgttggct tcgttgcggc ggcccagtat 60

cggctcgcaa gcagtc 76

<210> 220
<211> 76
<212> DNA
<213> artificial sequence

<220>
<223> aptamere

<400> 220

gggtcaatgc caggtctcgt cgtggtgccg aagggagacg aattcgattg ccggtggcat 60

cggctcgcaa gcagtc 76

<210> 221
<211> 76
<212> DNA
<213> artificial sequence

<220>
<223> aptamere

<400> 221

gggtcaatgc caggtctcgg gcggtggaag tggggagaaa gtgggtattg tggccagtat 60

cggctcgcaa gcagtc 76

<210> 222
<211> 76
<212> DNA
<213> artificial sequence

<220>
<223> aptamere

<400> 222

gggtcaatgc caggtctcgg cagaacggga catattggaa tgcctctgtg tgtttagtat 60

cggctcgcaa gcagtc 76

<210> 223
<211> 76
<212> DNA
<213> artificial sequence

<220>
<223> aptamere

<400> 223

gggtcaatgc caggtctcct gaacggtgga attatgtcgg tgcgtgtacc tattcgggat 60

cggctcgcaa gcagtc 76

<210> 224
<211> 76
<212> DNA
<213> artificial sequence

<220>
<223> aptamere

<400> 224

gggtcaatgc caggtctcgg acaccagcac gtaggggagt atatatcaat gggggctat 60

cggctcgcaa gcagtc 76

<210> 225
<211> 76
<212> DNA
<213> artificial sequence

<220>
<223> aptamere

<400> 225

gggtcaatgc caggtctccg acgcgagagc cagggcgcgg tgtcttcttg tggggtagat 60

cggctcgcaa gcagtc 76

<210> 226
<211> 76
<212> DNA
<213> artificial sequence

<220>
<223> aptamere

<400> 226

gggtcaatgc caggtctctg tgagcgcagc actgtgtacc cccctattgt tatctggaat 60

cggctcgcaa gcagtc 76

<210> 227
<211> 76
<212> DNA
<213> artificial sequence

<220>
<223> aptamere

<400> 227

gggtcaatgc caggtctcgg gattttgacg cgcacgtggg accaccccctg cctagttcat 60

cggctcgcaa gcagtc 76

<210> 228
<211> 76
<212> DNA
<213> artificial sequence

<220>

<223> aptamere

<400> 228

gggtcaatgc caggtctcaa acccgatcca cgccccctgt agtgatccta agatgatcat 60

cggctcgcaa gcagtc 76

<210> 229
<211> 76
<212> DNA
<213> artificial sequence

<220>
<223> aptamere

<400> 229

gggtcaatgc caggtctcaa cgccccgccc actatgcgcc gtatatggac cagatcctat 60

cggctcgcaa gcagtc 76

<210> 230
<211> 76
<212> DNA
<213> artificial sequence

<220>
<223> aptamere

<400> 230

gggtcaatgc caggtctcag cacccggggc ctgaaagaag gcgaggtaat atggacttat 60

cggctcgcaa gcagtc 76

<210> 231
<211> 76
<212> DNA
<213> artificial sequence

<220>
<223> aptamere

<400> 231

gggtcaatgc caggtctcca ccgcctgaac gaacaagaaa gcgagaacca ggagctacat 60

cggctcgcaa gcagtc 76

<210> 232
<211> 75
<212> DNA
<213> artificial sequence

<220>
<223> aptamere

<400> 232

gggtcaatgc caggtctcgc tgcgcgcttc tgatgtactg aaggggtccg ggagtacatc 60

ggctcgcaag cagtc 75

<210> 233
<211> 16
<212> DNA
<213> artificial sequence

<220>
<223> consensus

<400> 233
gggtcggggt tatacg 16

<210> 234
<211> 18
<212> DNA
<213> artificial sequence

<220>
<223> consensus

<400> 234
gggtctggtg gttatacg 18

<210> 235
<211> 21
<212> DNA
<213> artificial sequence

<220>
<223> consensus

<400> 235
ccggttacct ggtgttgtgg c 21

<210> 236
<211> 22
<212> DNA
<213> artificial sequence

<220>
<223> consensus

<400> 236
ggaggctttt acaggcggtg cg 22

<210> 237
<211> 29
<212> DNA
<213> artificial sequence

<220>
<223> consensus

<400> 237
ggggggggtt gggggaccgt ccgtttgat 29

<210> 238
<211> 15
<212> DNA
<213> artificial sequence

<220>
<223> consensus

<400> 238
tgtggggggt tgggg 15

<210> 239
<211> 38
<212> DNA
<213> Artificial sequence

<220>
<223> Aptamere

<400> 239
caggtctcgg gcacgggtca ggcggttata cggtgccc 38

<210> 240
<211> 40
<212> DNA
<213> Artificial sequence

<220>
<223> Aptamere

<400> 240
gggcacgggt caggcggtta tacggtgccc attgttcttt 40

<210> 241
<211> 35
<212> DNA
<213> Artificial Sequence

<220>
<223> Aptamere

<400> 241
gggcacgggt caggcggtta tacggtgccc attgt 35

<210> 242
<211> 30
<212> DNA
<213> Artificial Sequence

<220>
<223> Aptamere

<400> 242
gggcacgggt caggcggtta tacggtgccc 30

<210> 243
<211> 24
<212> DNA
<213> Artificial Sequence

<220>
<223> Aptamere

<400> 243
cacgggtcag gcggttatac ggtg 24